(19) Europäisches Patentamt
European Patent Office
Office européen des brevets

(11) EP 4 253 387 A1

(12) **EUROPEAN PATENT APPLICATION**
published in accordance with Art. 153(4) EPC

(43) Date of publication:
**04.10.2023 Bulletin 2023/40**

(21) Application number: **21897136.4**

(22) Date of filing: **26.11.2021**

(51) International Patent Classification (IPC):
***C07D 519/00*** *(2006.01)* ***C07D 487/04*** *(2006.01)*
***C07D 471/04*** *(2006.01)* ***C07D 213/80*** *(2006.01)*
***C07D 213/75*** *(2006.01)* ***A61K 31/435*** *(2006.01)*
***A61K 31/495*** *(2006.01)* ***A61K 31/519*** *(2006.01)*
***A61P 31/12*** *(2006.01)* ***A61P 31/14*** *(2006.01)*
***A61P 31/22*** *(2006.01)*

(52) Cooperative Patent Classification (CPC):
**A61K 31/435; A61K 31/495; A61K 31/519; A61P 31/12; A61P 31/14; A61P 31/22; C07D 213/75; C07D 213/80; C07D 471/04; C07D 487/04; C07D 519/00;** Y02P 20/55

(86) International application number:
**PCT/CN2021/133540**

(87) International publication number:
**WO 2022/111636 (02.06.2022 Gazette 2022/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.11.2020 CN 202011346809**
**20.01.2021 CN 202110074443**
**14.04.2021 CN 202110401565**
**26.09.2021 CN 202111128775**

(71) Applicants:
- **Jiangsu Hengrui Pharmaceuticals Co., Ltd.**
  **Lianyungang, Jiangsu 222047 (CN)**
- **Shanghai Hengrui Pharmaceutical Co., Ltd.**
  **Shanghai 200245 (CN)**

(72) Inventors:
- **ZHANG, Xiaomin**
  **Shanghai 200245 (CN)**
- **HU, Weimin**
  **Shanghai 200245 (CN)**
- **HE, Feng**
  **Shanghai 200245 (CN)**
- **TAO, Weikang**
  **Shanghai 200245 (CN)**

(74) Representative: **Viganò, Elena et al**
**Dragotti & Associati S.r.l.**
**Via Nino Bixio, 7**
**20129 Milano (IT)**

(54) **FUSED TRICYCLIC COMPOUND, PREPARATION METHOD THEREFOR AND APPLICATION THEREOF IN MEDICINE**

(57) The present invention relates to a fused tricyclic compound, a preparation method therefor, and an application thereof in medicine. Specifically, the present invention relates to a fused tricyclic compound represented by general formula (I), a preparation method therefor, a pharmaceutical composition containing the compound, and a use thereof as a therapeutic agent, particularly a use as a TLR7/8/9 inhibitor and a use in preparing a drug for the treatment and/or prevention of inflammatory and autoimmune diseases. The definition of each group in general formula (I) is as defined in the description.

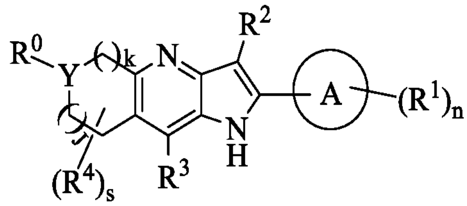

(I)

EP 4 253 387 A1

## Description

### TECHNICAL FIELD

The present disclosure belongs to the field of pharmaceutics, and relates to a fused tricyclic compound, a preparation method therefor, and pharmaceutical use thereof. In particular, the present disclosure relates to a fused tricyclic compound of general formula (I), a preparation method therefor, a pharmaceutical composition comprising same, and use thereof as a therapeutic agent, particularly use thereof as a TLR7/8/9 inhibitor and use thereof in the preparation of a medicament for treating and/or preventing inflammatory and autoimmune diseases.

### BACKGROUND

Toll-like receptors (TLRs) are a class of evolutionarily conserved transmembrane innate immune receptors, which are involved in the first line of defense in protecting human health and play an important role in recognizing pathogen-associated molecular patterns (PAMPs) (Kawai, T., et al., Nature Immunol., 11, 2010, 373-384). TLRs are expressed in various immune cells and can be classified by expression site into two categories: TLRs expressed on cell membranes (TLR1/2/4/5/6) and TLRs expressed on endosomal membranes (TLR3/7/8/9), which recognize different components and molecules in PAMPs. TLR7/8/9 is mainly highly expressed in DC cells and B cells. TLR7/8 mainly recognizes ssRNA, and TLR9 mainly recognizes CpG-DNA. TLR7/8/9 is activated upon binding to its ligand and binds to the adaptor protein MyD88 in cytoplasm, initiating the NF-κB and IRF pathways and activating DC cells to produce type I interferons and various other inflammatory cytokines. In B cells, TLR7/8/9 binds to nucleic acid substances to play an important role in the process of B cells producing antinuclear antibodies, and the type I interferons secreted by DC cells will promote further proliferation and activation of such autoimmune B cells, thereby causing a range of inflammatory reactions.

Systemic lupus erythematosus (SLE) is an autoimmune connective tissue disease. There are three major classes of first-line clinical drugs for SLE: hormones, immunosuppressants, and antimalarial drugs. In this century, there is only one new drug that has been approved by FDA-belimumab. However, it has moderate and delayed efficacy in only a small fraction of SLE patients (Navarra, S. V., et al., Lancet 2011, 377, 721). The treatments are very limited. Therefore, there is an urgent need for new therapies that help a larger fraction of the patient population and that are safe to use for a long time. Expression of TLR7/9 and type I interferons was found significantly up-regulated in PBMCs of patients with systemic lupus erythematosus (SLE) (Beverly D.LC, et al., Mol Immunol., 2014, 61:38-43). It was reported that overexpression of TLR7 could exacerbate autoimmune diseases and autoinflammations in mice (Santiago-Raber ML, et al., J Immunol., 2008, 181:1556-1562), while functional inhibition of TLR7/9 could ameliorate the pathology in lupus mice such as B6-*Fas$^{lpr}$* and BXSB mice (Dwight H.Kono, et al., PNAS, 2009, 106(29): 12061-12066). Given the close relationship of TLR7/8/9 to antinuclear antibodies and type I interferons, small-molecule inhibitors targeting TLR7/8/9 are very likely to have the potential to treat SLE.

Patent applications of disclosed TLR7/8/9 inhibitors include WO2019233941A1, WO2020020800 A1, WO2018049089A1, WO2017106607A1, CN109923108A, WO2020048605A1, and the like.

### SUMMARY

The present disclosure aims to provide a compound of general formula (I) or a pharmaceutically acceptable salt thereof:

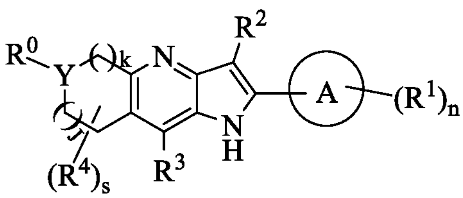

(I)

wherein:

Y is $CR^{4a}$ or a nitrogen atom;
ring A is selected from the group consisting of cycloalkyl, heterocyclyl, aryl, and heteroaryl;

$R^0$ is selected from the group consisting of a hydrogen atom, halogen, alkyl, alkenyl, alkynyl, heteroalkyl, alkoxy, haloalkyl, haloalkoxy, cyano, $-NR^gR^L$, nitro, hydroxy, hydroxyalkyl, and

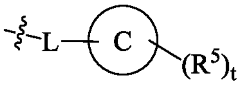

wherein the alkyl is optionally substituted with one or more substituents selected from the group consisting of alkoxy, haloalkoxy, cyano, $-NR^7R^8$, $-C(O)NR^7R^8$, cycloalkyl, heterocyclyl, aryl, and heteroaryl;

L is selected from the group consisting of a chemical bond, $NR^L$, an oxygen atom, a sulfur atom, and alkylene, wherein the alkylene is optionally substituted with one or more substituents selected from the group consisting of halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl;

$R^g$ and $R^L$ are identical or different and are each independently selected from the group consisting of a hydrogen atom, alkyl, alkenyl, alkynyl, haloalkyl, hydroxyalkyl, cycloalkyl, heterocyclyl, aryl, and heteroaryl;

ring C is selected from the group consisting of cycloalkyl, heterocyclyl, aryl, and heteroaryl;

each $R^1$ is identical or different and is independently selected from the group consisting of a hydrogen atom, halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl, wherein the alkyl, alkenyl, alkynyl, alkoxy, cycloalkyl, heterocyclyl, aryl, and heteroaryl are each independently and optionally substituted with one or more substituents selected from the group consisting of halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl;

$R^2$ is selected from the group consisting of a hydrogen atom, halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl, wherein the alkyl, alkenyl, alkynyl, alkoxy, cycloalkyl, heterocyclyl, aryl, and heteroaryl are each independently and optionally substituted with one or more substituents selected from the group consisting of halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl;

$R^3$ is selected from the group consisting of a hydrogen atom, halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl, wherein the alkyl, alkenyl, alkynyl, alkoxy, cycloalkyl, heterocyclyl, aryl, and heteroaryl are each independently and optionally substituted with one or more substituents selected from the group consisting of halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl;

each $R^4$ is identical or different and is independently selected from the group consisting of a hydrogen atom, halogen, alkyl, alkenyl, alkynyl, alkoxy, oxo, haloalkyl, haloalkoxy, cyano, amino, nitro, hydroxy, hydroxyalkyl, cycloalkyl, heterocyclyl, aryl, and heteroaryl, wherein the alkyl, alkenyl, alkynyl, alkoxy, cycloalkyl, heterocyclyl, aryl, and heteroaryl are each independently and optionally substituted with one or more substituents selected from the group consisting of halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl;

$R^{4a}$ is selected from the group consisting of a hydrogen atom, halogen, alkyl, alkenyl, alkynyl, alkoxy, haloalkyl, haloalkoxy, cyano, amino, nitro, hydroxy, and hydroxyalkyl; each $R^5$ is identical or different and is independently selected from the group consisting of a hydrogen atom, halogen, alkyl, alkenyl, alkynyl, alkoxy, oxo, haloalkyl, haloalkoxy, cyano, amino, $-NR^cR^d$, nitro, hydroxy, hydroxyalkyl, cycloalkyl, heterocyclyl, aryl, and heteroaryl, wherein the alkyl, alkenyl, alkynyl, alkoxy, cycloalkyl, heterocyclyl, aryl, and heteroaryl are each independently and optionally substituted with one or more substituents selected from the group consisting of halogen, alkyl, oxo, alkenyl, alkynyl, alkoxy, haloalkyl, haloalkoxy, cyano, amino, nitro, hydroxy, hydroxyalkyl, $-C(O)OR^6$, $-C(O)NR^7R^8$, $-NR^7R^8$, $-S(O)_2R^9$, cycloalkyl, heterocyclyl, aryl, and heteroaryl;

$R^6$ is selected from the group consisting of a hydrogen atom, alkyl, alkenyl, alkynyl, haloalkyl, hydroxyalkyl, cycloalkyl, heterocyclyl, aryl, and heteroaryl;

$R^c$, $R^d$, $R^7$, and $R^8$ are identical or different and are each independently selected from the group consisting of a hydrogen atom, alkyl, alkenyl, alkynyl, haloalkyl, hydroxyalkyl, cycloalkyl, heterocyclyl, aryl, and heteroaryl;

or $R^c$ and $R^d$, together with the nitrogen atom to which they are attached, form heterocyclyl, and the heterocyclyl is optionally substituted with one or more substituents selected from the group consisting of halogen, alkyl, oxo, alkenyl, alkynyl, alkoxy, haloalkyl, haloalkoxy, cyano, amino, nitro, hydroxy, hydroxyalkyl, cycloalkyl, heterocyclyl, aryl, and heteroaryl;

or $R^7$ and $R^8$, together with the nitrogen atom to which they are attached, form heterocyclyl, and the heterocyclyl is optionally substituted with one or more substituents selected from the group consisting of halogen, alkyl, oxo, alkenyl, alkynyl, alkoxy, haloalkyl, haloalkoxy, cyano, amino, nitro, hydroxy, hydroxyalkyl, cycloalkyl, heterocyclyl, aryl, and

heteroaryl;

$R^9$ is selected from the group consisting of a hydrogen atom, alkyl, alkenyl, alkynyl, haloalkyl, hydroxyalkyl, cyano, amino, hydroxy, cycloalkyl, heterocyclyl, aryl, and heteroaryl;

J is 0, 1, or 2;

k is 0, 1, or 2;

n is 0, 1, 2, 3, or 4;

s is 0, 1, 2, 3, or 4; and

t is 0, 1, 2, 3, or 4.

In some preferred embodiments of the present disclosure, the compound of general formula (I) or the pharmaceutically acceptable salt thereof is provided, wherein:

Y is $CR^{4a}$ or a nitrogen atom;

ring A is selected from the group consisting of cycloalkyl, heterocyclyl, aryl, and heteroaryl;

$R^0$ is selected from the group consisting of a hydrogen atom, halogen, alkyl, alkenyl, alkynyl, heteroalkyl, alkoxy, haloalkyl, haloalkoxy, cyano, $-NR^gR^L$, nitro, hydroxy, hydroxyalkyl, and

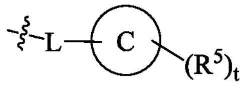;

L is selected from the group consisting of a chemical bond, $NR^L$, an oxygen atom, a sulfur atom, and alkylene, wherein the alkylene is optionally substituted with one or more substituents selected from the group consisting of halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl;

$R^g$ and $R^L$ are identical or different and are each independently selected from the group consisting of a hydrogen atom, alkyl, alkenyl, alkynyl, haloalkyl, hydroxyalkyl, cycloalkyl, heterocyclyl, aryl, and heteroaryl;

ring C is selected from the group consisting of cycloalkyl, heterocyclyl, aryl, and heteroaryl;

each $R^1$ is identical or different and is independently selected from the group consisting of a hydrogen atom, halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl, wherein the alkyl, alkenyl, alkynyl, alkoxy, cycloalkyl, heterocyclyl, aryl, and heteroaryl are each independently and optionally substituted with one or more substituents selected from the group consisting of halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl;

$R^2$ is selected from the group consisting of a hydrogen atom, halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl, wherein the alkyl, alkenyl, alkynyl, alkoxy, cycloalkyl, heterocyclyl, aryl, and heteroaryl are each independently and optionally substituted with one or more substituents selected from the group consisting of halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl;

$R^3$ is selected from the group consisting of a hydrogen atom, halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl, wherein the alkyl, alkenyl, alkynyl, alkoxy, cycloalkyl, heterocyclyl, aryl, and heteroaryl are each independently and optionally substituted with one or more substituents selected from the group consisting of halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl;

each $R^4$ is identical or different and is independently selected from the group consisting of a hydrogen atom, halogen, alkyl, alkenyl, alkynyl, alkoxy, oxo, haloalkyl, haloalkoxy, cyano, amino, nitro, hydroxy, hydroxyalkyl, cycloalkyl, heterocyclyl, aryl, and heteroaryl, wherein the alkyl, alkenyl, alkynyl, alkoxy, cycloalkyl, heterocyclyl, aryl, and heteroaryl are each independently and optionally substituted with one or more substituents selected from the group consisting of halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl;

$R^{4a}$ is selected from the group consisting of a hydrogen atom, halogen, alkyl, alkenyl, alkynyl, alkoxy, haloalkyl, haloalkoxy, cyano, amino, nitro, hydroxy, and hydroxyalkyl; each $R^5$ is identical or different and is independently selected from the group consisting of a hydrogen atom, halogen, alkyl, alkenyl, alkynyl, alkoxy, oxo, haloalkyl, haloalkoxy, cyano, amino, $-NR^cR^d$, nitro, hydroxy, hydroxyalkyl, cycloalkyl, heterocyclyl, aryl, and heteroaryl, wherein the alkyl, alkenyl, alkynyl, alkoxy, cycloalkyl, heterocyclyl, aryl, and heteroaryl are each independently and optionally substituted with one or more substituents selected from the group consisting of halogen, alkyl, oxo, alkenyl, alkynyl, alkoxy, haloalkyl, haloalkoxy, cyano, amino, nitro, hydroxy, hydroxyalkyl, $-C(O)OR^6$, $-C(O)NR^7R^8$, $-NR^7R^8$, $-S(O)_2R^9$,

cycloalkyl, heterocyclyl, aryl, and heteroaryl;

$R^6$ is selected from the group consisting of a hydrogen atom, alkyl, alkenyl, alkynyl, haloalkyl, hydroxyalkyl, cycloalkyl, heterocyclyl, aryl, and heteroaryl;

$R^c$, $R^d$, $R^7$, and $R^8$ are identical or different and are each independently selected from the group consisting of a hydrogen atom, alkyl, alkenyl, alkynyl, haloalkyl, hydroxyalkyl, cycloalkyl, heterocyclyl, aryl, and heteroaryl;

or $R^c$ and $R^d$, together with the nitrogen atom to which they are attached, form heterocyclyl, and the heterocyclyl is optionally substituted with one or more substituents selected from the group consisting of halogen, alkyl, oxo, alkenyl, alkynyl, alkoxy, haloalkyl, haloalkoxy, cyano, amino, nitro, hydroxy, hydroxyalkyl, cycloalkyl, heterocyclyl, aryl, and heteroaryl;

or $R^7$ and $R^8$, together with the nitrogen atom to which they are attached, form heterocyclyl, and the heterocyclyl is optionally substituted with one or more substituents selected from the group consisting of halogen, alkyl, oxo, alkenyl, alkynyl, alkoxy, haloalkyl, haloalkoxy, cyano, amino, nitro, hydroxy, hydroxyalkyl, cycloalkyl, heterocyclyl, aryl, and heteroaryl;

$R^9$ is selected from the group consisting of a hydrogen atom, alkyl, alkenyl, alkynyl, haloalkyl, hydroxyalkyl, cyano, amino, hydroxy, cycloalkyl, heterocyclyl, aryl, and heteroaryl;

J is 0, 1, or 2;

k is 0, 1, or 2;

n is 0, 1, 2, 3, or 4;

s is 0, 1, 2, 3, or 4; and

t is 0, 1, 2, 3, or 4.

In some preferred embodiments of the present disclosure, the compound of general formula (I) or the pharmaceutically acceptable salt thereof is provided, wherein:

Y is $CR^{4a}$ or a nitrogen atom;

ring A is selected from the group consisting of cycloalkyl, heterocyclyl, aryl, and heteroaryl;

$R^0$ is selected from the group consisting of a hydrogen atom, halogen, alkyl, alkenyl, alkynyl, heteroalkyl, alkoxy, haloalkyl, haloalkoxy, cyano, $-NR^gR^L$, nitro, hydroxy, hydroxyalkyl, and

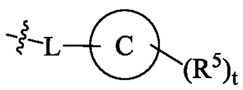
;

L is selected from the group consisting of a chemical bond, $NR^L$, an oxygen atom, a sulfur atom, and alkylene, wherein the alkylene is optionally substituted with one or more substituents selected from the group consisting of halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl;

$R^g$ and $R^L$ are identical or different and are each independently selected from the group consisting of a hydrogen atom, alkyl, alkenyl, alkynyl, haloalkyl, hydroxyalkyl, cycloalkyl, heterocyclyl, aryl, and heteroaryl;

ring C is selected from the group consisting of cycloalkyl, heterocyclyl, aryl, and heteroaryl;

each $R^1$ is identical or different and is independently selected from the group consisting of a hydrogen atom, halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl, wherein the alkyl, alkenyl, alkynyl, alkoxy, cycloalkyl, heterocyclyl, aryl, and heteroaryl are each independently and optionally substituted with one or more substituents selected from the group consisting of halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl;

$R^2$ is selected from the group consisting of a hydrogen atom, halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl, wherein the alkyl, alkenyl, alkynyl, alkoxy, cycloalkyl, heterocyclyl, aryl, and heteroaryl are each independently and optionally substituted with one or more substituents selected from the group consisting of halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl;

$R^3$ is selected from the group consisting of a hydrogen atom, halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl, wherein the alkyl, alkenyl, alkynyl, alkoxy, cycloalkyl, heterocyclyl, aryl, and heteroaryl are each independently and optionally substituted with one or more substituents selected from the group consisting of halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl;

each $R^4$ is identical or different and is independently selected from the group consisting of a hydrogen atom, halogen,

alkyl, alkenyl, alkynyl, alkoxy, oxo, haloalkyl, haloalkoxy, cyano, amino, nitro, hydroxy, hydroxyalkyl, cycloalkyl, heterocyclyl, aryl, and heteroaryl, wherein the alkyl, alkenyl, alkynyl, alkoxy, cycloalkyl, heterocyclyl, aryl, and heteroaryl are each independently and optionally substituted with one or more substituents selected from the group consisting of halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl;

$R^{4a}$ is selected from the group consisting of a hydrogen atom, halogen, alkyl, alkenyl, alkynyl, alkoxy, haloalkyl, haloalkoxy, cyano, amino, nitro, hydroxy, and hydroxyalkyl; each $R^5$ is identical or different and is independently selected from the group consisting of a hydrogen atom, halogen, alkyl, alkenyl, alkynyl, alkoxy, oxo, haloalkyl, haloalkoxy, cyano, amino, nitro, hydroxy, hydroxyalkyl, cycloalkyl, heterocyclyl, aryl, and heteroaryl, wherein the alkyl, alkenyl, alkynyl, alkoxy, cycloalkyl, heterocyclyl, aryl, and heteroaryl are each independently and optionally substituted with one or more substituents selected from the group consisting of halogen, alkyl, oxo, alkenyl, alkynyl, alkoxy, haloalkyl, haloalkoxy, cyano, amino, nitro, hydroxy, hydroxyalkyl, $-C(O)OR^6$, $-C(O)NR^7R^8$, $-NR^7R^8$, $-S(O)_2R^9$, cycloalkyl, heterocyclyl, aryl, and heteroaryl;

$R^6$ is selected from the group consisting of a hydrogen atom, alkyl, alkenyl, alkynyl, haloalkyl, hydroxyalkyl, cycloalkyl, heterocyclyl, aryl, and heteroaryl;

$R^7$ and $R^8$ are identical or different and are each independently selected from the group consisting of a hydrogen atom, alkyl, alkenyl, alkynyl, haloalkyl, hydroxyalkyl, cycloalkyl, heterocyclyl, aryl, and heteroaryl;

or $R^7$ and $R^8$, together with the nitrogen atom to which they are attached, form heterocyclyl, and the heterocyclyl is optionally substituted with one or more substituents selected from the group consisting of halogen, alkyl, oxo, alkenyl, alkynyl, alkoxy, haloalkyl, haloalkoxy, cyano, amino, nitro, hydroxy, hydroxyalkyl, cycloalkyl, heterocyclyl, aryl, and heteroaryl;

$R^9$ is selected from the group consisting of a hydrogen atom, alkyl, alkenyl, alkynyl, haloalkyl, hydroxyalkyl, cyano, amino, hydroxy, cycloalkyl, heterocyclyl, aryl, and heteroaryl;

J is 0, 1, or 2;

k is 0, 1, or 2;

n is 0, 1, 2, 3, or 4;

s is 0, 1, 2, 3, or 4; and

t is 0, 1, 2, 3, or 4.

In some preferred embodiments of the present disclosure, the compound of general formula (I) or the pharmaceutically acceptable salt thereof is provided, wherein each $R^5$ is identical or different and is independently selected from the group consisting of a hydrogen atom, halogen, alkyl, alkenyl, alkynyl, alkoxy, oxo, haloalkyl, haloalkoxy, cyano, amino, nitro, hydroxy, hydroxyalkyl, cycloalkyl, heterocyclyl, aryl, and heteroaryl, wherein the alkyl, alkenyl, alkynyl, alkoxy, cycloalkyl, heterocyclyl, aryl, and heteroaryl are each independently and optionally substituted with one or more substituents selected from the group consisting of halogen, alkyl, oxo, alkenyl, alkynyl, alkoxy, haloalkyl, haloalkoxy, cyano, amino, nitro, hydroxy, hydroxyalkyl, $-C(O)OR^6$, $-C(O)NR^7R^8$, $-NR^7R^8$, $-S(O)_2R^9$, cycloalkyl, heterocyclyl, aryl, and heteroaryl;

$R^6$ is selected from the group consisting of a hydrogen atom, alkyl, alkenyl, alkynyl, haloalkyl, hydroxyalkyl, cycloalkyl, heterocyclyl, aryl, and heteroaryl;

$R^7$ and $R^8$ are identical or different and are each independently selected from the group consisting of a hydrogen atom, alkyl, alkenyl, alkynyl, haloalkyl, hydroxyalkyl, cycloalkyl, heterocyclyl, aryl, and heteroaryl;

or $R^7$ and $R^8$, together with the nitrogen atom to which they are attached, form heterocyclyl, and the heterocyclyl is optionally substituted with one or more substituents selected from the group consisting of halogen, alkyl, oxo, alkenyl, alkynyl, alkoxy, haloalkyl, haloalkoxy, cyano, amino, nitro, hydroxy, hydroxyalkyl, cycloalkyl, heterocyclyl, aryl, and heteroaryl;

$R^9$ is selected from the group consisting of a hydrogen atom, alkyl, alkenyl, alkynyl, haloalkyl, hydroxyalkyl, cyano, amino, hydroxy, cycloalkyl, heterocyclyl, aryl, and heteroaryl.

In some preferred embodiments of the present disclosure, the compound of general formula (I) or the pharmaceutically acceptable salt thereof is a compound of general formula (II) or a pharmaceutically acceptable salt thereof:

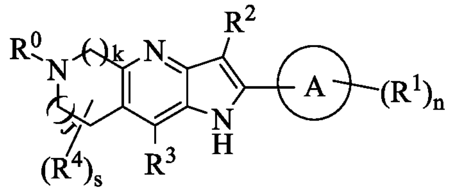

( II )

wherein:
ring A, $R^0$, $R^1$ to $R^4$, J, k, n, and s are as defined in general formula (I).

In some preferred embodiments of the present disclosure, the compound of general formula (I) or the pharmaceutically acceptable salt thereof is a compound of general formula (III) or a pharmaceutically acceptable salt thereof:

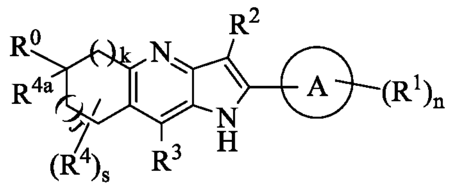

( III )

wherein:
ring A, $R^0$, $R^1$ to $R^4$, $R^{4a}$, J, k, n, and s are as defined in general formula (I).

In some preferred embodiments of the present disclosure, the compound of general formula (I) or general formula (III) or the pharmaceutically acceptable salt thereof is a compound of general formula (IV) or a pharmaceutically acceptable salt thereof:

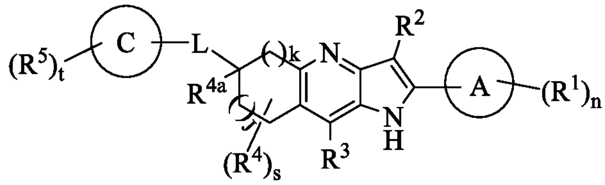

( IV )

wherein:
ring A, ring C, L, $R^1$ to $R^5$, $R^{4a}$, J, k, n, s, and t are as defined in general formula (I).

In some preferred embodiments of the present disclosure, the compound of general formula (I) or general formula (II) or the pharmaceutically acceptable salt thereof is a compound of general formula (IIG) or a pharmaceutically acceptable salt thereof:

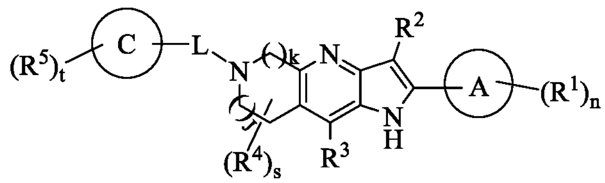

( IIG )

wherein:
ring A, ring C, L, $R^1$ to $R^5$, J, k, n, s, and t are as defined in general formula (I).

In some preferred embodiments of the present disclosure, the compound of general formula (I), general formula (II), general formula (IIG), general formula (III), or general formula (IV) or the pharmaceutically acceptable salt thereof

is provided, wherein each $R^5$ is identical or different and is independently selected from the group consisting of a hydrogen atom, halogen, $C_{1-6}$ alkyl, $C_{1-6}$ haloalkyl, $-NR^cR^d$, and 3- to 12-membered heterocyclyl, and $R^c$ and $R^d$ are identical or different and are each independently selected from the group consisting of a hydrogen atom, $C_{1-6}$ alkyl and 3- to 12-membered heterocyclyl; preferably, each $R^5$ is identical or different and is independently selected from the group consisting of a hydrogen atom, halogen, $C_{1-6}$ alkyl, $C_{1-6}$ haloalkyl, amino, and 3- to 6-membered heterocyclyl; more preferably, each $R^5$ is identical or different and is independently selected from the group consisting of a hydrogen atom, amino, and piperazinyl.

In some preferred embodiments of the present disclosure, the compound of general formula (I), general formula (II), or general formula (IIG) or the pharmaceutically acceptable salt thereof is a compound of general formula (V) or a pharmaceutically acceptable salt thereof:

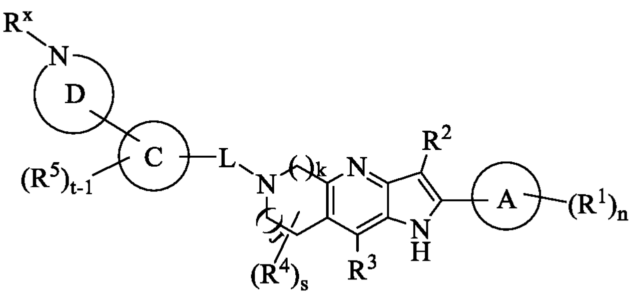

(V)

wherein:

ring D is 4- to 12-membered heterocyclyl containing at least one nitrogen atom;
$R^x$ is selected from the group consisting of a hydrogen atom, $C_{1-6}$ alkyl, and $C_{1-6}$ haloalkyl;
t is 1, 2, 3, or 4;
ring A, ring C, L, $R^1$ to $R^5$, J, k, n, and s are as defined in general formula (I).

In some preferred embodiments of the present disclosure, the compound of general formula (I), general formula (II), or general formula (IIG) or the pharmaceutically acceptable salt thereof is a compound of general formula (VI) or a pharmaceutically acceptable salt thereof:

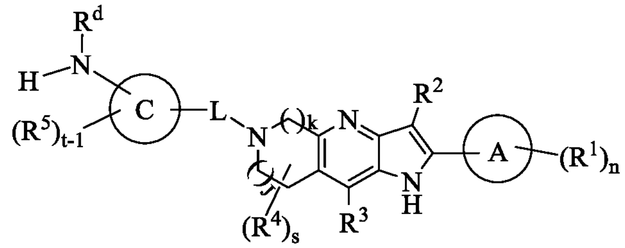

(VI)

wherein:

t is 1, 2, 3, or 4;
ring A, ring C, L, $R^1$ to $R^5$, $R^d$, J, k, n, and s are as defined in general formula (I).

In some preferred embodiments of the present disclosure, the compound of general formula (I), general formula (II), general formula (IIG), general formula (III), general formula (IV), general formula (V), or general formula (VI) or the pharmaceutically acceptable salt thereof is provided, wherein ring A is 6- to 10-membered aryl or 5- to 10-membered heteroaryl; preferably, ring A is pyridinyl or

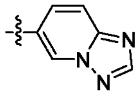;

more preferably, ring A is pyridinyl.

In some preferred embodiments of the present disclosure, the compound of general formula (I), general formula (II), general formula (IIG), general formula (III), general formula (IV), general formula (V), or general formula (VI) or the pharmaceutically acceptable salt thereof is provided, wherein J is 0 or 1, and k is 1 or 2; preferably, J is 1, and k is 1.

In some preferred embodiments of the present disclosure, the compound of general formula (I), general formula (II), or general formula (III) or the pharmaceutically acceptable salt thereof is provided, wherein $R^0$ is selected from the group consisting of a hydrogen atom, $C_{1-6}$ alkyl, $-NR^gR^L$, and

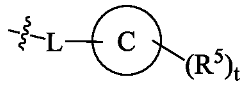,

wherein the $C_{1-6}$ alkyl is optionally substituted with one or more $-C(O)NR^7R^8$, and L, ring C, $R^5$, $R^7$, $R^8$, $R^g$, $R^L$, and t are as defined in general formula (I); preferably, $R^0$ is a hydrogen atom or

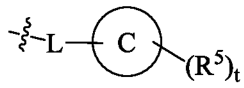,

L, ring C, $R^5$, and t are as defined in general formula (I); most preferably, $R^0$ is a hydrogen atom.

In some preferred embodiments of the present disclosure, the compound of general formula (I) or the pharmaceutically acceptable salt thereof is provided, wherein when Y is a nitrogen atom, and $R^0$ is selected from the group consisting of a hydrogen atom, $C_{1-6}$ alkyl, and

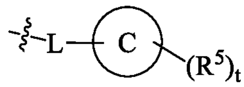,

wherein the $C_{1-6}$ alkyl is optionally substituted with one or more $-C(O)NR^7R^8$; when Y is $CR^{4a}$, $R^0$ is $-NR^gR^L$ or

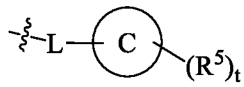,

and L, ring C, $R^{4a}$, $R^5$, $R^7$, $R^8$, $R^g$, $R^L$, and t are as defined in general formula (I).

In some preferred embodiments of the present disclosure, the compound of general formula (I), general formula (II), or general formula (III) or the pharmaceutically acceptable salt thereof is provided, wherein $R^0$ is selected from the group consisting of a hydrogen atom, $C_{1-6}$ alkyl, $-NR^gR^L$, and

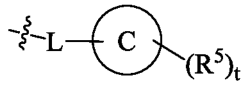,

and L, ring C, $R^5$, $R^g$, $R^L$, and t are as defined in general formula (I).

In some preferred embodiments of the present disclosure, the compound of general formula (I) or the pharmaceutically acceptable salt thereof is provided, wherein when Y is a nitrogen atom, $R^0$ is selected from the group consisting of a hydrogen atom, $C_{1-6}$ alkyl, and

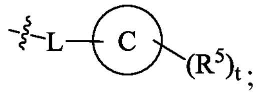

when Y is $CR^{4a}$, $R^0$ is $-NR^gR^L$ or

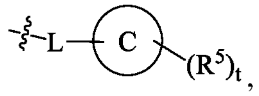

and L, ring C, $R^{4a}$, $R^5$, $R^g$, $R^L$, and t are as defined in general formula (I).

In some preferred embodiments of the present disclosure, when Y is a nitrogen atom, $R^0$ is a hydrogen atom or $C_{1-6}$ alkyl; when Y is $CR^{4a}$, $R^0$ is $-NR^gR^L$ or

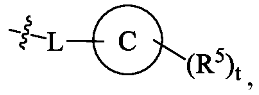

and L, ring C, $R^{4a}$, $R^5$, $R^g$, $R^L$, and t are as defined in general formula (I).

In some preferred embodiments of the present disclosure, the compound of general formula (II) or the pharmaceutically acceptable salt thereof is provided, wherein $R^0$ is selected from the group consisting of a hydrogen atom, $C_{1-6}$ alkyl, and

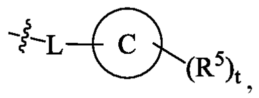

wherein the $C_{1-6}$ alkyl is optionally substituted with one or more $-C(O)NR^7R^8$, and L, ring C, $R^5$, $R^7$, $R^8$, and t are as defined in general formula (I); preferably, $R^0$ is a hydrogen atom or

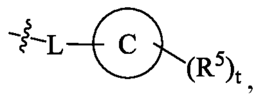

and L, ring C, $R^5$, and t are as defined in general formula (I); most preferably, $R^0$ is a hydrogen atom.

In some preferred embodiments of the present disclosure, the compound of general formula (II) or the pharmaceutically acceptable salt thereof is provided, wherein $R^0$ is selected from the group consisting of a hydrogen atom, $C_{1-6}$ alkyl, and

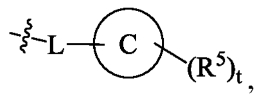

and L, ring C, $R^5$, and t are as defined in general formula (I).

In some preferred embodiments of the present disclosure, the compound of general formula (III) or the pharmaceutically acceptable salt thereof is provided, wherein $R^0$ is $-NR^gR^L$ or

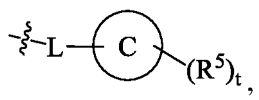

and L, ring C, $R^5$, $R^g$, $R^L$, and t are as defined in general formula (I).

In some preferred embodiments of the present disclosure, the compound of general formula (I), general formula (II), or general formula (III) or the pharmaceutically acceptable salt thereof is provided, wherein $R^7$ and $R^8$ are identical or different and are each independently a hydrogen atom or $C_{1-6}$ alkyl; preferably, $R^7$ and $R^8$ are both hydrogen atoms.

In some preferred embodiments of the present disclosure, the compound of general formula (I) or general formula (III) or the pharmaceutically acceptable salt thereof is provided, wherein $R^g$ is a hydrogen atom, and $R^L$ is a hydrogen atom or $C_{1-6}$ alkyl; preferably, $R^g$ and $R^L$ are both hydrogen atoms.

In some preferred embodiments of the present disclosure, the compound of general formula (I), general formula (II), general formula (IIG), general formula (III), general formula (IV), general formula (V), or general formula (VI) or the pharmaceutically acceptable salt thereof is provided, wherein L is $NR^L$ or $C_{1-6}$ alkylene, $R^L$ is a hydrogen atom or $C_{1-6}$ alkyl; preferably, L is $NR^L$ or $C_{1-6}$ alkylene, and $R^L$ is a hydrogen atom.

In some preferred embodiments of the present disclosure, the compound of general formula (I), general formula (II), general formula (IIG), general formula (III), or general formula (IV) or the pharmaceutically acceptable salt thereof is provided, wherein ring C is selected from the group consisting of 3- to 8-membered cycloalkyl, 3- to 8-membered heterocyclyl, 6- to 10-membered aryl, and 5- to 10-membered heteroaryl; preferably, ring C is selected from the group consisting of 3- to 8-membered cycloalkyl, 3- to 6-membered heterocyclyl, and 5- or 6-membered heteroaryl; more preferably, ring C is selected from the group consisting of tetrahydrofuranyl, bicyclo[2.2.2]octyl and pyridinyl; most preferably, ring C is bicyclo[2.2.2]octyl or pyridinyl.

In some preferred embodiments of the present disclosure, the compound of general formula (I), general formula (II), general formula (IIG), general formula (III), or general formula (IV) or the pharmaceutically acceptable salt thereof is provided, wherein L is $NR^L$ or $C_{1-6}$ alkylene; $R^L$ is a hydrogen atom or $C_{1-6}$ alkyl; and/or ring C is selected from the group consisting of 3- to 8-membered cycloalkyl, 3- to 8-membered heterocyclyl, 6-to 10-membered aryl, and 5- to 10-membered heteroaryl.

In some preferred embodiments of the present disclosure, the compound of general formula (I), general formula (II), general formula (IIG), general formula (V), or general formula (VI) or the pharmaceutically acceptable salt thereof is provided, wherein L is $C_{1-6}$ alkylene.

In some preferred embodiments of the present disclosure, the compound of general formula (V) or the pharmaceutically acceptable salt thereof is provided, wherein ring C is 5- to 10-membered heteroaryl; preferably, ring C is pyridinyl.

In some preferred embodiments of the present disclosure, the compound of general formula (VI) or the pharmaceutically acceptable salt thereof is provided, wherein ring C is 3- to 8-membered cycloalkyl; preferably, ring C is bicyclo[2.2.2]octyl.

In some preferred embodiments of the present disclosure, the compound of general formula (I), general formula (III), or general formula (IV) or the pharmaceutically acceptable salt thereof is provided, wherein L is $NR^L$, and $R^L$ is a hydrogen atom or $C_{1-6}$ alkyl; preferably, L is $NR^L$, and $R^L$ is a hydrogen atom.

In some preferred embodiments of the present disclosure, the compound of general formula (I), general formula (III), or general formula (IV) or the pharmaceutically acceptable salt thereof is provided, wherein ring C is 3- to 8-membered heterocyclyl; preferably, ring C is 3- to 6-membered heterocyclyl; more preferably, ring C is tetrahydrofuranyl.

In some preferred embodiments of the present disclosure, the compound of general formula (I), general formula (III), or general formula (IV) or the pharmaceutically acceptable salt thereof is provided, wherein L is $NR^L$, and $R^L$ is a hydrogen atom or $C_{1-6}$ alkyl; and/or ring C is 3- to 8-membered heterocyclyl.

In some preferred embodiments of the present disclosure, the compound of general formula (I), general formula (II), general formula (IIG), general formula (III), general formula (IV), general formula (V), or general formula (VI) or the pharmaceutically acceptable salt thereof is provided, wherein each $R^1$ is each identical or different and is independently selected from the group consisting of a hydrogen atom, halogen, $C_{1-6}$ alkyl, and $C_{1-6}$ haloalkyl; preferably, each $R^1$ is identical or different and is independently a hydrogen atom or $C_{1-6}$ alkyl.

In some preferred embodiments of the present disclosure, the compound of general formula (I), general formula (II), general formula (IIG), general formula (III), general formula (IV), general formula (V), or general formula (VI) or the pharmaceutically acceptable salt thereof is provided, wherein $R^2$ is selected from the group consisting of a hydrogen atom, halogen, $C_{1-6}$ alkyl, $C_{1-6}$ haloalkyl, and 3- to 12-membered heterocyclyl; preferably, $R^2$ is $C_{1-6}$ alkyl or 3- to 6-membered heterocyclyl; more preferably, $R^2$ is isopropyl or tetrahydropyranyl (for example,

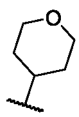 ).

In some preferred embodiments of the present disclosure, the compound of general formula (I), general formula

(II), general formula (IIG), general formula (III), general formula (IV), general formula (V), or general formula (VI) or the pharmaceutically acceptable salt thereof is provided, wherein $R^2$ is selected from the group consisting of a hydrogen atom, halogen, $C_{1-6}$ alkyl, and $C_{1-6}$ haloalkyl; preferably, $R^2$ is $C_{1-6}$ alkyl.

In some preferred embodiments of the present disclosure, the compound of general formula (I), general formula (II), general formula (IIG), general formula (III), general formula (IV), general formula (V), or general formula (VI) or the pharmaceutically acceptable salt thereof is provided, wherein $R^3$ is selected from the group consisting of a hydrogen atom, halogen, $C_{1-6}$ alkyl, and $C_{1-6}$ haloalkyl; preferably, $R^3$ is a hydrogen atom.

In some preferred embodiments of the present disclosure, the compound of general formula (I), general formula (II), general formula (IIG), general formula (III), general formula (IV), general formula (V), or general formula (VI) or the pharmaceutically acceptable salt thereof is provided, wherein each $R^4$ is identical or different and is independently a hydrogen atom or $C_{1-6}$ alkyl; preferably, $R^4$ is a hydrogen atom.

In some preferred embodiments of the present disclosure, the compound of general formula (I), general formula (III), or general formula (IV) or the pharmaceutically acceptable salt thereof is provided, wherein $R^{4a}$ is a hydrogen atom.

In some preferred embodiments of the present disclosure, the compound of general formula (I), general formula (II), general formula (IIG), general formula (III), or general formula (IV) or the pharmaceutically acceptable salt thereof is provided, wherein each $R^5$ is identical or different and is independently selected from the group consisting of a hydrogen atom, halogen, $C_{1-6}$ alkyl, $C_{1-6}$ haloalkyl, $-NR^cR^d$, and 3- to 12-membered heterocyclyl, and $R^c$ and $R^d$ are identical or different and are each independently selected from the group consisting of a hydrogen atom, $C_{1-6}$ alkyl, and 3- to 12-membered heterocyclyl; preferably, each $R^5$ is identical or different and is independently selected from the group consisting of a hydrogen atom, $-NR^cR^d$, and 3- to 6-membered heterocyclyl, $R^c$ and $R^d$ are identical or different and are each independently a hydrogen atom or $C_{1-6}$ alkyl; more preferably, each $R^5$ is identical or different and is independently selected from the group consisting of a hydrogen atom, amino, and piperazinyl.

In some preferred embodiments of the present disclosure, the compound of general formula (I), general formula (II), general formula (IIG), general formula (III), or general formula (IV) or the pharmaceutically acceptable salt thereof is provided, wherein each $R^5$ is identical or different and is independently selected from the group consisting of a hydrogen atom, halogen, $C_{1-6}$ alkyl, $C_{1-6}$ haloalkyl, and 3- to 12-membered heterocyclyl; preferably, each $R^5$ is identical or different and is independently a hydrogen atom or 3-to 6-membered heterocyclyl; more preferably, each $R^5$ is identical or different and is independently a hydrogen atom or piperazinyl.

In some preferred embodiments of the present disclosure, the compound of general formula (V) or the pharmaceutically acceptable salt thereof is provided, wherein each $R^5$ is identical or different and is independently selected from the group consisting of a hydrogen atom, halogen, $C_{1-6}$ alkyl, and $C_{1-6}$ haloalkyl; preferably, $R^5$ is a hydrogen atom.

In some preferred embodiments of the present disclosure, the compound of general formula (VI) or the pharmaceutically acceptable salt thereof is provided, wherein each $R^5$ is identical or different and is independently selected from the group consisting of a hydrogen atom, halogen, $C_{1-6}$ alkyl, and $C_{1-6}$ haloalkyl; preferably, $R^5$ is a hydrogen atom.

In some preferred embodiments of the present disclosure, the compound of general formula (I), general formula (II), general formula (III), or general formula (IV) or the pharmaceutically acceptable salt thereof is provided, wherein each $R^5$ is identical or different and is independently selected from the group consisting of a hydrogen atom, halogen, $C_{1-6}$ alkyl, and $C_{1-6}$ haloalkyl; preferably, $R^5$ is a hydrogen atom.

In some preferred embodiments of the present disclosure, the compound of general formula (I), general formula (II), general formula (IIG), general formula (III), general formula (IV), general formula (V), or general formula (VI) or the pharmaceutically acceptable salt thereof is provided, wherein n is 0, 1, 2, 3, or 4; preferably, n is 0, 1, or 2.

In some preferred embodiments of the present disclosure, the compound of general formula (I), general formula (II), general formula (IIG), general formula (III), general formula (IV), general formula (V), or general formula (VI) or the pharmaceutically acceptable salt thereof is provided, wherein s is 0, 1, 2, 3, or 4; preferably, s is 0 or 1; more preferably, s is 0.

In some preferred embodiments of the present disclosure, the compound of general formula (I), general formula (II), general formula (IIG), general formula (III), or general formula (IV) or the pharmaceutically acceptable salt thereof is provided, wherein t is 0, 1, 2, 3, or 4; preferably, t is 0, 1, or 2.

In some preferred embodiments of the present disclosure, the compound of general formula (V) or general formula (VI) or the pharmaceutically acceptable salt thereof is provided, wherein t is 1, 2, 3, or 4; preferably, t is 1 or 2; more preferably, t is 1.

In some preferred embodiments of the present disclosure, the compound of general formula (V) or the pharmaceutically acceptable salt thereof is provided, wherein ring D is 4- to 12-membered heterocyclyl containing at least one nitrogen atom; preferably, ring D is 4- to 6-membered heterocyclyl containing at least one nitrogen atom; more preferably, ring D is piperazinyl.

In some preferred embodiments of the present disclosure, the compound of general formula (VI) or the pharmaceutically acceptable salt thereof is provided, wherein $R^d$ is selected from the group consisting of a hydrogen atom, $C_{1-6}$ alkyl, and 3- to 12-membered heterocyclyl; preferably, $R^d$ is a hydrogen atom.

In some preferred embodiments of the present disclosure, the compound of general formula (I), general formula (II), general formula (IIG), general formula (III), or general formula (IV) or the pharmaceutically acceptable salt thereof is provided, wherein each $R^5$ is identical or different and is independently selected from the group consisting of halogen, $C_{1-6}$ alkyl, $C_{1-6}$ haloalkyl, $-NR^cR^d$, and 3- to 12-membered heterocyclyl; $R^c$ and $R^d$ are identical or different and are each independently selected from the group consisting of a hydrogen atom, $C_{1-6}$ alkyl, and 3- to 12-membered heterocyclyl; t is 0, 1, 2, 3, or 4.

In some preferred embodiments of the present disclosure, the compound of general formula (I), general formula (II), general formula (IIG), general formula (III), or general formula (IV) or the pharmaceutically acceptable salt thereof is provided, wherein each $R^5$ is identical or different and is independently selected from the group consisting of halogen, $C_{1-6}$ alkyl, $C_{1-6}$ haloalkyl, $-NR^cR^d$, and 3- to 12-membered heterocyclyl; $R^c$ and $R^d$ are identical or different and are each independently selected from the group consisting of a hydrogen atom, $C_{1-6}$ alkyl, and 3- to 12-membered heterocyclyl; t is 0, 1, or 2.

In some preferred embodiments of the present disclosure, the compound of general formula (I), general formula (II), general formula (IIG), general formula (III), or general formula (IV) or the pharmaceutically acceptable salt thereof is provided, wherein each $R^5$ is identical or different and is independently selected from the group consisting of halogen, $C_{1-6}$ alkyl, $C_{1-6}$ haloalkyl, amino, and 3- to 6-membered heterocyclyl; t is 0, 1, or 2.

In some preferred embodiments of the present disclosure, the compound of general formula (I), general formula (II), general formula (IIG), general formula (III), or general formula (IV) or the pharmaceutically acceptable salt thereof is provided, wherein each $R^5$ is identical or different and is independently $-NR^cR^d$ or 3- to 6-membered heterocyclyl; $R^c$ and $R^d$ are identical or different and are each independently a hydrogen atom or $C_{1-6}$ alkyl; t is 1.

In some preferred embodiments of the present disclosure, the compound of general formula (I), general formula (II), general formula (IIG), general formula (III), general formula (IV), general formula (V), or general formula (VI) or the pharmaceutically acceptable salt thereof is provided, wherein each $R^1$ is identical or different and is independently selected from the group consisting of halogen, $C_{1-6}$ alkyl, and $C_{1-6}$ haloalkyl; n is 0, 1, or 2.

In some preferred embodiments of the present disclosure, the compound of general formula (I), general formula (II), general formula (IIG), general formula (III), general formula (IV), general formula (V), or general formula (VI) or the pharmaceutically acceptable salt thereof is provided, wherein $R^1$ is $C_{1-6}$ alkyl; n is 0, 1, or 2.

In some preferred embodiments of the present disclosure, the compound of general formula (I), general formula (II), general formula (IIG), general formula (III), general formula (IV), general formula (V), or general formula (VI) or the pharmaceutically acceptable salt thereof is provided, wherein each $R^4$ is identical or different and is independently $C_{1-6}$ alkyl; s is 0, 1, 2, 3, or 4.

In some preferred embodiments of the present disclosure, the compound of general formula (I), general formula (II), general formula (IIG), general formula (III), general formula (IV), general formula (V), or general formula (VI) or the pharmaceutically acceptable salt thereof is provided, wherein each $R^4$ is identical or different and is independently $C_{1-6}$ alkyl; s is 0 or 1.

In some preferred embodiments of the present disclosure, the compound of general formula (I) or the pharmaceutically acceptable salt thereof is provided, wherein:

ring A is 6- to 10-membered aryl or 5- to 10-membered heteroaryl;
Y is $CR^{4a}$ or a nitrogen atom;
$R^{4a}$ is a hydrogen atom;
$R^0$ is selected from the group consisting of a hydrogen atom, $C_{1-6}$ alkyl, $-NR^gR^L$, and

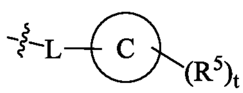,

wherein the $C_{1-6}$ alkyl is optionally substituted with one or more $-C(O)NR^7R^8$;
L is $C_{1-6}$ alkylene or $NR^L$;
$R^g$ is a hydrogen atom;
$R^L$ is a hydrogen atom or $C_{1-6}$ alkyl;
ring C is selected from the group consisting of 3- to 8-membered cycloalkyl, 3- to 8-membered heterocyclyl, 6- to 10-membered aryl, and 5- to 10-membered heteroaryl; each $R^5$ is identical or different and is independently selected from the group consisting of halogen, $C_{1-6}$ alkyl, $C_{1-6}$ haloalkyl, $-NR^cR^d$, and 3- to 12-membered heterocyclyl;
$R^c$ and $R^d$ are identical or different and are each independently selected from the group consisting of a hydrogen atom, $C_{1-6}$ alkyl, and 3- to 12-membered heterocyclyl;
each $R^1$ is identical or different and is independently selected from the group consisting of halogen, $C_{1-6}$ alkyl, and

$C_{1-6}$ haloalkyl;
$R^2$ is selected from the group consisting of a hydrogen atom, halogen, $C_{1-6}$ alkyl, $C_{1-6}$ haloalkyl, and 3- to 12-membered heterocyclyl;
$R^3$ is selected from the group consisting of a hydrogen atom, halogen, $C_{1-6}$ alkyl, and $C_{1-6}$ haloalkyl;
each $R^4$ is identical or different and is independently $C_{1-6}$ alkyl;
$R^7$ and $R^8$ are identical or different and are each independently a hydrogen atom or $C_{1-6}$ alkyl;
J is 1, and k is 1;
n is 0, 1, or 2;
s is 0, 1, 2, 3, or 4; and
t is 0, 1, 2, 3, or 4.

In some preferred embodiments of the present disclosure, the compound of general formula (I) or the pharmaceutically acceptable salt thereof is provided, wherein:

ring A is 6- to 10-membered aryl or 5- to 10-membered heteroaryl;
Y is $CR^{4a}$ or a nitrogen atom;
$R^{4a}$ is a hydrogen atom;
$R^0$ is selected from the group consisting of a hydrogen atom, $C_{1-6}$ alkyl, $-NR^gR^L$ and

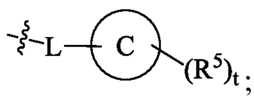

L is $C_{1-6}$ alkylene or $NR^L$;
$R^g$ is a hydrogen atom;
$R^L$ is a hydrogen atom or $C_{1-6}$ alkyl;
ring C is selected from the group consisting of 3- to 8-membered cycloalkyl, 3- to 8-membered heterocyclyl, 6- to 10-membered aryl, and 5- to 10-membered heteroaryl;
each $R^5$ is identical or different and is independently selected from the group consisting of halogen, $C_{1-6}$ alkyl, $C_{1-6}$ haloalkyl, $-NR^cR^d$, and 3- to 12-membered heterocyclyl, and $R^c$ and $R^d$ are identical or different and are each independently selected from the group consisting of a hydrogen atom, $C_{1-6}$ alkyl, and 3- to 12-membered heterocyclyl;
each $R^1$ is identical or different and is independently selected from the group consisting of halogen, $C_{1-6}$ alkyl, and $C_{1-6}$ haloalkyl;
$R^2$ is selected from the group consisting of a hydrogen atom, halogen, $C_{1-6}$ alkyl, $C_{1-6}$ haloalkyl, and 3- to 12-membered heterocyclyl;
$R^3$ is selected from the group consisting of a hydrogen atom, halogen, $C_{1-6}$ alkyl, and $C_{1-6}$ haloalkyl;
each $R^4$ is identical or different and is independently $C_{1-6}$ alkyl;
J is 1, and k is 1;
n is 0, 1, or 2;
s is 0, 1, 2, 3, or 4; and
t is 0, 1, 2, 3, or 4.

In some preferred embodiments of the present disclosure, the compound of general formula (I) or the pharmaceutically acceptable salt thereof is provided, wherein:

ring A is 6- to 10-membered aryl or 5- to 10-membered heteroaryl;
Y is $CR^{4a}$ or a nitrogen atom;
$R^{4a}$ is a hydrogen atom;
$R^0$ is selected from the group consisting of a hydrogen atom, $C_{1-6}$ alkyl, $-NR^gR^L$ and

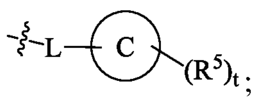

L is $C_{1-6}$ alkylene or $NR^L$;
$R^g$ is a hydrogen atom;

$R^L$ is a hydrogen atom or $C_{1-6}$ alkyl;
ring C is 5- to 10-membered heteroaryl or 3- to 8-membered heterocyclyl;
each $R^5$ is identical or different and is independently selected from the group consisting of halogen, $C_{1-6}$ alkyl, $C_{1-6}$ haloalkyl, and 3- to 12-membered heterocyclyl;
each $R^1$ is identical or different and is independently selected from the group consisting of halogen, $C_{1-6}$ alkyl, and $C_{1-6}$ haloalkyl;
$R^2$ is selected from the group consisting of a hydrogen atom, halogen, $C_{1-6}$ alkyl, $C_{1-6}$ haloalkyl, and 3- to 12-membered heterocyclyl;
$R^3$ is selected from the group consisting of a hydrogen atom, halogen, $C_{1-6}$ alkyl, and $C_{1-6}$ haloalkyl;
each $R^4$ is identical or different and is independently $C_{1-6}$ alkyl;
J is 1, and k is 1;
n is 0, 1, or 2;
s is 0, 1, 2, 3, or 4; and
t is 0, 1, 2, 3, or 4.

In some preferred embodiments of the present disclosure, the compound of general formula (I) or the pharmaceutically acceptable salt thereof is provided, wherein:

ring A is 6- to 10-membered aryl or 5- to 10-membered heteroaryl;
Y is $CR^{4a}$ or a nitrogen atom;
$R^{4a}$ is a hydrogen atom;
$R^0$ is selected from the group consisting of a hydrogen atom, $C_{1-6}$ alkyl, $-NR^gR^L$ and

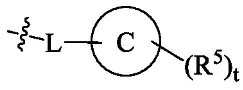
;

L is $NR^L$;
$R^g$ is a hydrogen atom;
$R^L$ is a hydrogen atom or $C_{1-6}$ alkyl;
ring C is 3- to 8-membered heterocyclyl;
each $R^5$ is identical or different and is independently selected from the group consisting of halogen, $C_{1-6}$ alkyl, and $C_{1-6}$ haloalkyl;
each $R^1$ is identical or different and is independently selected from the group consisting of halogen, $C_{1-6}$ alkyl, and $C_{1-6}$ haloalkyl;
$R^2$ is selected from the group consisting of a hydrogen atom, halogen, $C_{1-6}$ alkyl, and $C_{1-6}$ haloalkyl;
$R^3$ is selected from the group consisting of a hydrogen atom, halogen, $C_{1-6}$ alkyl, and $C_{1-6}$ haloalkyl;
each $R^4$ is identical or different and is independently $C_{1-6}$ alkyl;
J is 1, and k is 1;
n is 0, 1, or 2;
s is 0, 1, 2, 3, or 4; and
t is 0, 1, 2, 3, or 4.

In some preferred embodiments of the present disclosure, the compound of general formula (II) or the pharmaceutically acceptable salt thereof is provided, wherein:

ring A is pyridinyl or

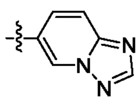
;

$R^0$ is selected from the group consisting of a hydrogen atom, $C_{1-6}$ alkyl, and

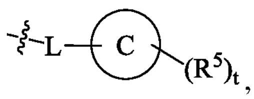

wherein the $C_{1-6}$ alkyl is optionally substituted with one or more $-C(O)NR^7R^8$;
L is $C_{1-6}$ alkylene;
ring C is selected from the group consisting of 3- to 8-membered cycloalkyl, 3- to 8-membered heterocyclyl, 6- to 10-membered aryl, and 5- to 10-membered heteroaryl; each $R^5$ is identical or different and is independently selected from the group consisting of halogen, $C_{1-6}$ alkyl, $C_{1-6}$ haloalkyl, $-NR^cR^d$, and 3- to 12-membered heterocyclyl;
$R^c$ and $R^d$ are identical or different and are each independently selected from the group consisting of a hydrogen atom, $C_{1-6}$ alkyl, and 3- to 12-membered heterocyclyl;
each $R^1$ is identical or different and is independently selected from the group consisting of halogen, $C_{1-6}$ alkyl, and $C_{1-6}$ haloalkyl;
$R^2$ is selected from the group consisting of a hydrogen atom, halogen, $C_{1-6}$ alkyl, $C_{1-6}$ haloalkyl, and 3- to 12-membered heterocyclyl;
$R^3$ is selected from the group consisting of a hydrogen atom, halogen, $C_{1-6}$ alkyl, and $C_{1-6}$ haloalkyl;
each $R^4$ is identical or different and is independently $C_{1-6}$ alkyl;
$R^7$ and $R^8$ are identical or different and are each independently a hydrogen atom or $C_{1-6}$ alkyl;
J is 1, and k is 1;
n is 0, 1, or 2;
s is 0 or 1; and
t is 0, 1, or 2.

In some preferred embodiments of the present disclosure, the compound of general formula (II) or the pharmaceutically acceptable salt thereof is provided, wherein:

ring A is pyridinyl or

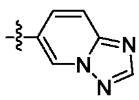
;

$R^0$ is selected from the group consisting of a hydrogen atom, $C_{1-6}$ alkyl, and

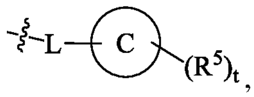

wherein the $C_{1-6}$ alkyl is optionally substituted with one or more $-C(O)NR^7R^8$;
L is $C_{1-6}$ alkylene;
ring C is selected from the group consisting of tetrahydrofuranyl, bicyclo[2.2.2]octyl, and pyridinyl;
each $R^5$ is identical or different and is independently $-NR^cR^d$ or 3- to 6-membered heterocyclyl;
$R^c$ and $R^d$ are identical or different and are each independently a hydrogen atom or $C_{1-6}$ alkyl;
$R^1$ is $C_{1-6}$ alkyl;
$R^2$ is $C_{1-6}$ alkyl or 3- to 6-membered heterocyclyl;
$R^3$ is a hydrogen atom;
$R^7$ and $R^8$ are identical or different and are each independently a hydrogen atom or $C_{1-6}$ alkyl;
J is 1, and k is 1;
n is 0, 1, or 2;
s is 0; and
t is 1.

In some preferred embodiments of the present disclosure, the compound of general formula (II) or the pharmaceutically acceptable salt thereof is provided, wherein:

ring A is pyridinyl;
$R^0$ is selected from the group consisting of a hydrogen atom, $C_{1-6}$ alkyl, and

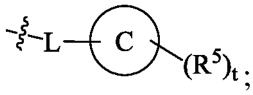

L is $C_{1-6}$ alkylene;
ring C is selected from the group consisting of 3- to 8-membered cycloalkyl, 3- to 8-membered heterocyclyl, and 5- to 10-membered heteroaryl;
each $R^5$ is identical or different and is independently selected from the group consisting of a hydrogen atom, halogen, $C_{1-6}$ alkyl, $C_{1-6}$ haloalkyl, amino, and 3- to 6-membered heterocyclyl;
each $R^1$ is identical or different and is independently selected from the group consisting of a hydrogen atom, halogen, $C_{1-6}$ alkyl, and $C_{1-6}$ haloalkyl;
$R^2$ is selected from the group consisting of a hydrogen atom, halogen, $C_{1-6}$ alkyl, $C_{1-6}$ haloalkyl, and 3- to 12-membered heterocyclyl;
$R^3$ is selected from the group consisting of a hydrogen atom, halogen, $C_{1-6}$ alkyl, and $C_{1-6}$ haloalkyl;
each $R^4$ is identical or different and is independently a hydrogen atom or $C_{1-6}$ alkyl;
J is 1, and k is 1;
n is 0, 1, or 2;
s is 1; and
t is 2.

In some preferred embodiments of the present disclosure, the compound of general formula (II) or the pharmaceutically acceptable salt thereof is provided, wherein:

ring A is pyridinyl;
$R^0$ is selected from the group consisting of a hydrogen atom, $C_{1-6}$ alkyl, and

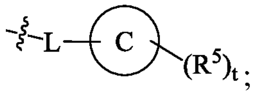

L is $C_{1-6}$ alkylene;
ring C is 5- to 10-membered heteroaryl;
each $R^5$ is identical or different and is independently selected from the group consisting of a hydrogen atom, halogen, $C_{1-6}$ alkyl, $C_{1-6}$ haloalkyl, and 3- to 12-membered heterocyclyl;
each $R^1$ is identical or different and is independently selected from the group consisting of a hydrogen atom, halogen, $C_{1-6}$ alkyl, and $C_{1-6}$ haloalkyl;
$R^2$ is selected from the group consisting of a hydrogen atom, halogen, $C_{1-6}$ alkyl, $C_{1-6}$ haloalkyl, and 3- to 12-membered heterocyclyl;
$R^3$ is selected from the group consisting of a hydrogen atom, halogen, $C_{1-6}$ alkyl, and $C_{1-6}$ haloalkyl;
each $R^4$ is identical or different and is independently a hydrogen atom or $C_{1-6}$ alkyl;
J is 1, and k is 1;
n is 0, 1, or 2;
s is 1; and
t is 2.

In some preferred embodiments of the present disclosure, the compound of general formula (II) or the pharmaceutically acceptable salt thereof is provided, wherein:

ring A is pyridinyl or

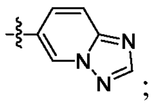

$R^0$ is a hydrogen atom or

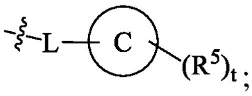

L is $C_{1-6}$ alkylene;
ring C is bicyclo[2.2.2]octyl or pyridinyl;
$R^1$ is $C_{1-6}$ alkyl;
$R^2$ is $C_{1-6}$ alkyl or 3- to 6-membered heterocyclyl;
$R^3$ is a hydrogen atom;
each $R^5$ is identical or different and is independently $-NR^cR^d$ or 3- to 6-membered heterocyclyl;
$R^c$ and $R^d$ are identical or different and are each independently a hydrogen atom or $C_{1-6}$ alkyl;
t is 1;
J is 1, and k is 1;
n is 0, 1, or 2; and
s is 0.

In some preferred embodiments of the present disclosure, the compound of general formula (II) or the pharmaceutically acceptable salt thereof is provided, wherein:

ring A is pyridinyl or

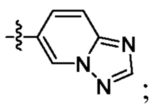

$R^0$ is a hydrogen atom or $C_{1-6}$ alkyl, wherein the $C_{1-6}$ alkyl is optionally substituted with one or more $-C(O)NR^7R^8$;
$R^1$ is $C_{1-6}$ alkyl;
$R^2$ is $C_{1-6}$ alkyl or 3- to 6-membered heterocyclyl;
$R^3$ is a hydrogen atom;
$R^7$ and $R^8$ are identical or different and are each independently a hydrogen atom or $C_{1-6}$ alkyl;
J is 1, and k is 1;
n is 0, 1, or 2; and
s is 0.

In some preferred embodiments of the present disclosure, the compound of general formula (II) or the pharmaceutically acceptable salt thereof is provided, wherein:

ring A is pyridinyl;
$R^0$ is a hydrogen atom;
$R^1$ is $C_{1-6}$ alkyl;
$R^2$ is $C_{1-6}$ alkyl;
$R^3$ is a hydrogen atom;
J is 1, and k is 1;
n is 0, 1, or 2; and
s is 0.

In some preferred embodiments of the present disclosure, the compound of general formula (IIG) or the pharmaceutically acceptable salt thereof is provided, wherein:

ring A is pyridinyl;
L is $C_{1-6}$ alkylene;
ring C is selected from the group consisting of 3- to 8-membered cycloalkyl, 3- to 6-membered heterocyclyl, and 5- or 6-membered heteroaryl;
each $R^5$ is identical or different and is independently selected from the group consisting of halogen, $C_{1-6}$ alkyl, $C_{1-6}$ haloalkyl, amino, and 3- to 6-membered heterocyclyl;
each $R^1$ is identical or different and is independently selected from the group consisting of halogen, $C_{1-6}$ alkyl, and $C_{1-6}$ haloalkyl;
$R^2$ is selected from the group consisting of halogen, $C_{1-6}$ alkyl, $C_{1-6}$ haloalkyl, and 3- to 12-membered heterocyclyl;
$R^3$ is selected from the group consisting of a hydrogen atom, halogen, $C_{1-6}$ alkyl, and $C_{1-6}$ haloalkyl;
J is 1, and k is 1;
n is 0, 1, or 2;
s is 0; and
t is 0, 1, or 2.

In some preferred embodiments of the present disclosure, the compound of general formula (IIG) or the pharmaceutically acceptable salt thereof is provided, wherein:

ring A is pyridinyl;
L is $C_{1-6}$ alkylene;
ring C is selected from the group consisting of 3- to 8-membered cycloalkyl, 3- to 8-membered heterocyclyl, and 5- to 10-membered heteroaryl;
each $R^5$ is identical or different and is independently selected from the group consisting of a hydrogen atom, halogen, $C_{1-6}$ alkyl, $C_{1-6}$ haloalkyl, amino, and 3- to 6-membered heterocyclyl;
each $R^1$ is identical or different and is independently selected from the group consisting of a hydrogen atom, halogen, $C_{1-6}$ alkyl, and $C_{1-6}$ haloalkyl;
$R^2$ is selected from the group consisting of a hydrogen atom, halogen, $C_{1-6}$ alkyl, $C_{1-6}$ haloalkyl, and 3- to 12-membered heterocyclyl;
$R^3$ is selected from the group consisting of a hydrogen atom, halogen, $C_{1-6}$ alkyl, and $C_{1-6}$ haloalkyl;
each $R^4$ is identical or different and is independently a hydrogen atom or $C_{1-6}$ alkyl;
J is 1, and k is 1;
n is 0, 1, or 2;
s is 1; and
t is 2.

In some preferred embodiments of the present disclosure, the compound of general formula (IIG) or the pharmaceutically acceptable salt thereof is provided, wherein:

ring A is pyridinyl;
L is $C_{1-6}$ alkylene;
ring C is 5- to 10-membered heteroaryl;
each $R^5$ is identical or different and is independently selected from the group consisting of a hydrogen atom, halogen, $C_{1-6}$ alkyl, $C_{1-6}$ haloalkyl, and 3- to 12-membered heterocyclyl;
each $R^1$ is identical or different and is independently selected from the group consisting of a hydrogen atom, halogen, $C_{1-6}$ alkyl, and $C_{1-6}$ haloalkyl;
$R^2$ is selected from the group consisting of a hydrogen atom, halogen, $C_{1-6}$ alkyl, $C_{1-6}$ haloalkyl, and 3- to 12-membered heterocyclyl;
$R^3$ is selected from the group consisting of a hydrogen atom, halogen, $C_{1-6}$ alkyl, and $C_{1-6}$ haloalkyl;
each $R^4$ is identical or different and is independently a hydrogen atom or $C_{1-6}$ alkyl;
J is 1, and k is 1;
n is 0, 1, or 2;
s is 1; and
t is 2.

In some preferred embodiments of the present disclosure, the compound of general formula (V) or the pharmaceutically acceptable salt thereof is provided, wherein:

ring A is pyridinyl;

L is $C_{1-6}$ alkylene;
ring C is pyridinyl;
ring D is 4- to 6-membered heterocyclyl containing at least one nitrogen atom;
$R^x$ is selected from the group consisting of a hydrogen atom, $C_{1-6}$ alkyl, and $C_{1-6}$ haloalkyl;
$R^1$ is $C_{1-6}$ alkyl;
$R^2$ is $C_{1-6}$ alkyl or 3- to 6-membered heterocyclyl;
$R^3$ is a hydrogen atom;
J is 1, and k is 1;
n is 0, 1, or 2;
s is 0; and
t is 1.

In some preferred embodiments of the present disclosure, the compound of general formula (V) or the pharmaceutically acceptable salt thereof is provided, wherein:

ring A is pyridinyl;
L is $C_{1-6}$ alkylene;
ring C is pyridinyl;
ring D is 4- to 6-membered heterocyclyl containing at least one nitrogen atom;
$R^x$ is selected from the group consisting of a hydrogen atom, $C_{1-6}$ alkyl, and $C_{1-6}$ haloalkyl;
$R^5$ is a hydrogen atom;
each $R^1$ is identical or different and is independently a hydrogen atom or $C_{1-6}$ alkyl;
$R^2$ is $C_{1-6}$ alkyl or 3- to 6-membered heterocyclyl;
$R^3$ is a hydrogen atom;
$R^4$ is a hydrogen atom;
J is 1, and k is 1;
n is 0, 1, or 2;
s is 1; and
t is 2.

In some preferred embodiments of the present disclosure, the compound of general formula (VI) or the pharmaceutically acceptable salt thereof is provided, wherein:

ring A is pyridinyl;
L is $C_{1-6}$ alkylene;
ring C is 3- to 8-membered cycloalkyl;
$R^1$ is $C_{1-6}$ alkyl;
$R^2$ is $C_{1-6}$ alkyl or 3- to 6-membered heterocyclyl;
$R^3$ is a hydrogen atom; J is 1, and k is 1;
n is 0, 1, or 2;
s is 0; and
t is 1.

In some preferred embodiments of the present disclosure, the compound of general formula (VI) or the pharmaceutically acceptable salt thereof is provided, wherein:

ring A is pyridinyl;
L is $C_{1-6}$ alkylene;
ring C is 3- to 8-membered cycloalkyl;
$R^5$ is a hydrogen atom;
each $R^1$ is identical or different and is independently a hydrogen atom or $C_{1-6}$ alkyl;
$R^2$ is $C_{1-6}$ alkyl or 3- to 6-membered heterocyclyl;
$R^3$ is a hydrogen atom;
$R^4$ is a hydrogen atom;
J is 1, and k is 1;
n is 0, 1, or 2;
s is 1; and
t is 2.

Table A. Typical compounds disclosed herein include, but are not limited to:

| Example No. | Structures and names of compounds |
|---|---|
| 1 | 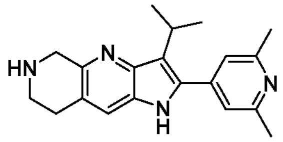 1 |
| | 2-(2,6-Dimethylpyridin-4-yl)-3-isopropyl-5,6,7,8-tetrahydro-1*H*-pyrrolo[3,2-*b*][ 1,7]naphthyridine **1** |
| 2 | 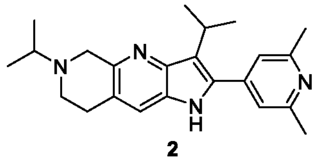 2 |
| | 2-(2,6-Dimethylpyridin-4-yl)-3,6-diisopropyl-5,6,7,8-tetrahydro-1*H*-pyrrolo[3,2-*b*][1,7]naphthyridine **2** |
| 3 | 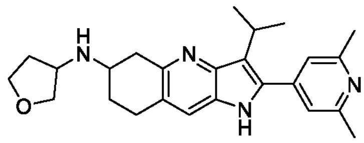 3 |
| | 2-(2,6-Dimethylpyridin-4-yl)-3-isopropyl-*N*-(tetrahydrofuran-3-yl)-5,6,7,8-tetrah ydro-1*H*-pyrrolo[3,2-*b*] quinolin-6-amine **3** |
| 4 | 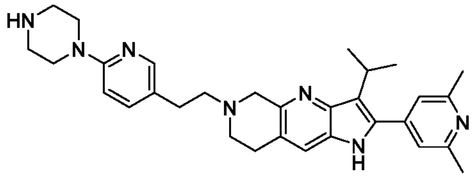 4 |
| | 2-(2,6-Dimethylpyridin-4-yl)-3-isopropyl-6-(2-(6-(piperazin-4-yl)pyridin-3-yl)et hyl)-5,6,7,8-tetrahydro-1*H*-pyrrolo[3,2-*b*][1,7]naphthyridine **4** |
| 5 | 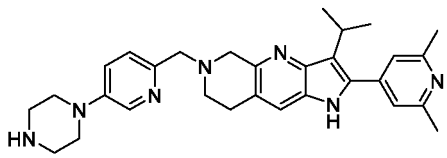 5 |
| | 2-(2,6-Dimethylpyridin-4-yl)-3-isopropyl-6-((5-(piperazin-1-yl)pyridin-2-yl)met hyl)-5,6,7,8-tetrahydro-1*H*-pyrrolo[3,2-*b*][1,7]naphthyridine **5** |

(continued)

| Example No. | Structures and names of compounds |
|---|---|
| **6** | 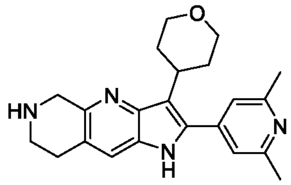 **6** |
| | 2-(2,6-Dimethylpyridin-4-yl)-3-(tetrahydro-2*H*-pyran-4-yl)-5,6,7,8-tetrahydro-1 *H*-pyrrolo[3,2-*b*][1,7] naphthyridine **6** |
| **7** | 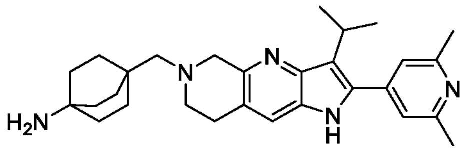 **7** |
| | 4-((2-(2,6-Dimethylpyridin-4-yl)-3-isopropyl-1,5,7,8-tetrahydro-6*H*-pyrrolo[3,2-*b*][1,7]naphthyridin-6-yl) methyl)bicyclo[2.2.2]octyl-1-amine **7** |
| **8** | 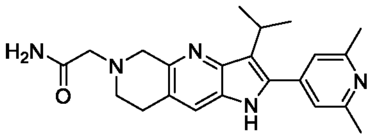 **8** |
| | 2-(2-(2,6-Dimethylpyridin-4-yl)-3-isopropyl-1,5,7,8-tetrahydro-6*H*-pyrrolo[3,2-*b*][1,7]naphthyridin-6-yl) acetamide **8** |
| **9** | 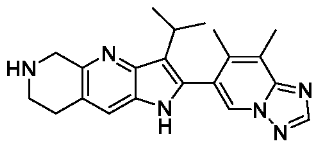 **9** |
| | 2-(7,8-Dimethyl-[1,2,4]triazolo[1,5-*a*]pyridin-6-yl)-3-isopropyl-5,6,7,8-tetrahydr o-1*H*-pyrrolo[3,2-*b*][1,7] naphthyridine **9** |
| **10** | 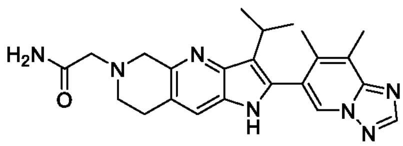 **10** |
| | 2-(2-(7,8-Dimethyl-[1,2,4]triazolo[1,5-*a*]pyridin-6-yl)-3-isopropyl-1,5,7,8-tetrah ydro-6*H*-pyrrolo[3,2-*b*][1,7] naphthyridin-6-yl)acetamide **10** |

Another aspect of the present disclosure relates to a compound of general formula (IIA) or a pharmaceutically acceptable salt thereof:

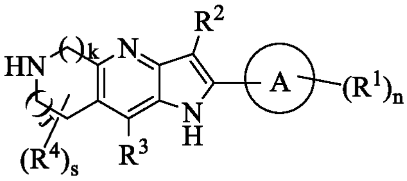

( IIA )

wherein:

ring A, $R^1$ to $R^4$, J, k, n, and s are as defined in the compound of general formula (II).

Another aspect of the present disclosure relates to a compound of general formula (IVA) or a pharmaceutically acceptable salt thereof:

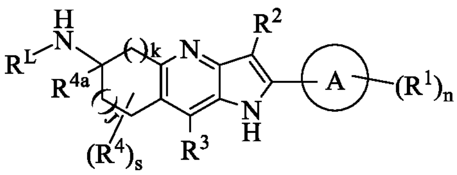

( IVA )

wherein:

ring A, $R^1$ to $R^4$, $R^{4a}$, $R^L$, J, k, n, and s are as defined in the compound of general formula (IV).

Another aspect of the present disclosure relates to a compound of general formula (IIB) or a pharmaceutically acceptable salt thereof:

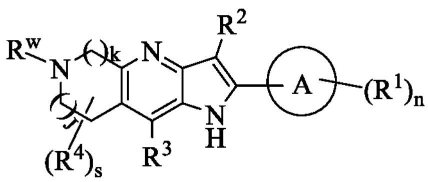

( IIB )

wherein:

$R^w$ is an amino protecting group, preferably tert-butoxycarbonyl;
ring A, $R^1$ to $R^4$, J, k, n, and s are as defined in the compound of general formula (II).

Another aspect of the present disclosure relates to a compound of general formula (VA) or a pharmaceutically acceptable salt thereof:

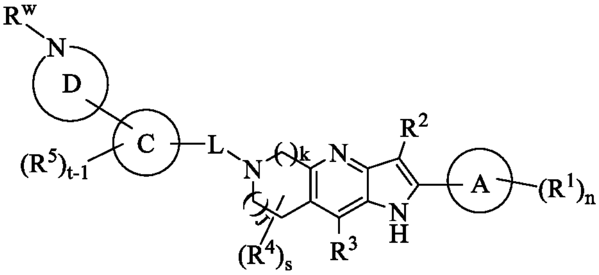

( VA )

wherein:

$R^w$ is an amino protecting group, preferably tert-butoxycarbonyl;
ring D is 4- to 12-membered heterocyclyl containing at least one nitrogen atom;
t is 1, 2, 3, or 4;
ring A, ring C, L, $R^1$ to $R^5$, J, k, n, and s are as defined in general formula (V).

Another aspect of the present disclosure relates to a compound of general formula (VIA) or a pharmaceutically acceptable salt thereof:

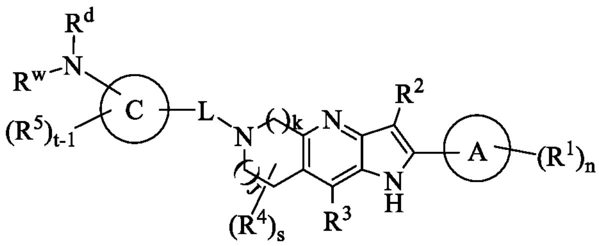

**( VIA )**

wherein:

$R^w$ is an amino protecting group, preferably tert-butoxycarbonyl;
t is 1, 2, 3, or 4;
ring A, ring C, L, $R^1$ to $R^5$, $R^d$, J, k, n, and s are as defined in general formula (VI).

In some preferred embodiments of the present disclosure, the compound of general formula (IIA) or the pharmaceutically acceptable salt thereof is provided, wherein:

ring A is pyridinyl or

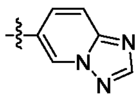 ;

$R^1$ is $C_{1-6}$ alkyl;
$R^2$ is $C_{1-6}$ alkyl;
$R^3$ is a hydrogen atom;
J is 1, and k is 1;
n is 0, 1, or 2; and
s is 0.

Table B. Typical intermediate compounds of the present disclosure include, but are not limited to:

| Exa mple | Structures and names of compounds |
|---|---|
| No. | |
| **1m** | 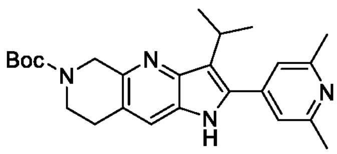 **1m** |
| | tert-Butyl 2-(2,6-dimethylpyridin-4-yl)-3-isopropyl-1,5,7,8-tetrahydro-6*H*-pyrrolo[3,2-*b*][1, 7] naphthyridine-6-carboxylate **1m** |

(continued)

| Exa mple No. | Structures and names of compounds |
|---|---|
| **3k** | 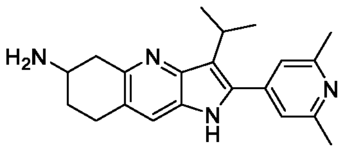 **3k** |
| | 2-(2,6-Dimethylpyridin-4-yl)-3-isopropyl-5,6,7,8-tetrahydro-1*H*-pyrrolo[3,2-b]q uinolin-6-amine **3k** |
| **4e** | 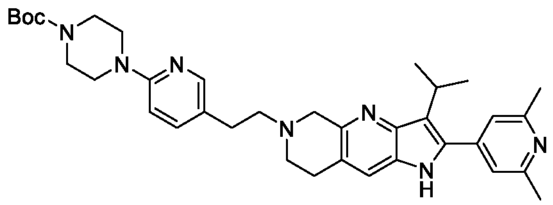 **4e** |
| | tert-Butyl 4-(5-(2-(2-(2,6-dimethylpyridin-4-yl)-3-isopropyl)-1,5,7,8-tetrahydro-6*H*-pyrrolo [3,2-*b*][1,7] naphthyridin-6-yl)ethyl)pyridin-2-yl)piperazine-1-carboxylate **4e** |
| **5e** | 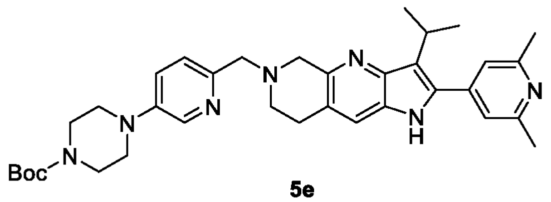 **5e** |
| | tert-Butyl 4-(6-((2-(2,6-dimethylpyridin-4-yl)-3-isopropyl)-1,5,7,8-tetrahydro-6*H*-pyrrolo[3 ,2-*b*][1,7] naphthyridin-6-yl)methyl)pyridin-3-yl)piperazine-1-carboxylate **5e** |
| **6d** | 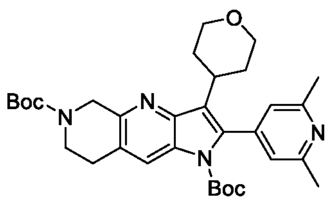 **6d** |
| | Di-tert-butyl 2-(2,6-dimethylpyridin-4-yl)-3-(tetrahydro-2H-pyran-4-yl)-7,8-dihydro-1*H*-pyrro lo[3,2-*b*] [1,7]naphthyridine-1,6(5*H*)-dicarboxylate **6d** |
| **7b** | 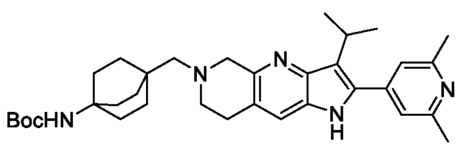 **7b** |
| | tert-Butyl (4-((2-(2,6-dimethylpyridin-4-yl)-3-isopropyl-1,5,7,8-tetrahydro-6*H*-pyrrolo[3,2-*b*][1,7] naphthyridin-6-yl)methyl)bicyclo[2.2.2]oct-1-yl)carbamate **7b** |

(continued)

| Exa mple No. | Structures and names of compounds |
|---|---|
| 9c | 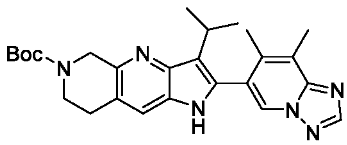 9c |
| | tert-Butyl 2-(7,8-dimethyl-[1,2,4]triazolo[1,5-*a*]pyridin-6-yl)-3-isopropyl-1,5,7,8-tetrahydr o-6*H*-pyrrolo [3,2-*b*][1,7]naphthyridine-6-carboxylate **9c** |

Another aspect of the present disclosure relates to a method for preparing a compound of general formula (II) or a pharmaceutically acceptable salt thereof, which comprises:

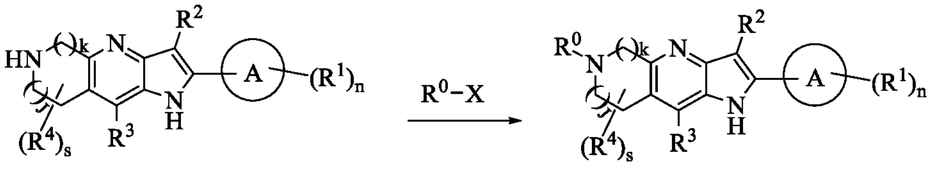

( IIA ) ( II )

conducting a nucleophilic substitution reaction of a compound of general formula (IIA) or a pharmaceutically acceptable salt thereof with $R^0$-X to give the compound of general formula (II) or the pharmaceutically acceptable salt thereof, wherein:

X is a leaving group, preferably halogen, and more preferably bromine or iodine;

$R^0$ is selected from the group consisting of alkyl, alkenyl, alkynyl, heteroalkyl, haloalkyl, hydroxyalkyl, and

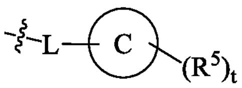 ,

wherein the alkyl is optionally substituted with one or more substituents selected from the group consisting of alkoxy, haloalkoxy, cyano, $-NR^7R^8$, $-C(O)NR^7R^8$, cycloalkyl, heterocyclyl, aryl, and heteroaryl; preferably, $R^0$ is selected from the group consisting of alkyl, alkenyl, alkynyl, heteroalkyl, haloalkyl, hydroxyalkyl, and

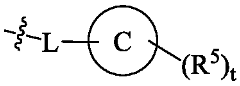 ;

L is a chemical bond or alkylene, wherein the alkylene is optionally substituted with one or more substituents selected from the group consisting of halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl;

ring A, ring C, $R^1$ to $R^5$, n, s, t, J, and k are as defined in the compound of general formula (II).

Another aspect of the present disclosure relates to a method for preparing a compound of general formula (IIG) or a pharmaceutically acceptable salt thereof, which comprises:

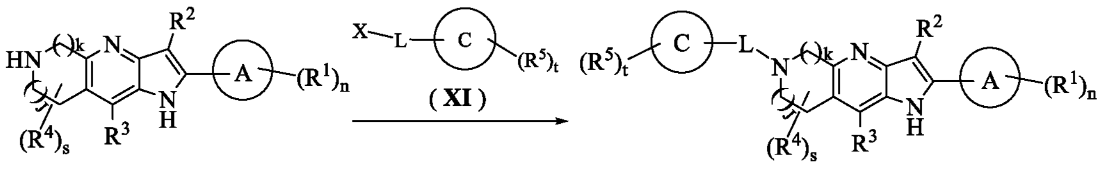

(IIA) (XI) (IIG)

conducting a nucleophilic substitution reaction of a compound of general formula (IIA) or a pharmaceutically acceptable salt thereof with a compound of general formula (XI) to give the compound of general formula (IIG) or the pharmaceutically acceptable salt thereof,
wherein:

X is a leaving group, preferably halogen, and more preferably bromine or iodine;
L is a chemical bond or alkylene, wherein the alkylene is optionally substituted with one or more substituents selected from the group consisting of halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl;
ring A, ring C, $R^1$ to $R^5$, n, s, t, J, and k are as defined in the compound of general formula (IIG).

Another aspect of the present disclosure relates to a method for preparing a compound of general formula (IIA) or a pharmaceutically acceptable salt thereof, which comprises:

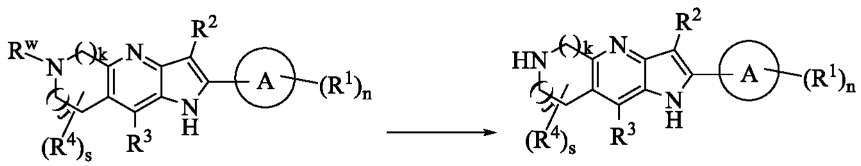

(IIB) (IIA)

removing the protecting group $R^w$ from a compound of general formula (IIB) or a pharmaceutically acceptable salt thereof to give the compound of general formula (IIA) or the pharmaceutically acceptable salt thereof,
wherein:

$R^w$ is an amino protecting group, preferably tert-butoxycarbonyl;
ring A, $R^1$ to $R^4$, n, s, J, and k are as defined in general formula (IIA).

Another aspect of the present disclosure relates to a method for preparing a compound of general formula (IV) or a pharmaceutically acceptable salt thereof, which comprises:

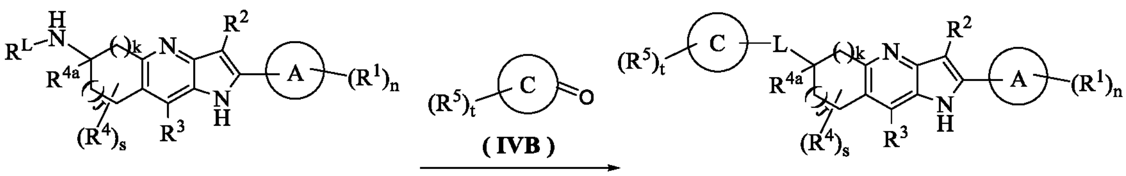

(IVA) (IVB) (IV)

conducting a reductive amination reaction of a compound of general formula (IVA) or a pharmaceutically acceptable salt thereof with a compound of general formula (IVB) to give the compound of general formula (IV) or the pharmaceutically acceptable salt thereof,
wherein:

L is $NR^L$;
$R^L$ is selected from the group consisting of a hydrogen atom, alkyl, alkenyl, alkynyl, haloalkyl, hydroxyalkyl, cycloalkyl, heterocyclyl, aryl, and heteroaryl; preferably, $R^L$ is a hydrogen atom or $C_{1-6}$ alkyl;
ring A, ring C, $R^1$ to $R^5$, $R^{4a}$, n, s, t, J, and k are as defined in general formula (IV).

Another aspect of the present disclosure relates to a method for preparing a compound of general formula (V) or a pharmaceutically acceptable salt thereof, which comprises:

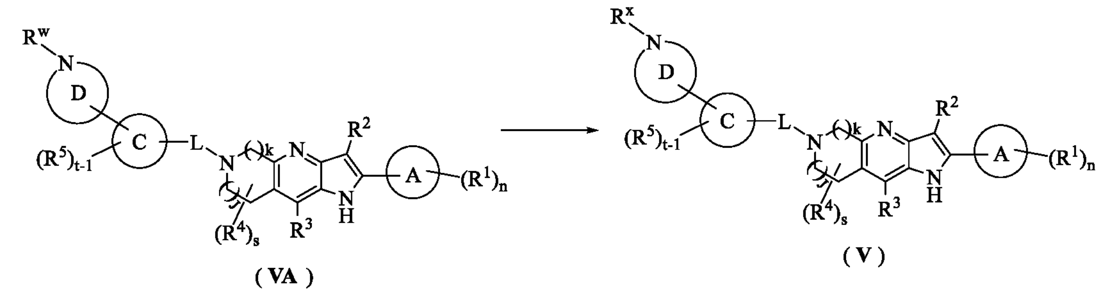

removing the protecting group $R^w$ from a compound of general formula (VA) or a pharmaceutically acceptable salt thereof, and optionally conducting a further reaction with an alkylating reagent $R^x$-W to give the compound of general formula (V) or the pharmaceutically acceptable salt thereof,
wherein:

W is a leaving group, preferably halogen;
$R^x$ is selected from the group consisting of a hydrogen atom, $C_{1-6}$ alkyl, and $C_{1-6}$ haloalkyl;
$R^w$ is an amino protecting group, preferably tert-butoxycarbonyl;
ring D is 4- to 12-membered heterocyclyl containing at least one nitrogen atom;
t is 1, 2, 3, or 4;
ring A, ring C, L, $R^1$ to $R^5$, J, k, n, and s are as defined in general formula (V).

Another aspect of the present disclosure relates to a method for preparing a compound of general formula (VI) or a pharmaceutically acceptable salt thereof, which comprises:

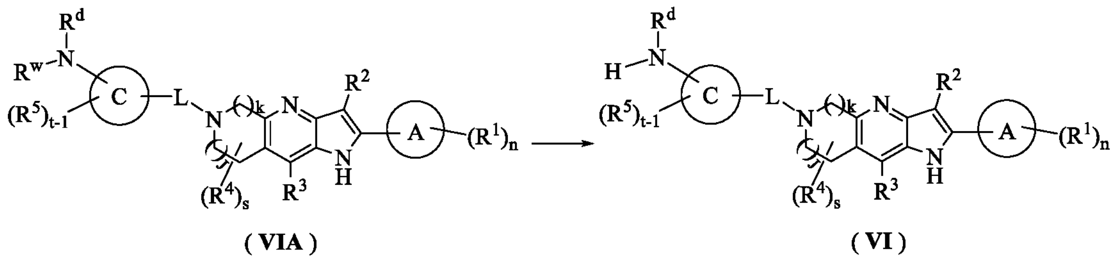

removing the protecting group $R^w$ from a compound of general formula (VIA) or a pharmaceutically acceptable salt thereof to give the compound of general formula (VI) or the pharmaceutically acceptable salt thereof,
wherein:

$R^w$ is an amino protecting group, preferably tert-butoxycarbonyl;
t is 1, 2, 3, or 4;
ring A, ring C, L, $R^1$ to $R^5$, $R^d$, J, k, n, and s are as defined in general formula (VI).

Another aspect of the present disclosure relates to a pharmaceutical composition comprising the compound of general formula (I), general formula (II), general formula (IIG), general formula (III), general formula (IV), general formula (V), or general formula (VI) of the present disclosure and a compound shown in Table A, or pharmaceutically acceptable salts thereof, and one or more pharmaceutically acceptable carriers, diluents, or excipients.
The present disclosure further relates to use of the compound of general formula (I), general formula (II), general formula (IIG), general formula (III), general formula (IV), general formula (V), or general formula (VI) and a compound shown in Table A, or pharmaceutically acceptable salts thereof, or the pharmaceutical composition comprising same in the preparation of a medicament for inhibiting TLR7 and/or TLR8 and/or TLR9.
The present disclosure further relates to use of the compound of general formula (I), general formula (II), general formula (IIG), general formula (III), general formula (IV), general formula (V), or general formula (VI) and a compound shown in Table A, or pharmaceutically acceptable salts thereof, or the pharmaceutical composition comprising same in

the preparation of a medicament for inhibiting TLR7, TLR8, or TLR9, preferably in the preparation of a medicament for inhibiting TLR7, or in the preparation of a medicament for inhibiting TLR7 and TLR8, or in the preparation of a medicament for inhibiting TLR7 and TLR9.

The present disclosure further relates to use of the compound of general formula (I), general formula (II), general formula (IIG), general formula (III), general formula (IV), general formula (V), or general formula (VI) and a compound shown in Table A, or pharmaceutically acceptable salts thereof, or the pharmaceutical composition comprising same in the preparation of a medicament for inhibiting TLR7, TLR8, and TLR9.

The present disclosure further relates to use of the compound of general formula (I), general formula (II), general formula (IIG), general formula (III), general formula (IV), general formula (V), or general formula (VI) and a compound shown in Table A, or pharmaceutically acceptable salts thereof, or the pharmaceutical composition comprising same in the preparation of a medicament for treating and/or preventing an inflammatory or autoimmune disease, wherein the inflammatory or autoimmune disease is preferably selected from the group consisting of systemic lupus erythematosus (SLE), rheumatoid arthritis, multiple sclerosis (MS), and Sjögren's syndrome.

The present disclosure further relates to a method for inhibiting TLR7 and/or TLR8 and/or TLR9, which comprises administering to a patient in need thereof a therapeutically effective amount of the compound of general formula (I), general formula (II), general formula (IIG), general formula (III), general formula (IV), general formula (V), or general formula (VI) and a compound shown in Table A, or pharmaceutically acceptable salts thereof, or the pharmaceutical composition comprising same.

The present disclosure further relates to a method for inhibiting TLR7, TLR8, or TLR9, preferably TLR7, preferably TLR7 and TLR8, and preferably TLR7 and TLR9, which comprises administering to a patient in need thereof a therapeutically effective amount of the compound of general formula (I), general formula (II), general formula (IIG), general formula (III), general formula (IV), general formula (V), or general formula (VI) and a compound shown in Table A, or pharmaceutically acceptable salts thereof, or the pharmaceutical composition comprising same.

The present disclosure further relates to a method for inhibiting TLR7, TLR8, and TLR9, which comprises administering to a patient in need thereof a therapeutically effective amount of the compound of general formula (I), general formula (II), general formula (IIG), general formula (III), general formula (IV), general formula (V), or general formula (VI) and a compound shown in Table A, or pharmaceutically acceptable salts thereof, or the pharmaceutical composition comprising same.

The present disclosure further relates to a method for treating and/or preventing an inflammatory or autoimmune disease, which comprises administering to a patient in need thereof a therapeutically effective amount of the compound of general formula (I), general formula (II), general formula (IIG), general formula (III), general formula (IV), general formula (V), or general formula (VI) and a compound shown in Table A, or pharmaceutically acceptable salts thereof, or the pharmaceutical composition comprising same, wherein the inflammatory or autoimmune disease is preferably selected from the group consisting of systemic lupus erythematosus (SLE), rheumatoid arthritis, multiple sclerosis (MS), and Sjögren's syndrome.

The present disclosure further relates to a compound of general formula (I), general formula (II), general formula (IIG), general formula (III), general formula (IV), general formula (V), or general formula (VI) and a compound shown in Table A, or pharmaceutically acceptable salts thereof, or a pharmaceutical composition comprising same for use as a medicament.

The present disclosure further relates to a compound of general formula (I), general formula (II), general formula (IIG), general formula (III), general formula (IV), general formula (V), or general formula (VI) and a compound shown in Table A, or pharmaceutically acceptable salts thereof, or a pharmaceutical composition comprising same for use in the inhibition of TLR7 and/or TLR8 and/or TLR9.

The present disclosure further relates to a compound of general formula (I), general formula (II), general formula (IIG), general formula (III), general formula (IV), general formula (V), or general formula (VI) and a compound shown in Table A, or pharmaceutically acceptable salts thereof, or a pharmaceutical composition comprising same for use in the inhibition of TLR7, TLR8, or TLR9, preferably in the inhibition of TLR7, or in the inhibition of TLR7 and TLR8, or in the inhibition of TLR7 and TLR9. The present disclosure further relates to a compound of general formula (I), general formula (II), general formula (IIG), general formula (III), general formula (IV), general formula (V), or general formula (VI) and a compound shown in Table A, or pharmaceutically acceptable salts thereof, or a pharmaceutical composition comprising same for use in the inhibition of TLR7, TLR8, and TLR9.

The present disclosure further relates to a compound of general formula (I), general formula (II), general formula (IIG), general formula (III), general formula (IV), general formula (V), or general formula (VI) and a compound shown in Table A, or pharmaceutically acceptable salts thereof, or a pharmaceutical composition comprising same for use in the treatment and/or prevention of an inflammatory or autoimmune disease, wherein the inflammatory or autoimmune disease is preferably selected from the group consisting of systemic lupus erythematosus (SLE), rheumatoid arthritis, multiple sclerosis (MS), and Sjögren's syndrome.

In view of their activity as selective inhibitors of TLR7, TLR8, or TLR9, the compounds of general formula (I),

general formula (II), general formula (IIG), general formula (III), general formula (IV), general formula (V), and general formula (VI) and the compounds shown in Table A can be used to treat diseases associated with the TLR7, TLR8, or TLR9 family receptor, including but not limited to: inflammatory diseases (such as Crohn's disease, ulcerative colitis, asthma, graft-versus-host disease, allograft rejection, and chronic obstructive pulmonary disease); autoimmune diseases (such as Graves' disease, rheumatoid arthritis, systemic lupus erythematosus, lupus nephritis, cutaneous lupus, and psoriasis); auto inflammatory diseases (including Cryopyrin-associated periodic syndrome (CAPS), TNF receptor-associated periodic syndrome (TRAPS), familial mediterranean fever (FMF), adult-onset Still's disease, systemic onset juvenile idiopathic arthritis, gout, and gouty arthritis); metabolic diseases (including type 2 diabetes, atherosclerosis, and myocardial infarction); destructive bone disorders (such as bone resorption disease, osteoarthritis, osteoporosis, and multiple myeloma-related bone disorder); proliferative disorders (such as acute myelogenous leukemia and chronic myelogenous leukemia); angiogenic disorders (such as angiogenic disorders of ocular neovasculization and infantile haemangiomas); infectious diseases (such as sepsis, septic shock, and Shigellosis); neurodegenerative diseases (such as Alzheimer's disease, Parkinson's disease, and cerebral ischemia or neurodegenerative disease caused by traumatic injury), oncologic diseases (such as metastatic melanoma, Kaposi's sarcoma, and multiple myeloma), and viral diseases (such as HIV infection and CMV retinitis, and AIDS), respectively.

More particularly, the specific conditions or diseases that can be treated with the compounds of the present disclosure include, but are not limited to, pancreatitis (acute or chronic), asthma, allergies, adult respiratory distress syndrome, chronic obstructive pulmonary disease, glomerulonephritis, rheumatoid arthritis, systemic lupus erythematosus, scleroderma, chronic thyroiditis, Graves' disease, autoimmune gastritis, diabetes, autoimmune hemolytic anemia, autoimmune neutropenia, thrombocytopenia, atopic dermatitis, chronic active hepatitis, myasthenia gravis, multiple sclerosis, inflammatory bowel disease, ulcerative colitis, Crohn's disease, psoriasis, graft-versus-host disease, endotoxin-induced inflammatory reaction, tuberculosis, atherosclerosis, muscle degeneration, cachexia, psoriatic arthritis, Reiter's syndrome, gout, traumatic arthritis, rubella arthritis, acute synovitis, pancreatic β-cell disease; diseases characterized by massive neutrophil infiltration; rheumatoid spondylitis, gouty arthritis and other arthritic conditions, cerebral malaria, chronic pulmonary inflammatory disease, silicosis, pulmonary sarcoidosis, bone resorption disease, allograft rejection, fever and myalgias caused by infection, cachexia secondary to infection, keloid formation, scar tissue formation, ulcerative colitis, pyresis, influenza, osteoporosis, osteoarthritis, acute myelogenous leukemia, chronic myelogenous leukemia, metastatic melanoma, Kaposi's sarcoma, multiple myeloma, sepsis, septic shock, and Shigellosis; Alzheimer's disease, Parkinson's disease, cerebral ischemia or neurodegenerative disease caused by traumatic injury; angiogenic disorders including ocular neovasculization and infantile haemangiomas; viral diseases including acute hepatitis infection (including hepatitis A, hepatitis B, and hepatitis C), HIV infection and CMV retinitis, AIDS, ARC or malignancy, and herpes; stroke, myocardial ischemia, ischemia in stroke heart attacks, organ hypoxia, vascular hyperplasia, cardiac and renal reperfusion injury, thrombosis, cardiac hypertrophy, thrombin-induced platelet aggregation, endotoxemia and/or toxic shock syndrome, conditions associated with prostaglandin endoperoxidase syndase-2, and pemphigus vulgaris. In preferred treatment methods, the condition is selected from the group consisting of Crohn's disease, ulcerative colitis, allograft rejection, rheumatoid arthritis, psoriasis, ankylosing spondylitis, psoriatic arthritis, and pemphigus vulgaris. In alternatively preferred treatment methods, the condition is selected from the group consisting of ischemia reperfusion injury, including cerebral ischemia reperfusions injury caused by stroke and cardiac ischemia reperfusion injury caused by myocardial infarction. In another preferred treatment method, the condition is multiple myeloma.

The active compound may be formulated into a form suitable for administration by any suitable route, and one or more pharmaceutically acceptable carriers are used to formulate the composition of the present disclosure by conventional methods. Thus, the active compound of the present disclosure may be formulated into a variety of dosage forms for oral administration, administration by injection (e.g., intravenous, intramuscular, or subcutaneous), or administration by inhalation or insufflation. The compound of the present disclosure may also be formulated into a sustained-release dosage form, such as tablets, hard or soft capsules, aqueous or oily suspensions, emulsions, injections, dispersible powders or granules, suppositories, lozenges, or syrups.

As a general guide, the active compound is preferably in a form of a unit dose or in a form of a single dose that can be self-administered by a patient. The unit dose of the compound or composition of the present disclosure may be in a tablet, capsule, cachet, vial, powder, granule, lozenge, suppository, regenerating powder, or liquid formulation. A suitable unit dose may be 0.1-1000 mg.

The pharmaceutical composition of the present disclosure may comprise, in addition to the active compound, one or more auxiliary materials selected from the group consisting of a filler (diluent), a binder, a wetting agent, a disintegrant, an excipient and the like. Depending on the method of administration, the composition may comprise 0.1 wt.% to 99 wt.% of the active compound.

The tablet comprises the active ingredient and a non-toxic pharmaceutically acceptable excipient that is used for mixing and is suitable for the preparation of the tablet. Such an excipient may be an inert excipient, a granulating agent, a disintegrant, a binder and a lubricant. Such a tablet may be uncoated or may be coated by known techniques for masking the taste of the drug or delaying the disintegration and absorption of the drug in the gastrointestinal tract

and thus enabling sustained release of the drug over a longer period.

An oral formulation in a soft gelatin capsule where the active ingredient is mixed with an inert solid diluent or with a water-soluble carrier or oil vehicle may also be provided. An aqueous suspension comprises the active substance and an excipient that is used for mixing and is suitable for the preparation of the aqueous suspension. Such an excipient is a suspending agent, a dispersant, or a wetting agent. The aqueous suspension may also comprise one or more preservatives, one or more colorants, one or more flavoring agents, and one or more sweeteners.

An oil suspension may be formulated by suspending the active ingredient in a vegetable oil, or in a mineral oil. The oil suspension may comprise a thickening agent. Sweeteners and flavoring agents may be added to provide a palatable formulation. Antioxidants may also be added to preserve the compositions.

The pharmaceutical composition of the present disclosure may also be in the form of an oil-in-water emulsion. The oil phase may be a vegetable oil or a mineral oil, or a mixture thereof. Suitable emulsifiers may be naturally occurring phospholipids, and the emulsion may also comprise a sweetener, a flavoring agent, a preservative, and an antioxidant. Such a formulation may also comprise a palliative, a preservative, a colorant, and an antioxidant.

The pharmaceutical composition of the present disclosure may be in the form of a sterile injectable aqueous solution. Acceptable vehicles or solvents that can be used include water, Ringer's solution and isotonic sodium chloride solution. A sterile injectable formulation may be a sterile injectable oil-in-water microemulsion in which an active ingredient is dissolved in an oil phase. The injection or microemulsion can be locally injected into the bloodstream of a patient in large quantities. Alternatively, it may be desirable to administer the solution and microemulsion in such a way as to maintain a constant circulating concentration of the compound of the present disclosure. To maintain such a constant concentration, a continuous intravenous delivery device may be used. An example of such a device is a Deltec CADD-PLUS. TM. 5400 intravenous injection pump.

The pharmaceutical composition of the present disclosure may be in the form of a sterile injectable aqueous or oil suspension for intramuscular and subcutaneous administration. The suspension can be prepared according to the prior art using suitable dispersants or wetting agents and suspending agents. The sterile injectable formulation may also be a sterile injection or suspension prepared in a parenterally acceptable non-toxic diluent or solvent. In addition, a sterile fixed oil may be conventionally used as a solvent or a suspending medium. For this purpose, any blend fixed oil may be used. In addition, fatty acids may also be used to prepare injections.

The compound of the present disclosure may be administered in the form of a suppository for rectal administration. Such a pharmaceutical composition can be prepared by mixing a drug with a suitable non-irritating excipient which is a solid at ambient temperature but a liquid in the rectum and therefore will melt in the rectum to release the drug.

The compound of the present disclosure can be administered in the form of dispersible powders and granules that are formulated into aqueous suspensions by adding water. Such a pharmaceutical composition can be prepared by mixing the active ingredient with a dispersant or a wetting agent, a suspending agent, or one or more preservatives. As is well known to those skilled in the art, the dose of the drug administered depends on a variety of factors, including but not limited to, the activity of the particular compound used, the age of the patient, the body weight of the patient, the health condition of the patient, the behavior of the patient, the diet of the patient, the time of administration, the route of administration, the rate of excretion, the combination of drugs, the severity of the disease, and the like. In addition, the optimal treatment regimen, such as the mode of administration, the daily dose of the compound, or the type of pharmaceutically acceptable salts, can be verified according to conventional treatment regimens.

## Description of the terms

Unless otherwise stated, the terms used in the specification and claims have the following meanings.

The term "alkyl" refers to a saturated straight-chain or branched-chain aliphatic hydrocarbon group having 1 to 20 (e.g., 1, 2, 3, 4, 5, 6, 7, 8, 9, 10, 11, 12, 13, 14, 15, 16, 17, 18, 19, or 20) carbon atoms (i.e., $C_{1-20}$ alkyl). The alkyl is preferably an alkyl group having 1 to 12 carbon atoms (i.e., $C_{1-12}$ alkyl), more preferably an alkyl group having 1 to 6 carbon atoms (i.e., $C_{1-6}$ alkyl). Non-limiting examples of alkyl include: methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various side-chain isomers thereof, and the like. Most preferred is a lower alkyl group having 1 to 6 carbon atoms; non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and the like. Alkyl may be substituted or unsubstituted. When substituted, it may be substituted at any accessible connection site, and the substituent is preferably selected from one or more of a D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkylene" refers to a divalent alkyl group, wherein the alkyl is as defined above; it has 1 to 20 (e.g., 1, 2, 3, 4, 5, 6, 7, 8, 9, 10, 11, 12, 13, 14, 15, 16, 17, 18, 19, or 20) carbon atoms (i.e., $C_{1-20}$ alkylene). The alkylene is preferably an alkylene group having 1 to 12 carbon atoms (i.e., $C_{1-12}$ alkylene), and more preferably an alkylene group having 1 to 6 carbon atoms (i.e., $C_{1-6}$ alkylene). Non-limiting examples of alkylene include, but are not limited to: methylene ($-CH_2-$), 1,1-ethylene ($-CH(CH_3)-$), 1,2-ethylene ($-CH_2CH_2-$), 1,1-propylene ($-CH(CH_2CH_3)-$), 1,2-propylene ($-CH_2CH(CH_3)-$), 1,3-propylene ($-CH_2CH_2CH_2-$), 1,4-butylene ($-CH_2CH_2CH_2CH_2-$), and the like. Alkylene may be substituted or unsubstituted. When substituted, it may be substituted at any accessible connection site, and the substituent is preferably selected from one or more of alkenyl, alkynyl, alkoxy, haloalkoxy, cycloalkyloxy, heterocyclyloxy, alkylthio, alkylamino, halogen, sulfhydryl, hydroxy, nitro, cyano, cycloalkyl, heterocyclyl, aryl, heteroaryl, cycloalkoxy, heterocycloalkoxy, cycloalkylthio, heterocycloalkylthio, and oxo.

The term "heteroalkyl" refers to alkyl in which one or more (preferably 1, 2, 3, 4, or 5) $-CH_2-$ are replaced by heteroatoms selected from the group consisting of N, O, and S, wherein the alkyl is as defined above. Heteroalkyl may be substituted or unsubstituted. When substituted, it may be substituted at any accessible connection site, and the substituent is preferably selected from one or more of a deuterium atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkenyl" refers to an alkyl group containing at least one carbon-carbon double bond in the molecule, wherein the alkyl is as defined above, and it has 2 to 12 (e.g., 2, 3, 4, 5, 6, 7, 8, 9, 10, 11, or 12) carbon atoms (i.e., $C_{2-12}$ alkenyl). The alkenyl is preferably an alkenyl group having 2 to 6 carbon atoms (i.e., $C_{2-6}$ alkenyl). Non-limiting examples include: ethenyl, propenyl, isopropenyl, butenyl, and the like. Alkenyl may be substituted or unsubstituted. When it is substituted, the substituent is preferably selected from one or more of alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkynyl" refers to an alkyl group containing at least one carbon-carbon triple bond in the molecule, wherein the alkyl is as defined above, and it has 2 to 12 (e.g., 2, 3, 4, 5, 6, 7, 8, 9, 10, 11, and 12) carbon atoms (i.e., $C_{2-12}$ alkynyl). The alkynyl is preferably an alkynyl group having 2 to 6 carbon atoms (i.e., $C_{2-6}$ alkynyl). Non-limiting examples include: ethynyl, propynyl, butynyl, pentynyl, hexynyl, and the like. Alkynyl may be substituted or unsubstituted. When it is substituted, the substituent is preferably selected from one or more of alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkoxy" refers to -O-(alkyl), wherein the alkyl is as defined above. Non-limiting examples include: methoxy, ethoxy, propoxy, butoxy, and the like. Alkoxy may be substituted or unsubstituted. When substituted, it may be substituted at any accessible connection site, and the substituent is preferably selected from one or more of a deuterium atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "cycloalkyl" refers to a saturated or partially unsaturated monocyclic or polycyclic hydrocarbon substituent, and the cycloalkyl ring has 3 to 20 (e.g., 3, 4, 5, 6, 7, 8, 9, 10, 11, 12, 13, 14, 15, 16, 17, 18, 19, or 20) carbon atoms (i.e., 3- to 20-membered cycloalkyl), preferably 3 to 12 carbon atoms (i.e., 3- to 12-membered cycloalkyl), more preferably 3 to 8 carbon atoms (i.e., 3- to 8-membered cycloalkyl), and most preferably 3 to 6 carbon atoms (i.e., 3- to 6-membered cycloalkyl). Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like. Polycyclic cycloalkyl includes spiro cycloalkyl, fused cycloalkyl, and bridged cycloalkyl.

The term "spiro cycloalkyl" refers to a 5- to 20-membered polycyclic group in which rings share one carbon atom (referred to as the spiro atom), and it may contain one or more double bonds. It is preferably 6- to 14-membered, and more preferably 7- to 10-membered (e.g., 7-membered, 8-membered, 9-membered, or 10-membered). According to the number of the spiro atoms shared among the rings, the spiro cycloalkyl may be monospiro cycloalkyl or polyspiro cycloalkyl (e.g., bispiro cycloalkyl), preferably monospiro cycloalkyl and bispiro cycloalkyl, and more preferably 3-membered/5-membered, 3-membered/6-membered, 4-membered/4-membered, 4-membered/5-membered, 4-membered/6-membered, 5-membered/5-membered, 5-membered/6-membered, 6-membered/4-membered, 6-membered/5-membered, or 6-membered/6-membered monospiro cycloalkyl. Non-limiting examples of spiro cycloalkyl include:

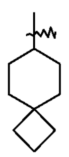 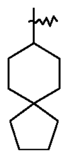 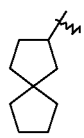 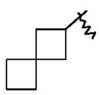 and 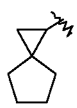 .

The term "fused cycloalkyl" refers to a 5- to 20-membered all-carbon polycyclic group in which rings share a pair of adjacent carbon atoms, wherein one or more of the rings may contain one or more double bonds. It is preferably 6- to 14-membered, and more preferably 7- to 10-membered (e.g., 7-membered, 8-membered, 9-membered, or 10-membered). According to the number of constituent rings, the fused cycloalkyl may be bicyclic, tricyclic, tetracyclic, etc., preferably bicyclic or tricyclic, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered bicyclic alkyl. Non-limiting examples of fused cycloalkyl include:

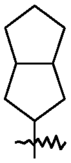 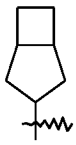 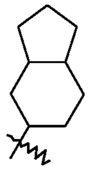 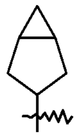 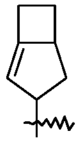 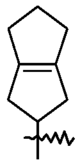 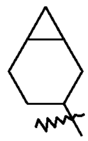 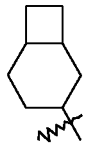 and 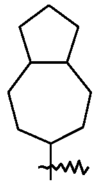.

The term "bridged cycloalkyl" refers to a 5- to 20-membered all-carbon polycyclic group in which any two rings share two carbon atoms that are not directly connected, and it may contain one or more double bonds. It is preferably 6- to 14-membered, and more preferably 7- to 10-membered (e.g., 7-membered, 8-membered, 9-membered, or 10-membered). According to the number of constituent rings, the bridged cycloalkyl may be bicyclic, tricyclic, tetracyclic, etc., preferably bicyclic, tricyclic, or tetracyclic, and more preferably bicyclic or tricyclic. Non-limiting examples of bridged cycloalkyl include:

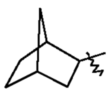 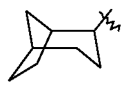 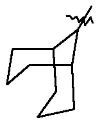 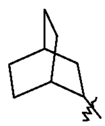 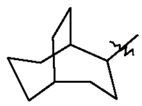

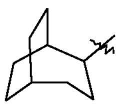 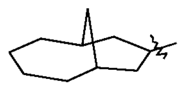 and 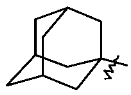.

The cycloalkyl ring includes those in which the cycloalkyl described above (including monocyclic, spiro, fused, and bridged ones) fuses with an aryl, heteroaryl, or heterocycloalkyl ring, wherein the ring connected to the parent structure is cycloalkyl; non-limiting examples include

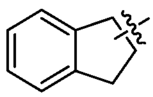, 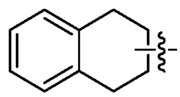, 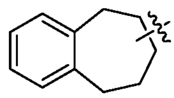,

etc., preferably

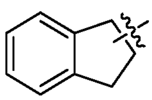 and 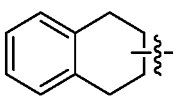.

Cycloalkyl may be substituted or unsubstituted. When substituted, it may be substituted at any accessible connection site, and the substituent is preferably selected from one or more of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "heterocyclyl" refers to a saturated or partially unsaturated monocyclic or polycyclic substituent having 3 to 20 ring atoms, of which one or more are heteroatoms selected from the group consisting of nitrogen, oxygen, and

sulfur, and the sulfur may optionally be substituted with oxo (i.e., to form sulfoxide or sulfone), but excluding a cyclic portion of -O-O-, -O-S-, or -S-S-; the other ring atoms are carbon. Preferably, it has 3 to 12 (e.g. 3, 4, 5, 6, 7, 8, 9, 10, 11, and 12) ring atoms, of which 1-4 (e.g. 1, 2, 3, and 4) are heteroatoms (i.e. 3- to 12-membered heterocyclyl) (e.g., 4- to 12-membered heterocyclyl containing at least one nitrogen atom); more preferably, it has 3 to 8 ring atoms (e.g., 3, 4, 5, 6, 7, and 8), of which 1-3 are heteroatoms (e.g., 1, 2, and 3) (i.e., 3-to 8-membered heterocyclyl); more preferably, it has 3 to 6 ring atoms, of which 1-3 are heteroatoms (i.e. 3- to 6-membered heterocyclyl) (e.g., 4- to 6-membered heterocyclyl containing at least one nitrogen atom); most preferably, it has 5 or 6 ring atoms, of which 1-3 are heteroatoms (i.e. 5- or 6-membered heterocyclyl). Non-limiting examples of monocyclic heterocyclyl include: pyrrolidinyl, tetrahydropyranyl, 1,2,3,6-tetrahydropyridinyl, piperidinyl, piperazinyl, morpholinyl, thiomorpholinyl, homopiperazinyl, and the like. Polycyclic heterocyclyl includes spiro heterocyclyl, fused heterocyclyl, and bridged heterocyclyl.

The term "spiro heterocyclyl" refers to a 5- to 20-membered polycyclic heterocyclyl group in which monocyclic rings share one atom (referred to as the spiro atom), wherein one or more ring atoms are heteroatoms selected from the group consisting of nitrogen, oxygen, and sulfur, and the sulfur may optionally be substituted with oxo (i.e., to form sulfoxide or sulfone); the other ring atoms are carbon. It may contain one or more double bonds. It is preferably 6- to 14-membered, and more preferably 7- to 10-membered (e.g., 7-membered, 8-membered, 9-membered, or 10-membered). According to the number of spiro atoms shared among the rings, the spiro heterocyclyl may be monospiro heterocyclyl or polyspiro heterocyclyl (e.g., bispiro heterocyclyl), preferably monospiro heterocyclyl and bispiro heterocyclyl, and more preferably 3-membered/5-membered, 3-membered/6-membered, 4-membered/4-membered, 4-membered/5-membered, 4-membered/6-membered, 5-membered/5-membered, 5-membered/6-membered, or 6-membered/6-membered monospiro heterocyclyl. Non-limiting examples of spiro heterocyclyl include:

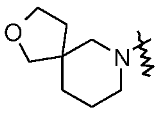 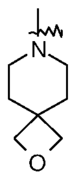 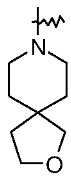 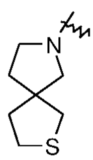 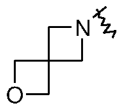 and 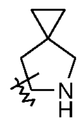 .

The term "fused heterocyclyl" refers to a 5- to 20-membered polycyclic heterocyclyl group in which rings share a pair of adjacent atoms, and one or more of the rings may contain one or more double bonds, wherein one or more ring atoms are heteroatoms selected from the group consisting of nitrogen, oxygen, and sulfur, and the sulfur may optionally be substituted with oxo (i.e., to form sulfoxide or sulfone); the other ring atoms are carbon. It is preferably 6- to 14-membered, and more preferably 7- to 10-membered (e.g., 7-membered, 8-membered, 9-membered, or 10-membered). According to the number of constituent rings, the fused heterocyclyl may be bicyclic, tricyclic, tetracyclic, etc., preferably bicyclic or tricyclic, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 6-membered/3-membered, 6-membered/4-membered, 6-membered/5-membered, 6-membered/6-membered, 6-membered/7-membered, 7-membered/5-membered, or 7-membered/6-membered bicyclic fused heterocyclyl. Non-limiting examples of fused heterocyclyl include:

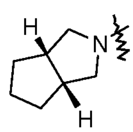 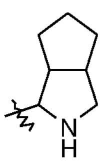 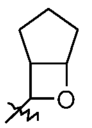 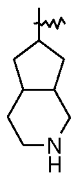 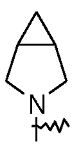 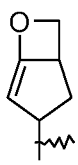 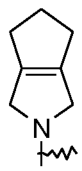 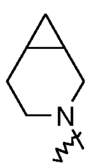

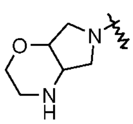 , 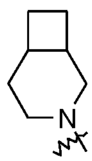 , 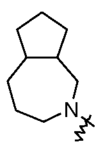 , 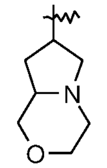 , 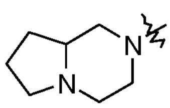 and 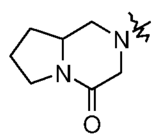 .

The term "bridged heterocyclyl" refers to a 5- to 14-membered polycyclic heterocyclyl group in which any two rings share two atoms that are not directly connected, and it may contain one or more double bonds, wherein one or

more ring atoms are heteroatoms selected from the group consisting of nitrogen, oxygen, and sulfur, and the sulfur may optionally be substituted with oxo (i.e., to form sulfoxide or sulfone); the other ring atoms are carbon. It is preferably 6- to 14-membered, and more preferably 7-to 10-membered (e.g., 7-membered, 8-membered, 9-membered, or 10-membered). According to the number of constituent rings, the bridged heterocyclyl may be bicyclic, tricyclic, tetracyclic, etc., preferably bicyclic, tricyclic, or tetracyclic, and more preferably bicyclic or tricyclic. Non-limiting examples of bridged heterocyclyl include:

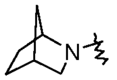 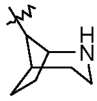 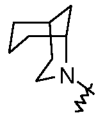 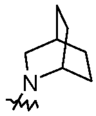

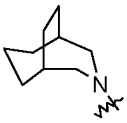 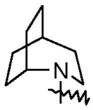 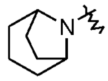 and 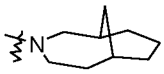 .

The heterocyclyl ring includes those in which the heterocyclyl described above (including monocyclic, spiro, fused, and bridged ones) fuses with an aryl, heteroaryl, or cycloalkyl ring, wherein the ring connected to the parent structure is heterocyclyl; its non-limiting examples include:

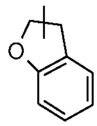 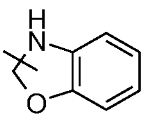 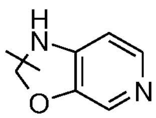 and 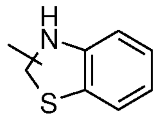 , etc.

Heterocyclyl may be substituted or unsubstituted. When substituted, it may be substituted at any accessible connection site, and the substituent is preferably selected from one or more of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "aryl" refers to a 6- to 14-membered, preferably 6- to 10-membered, all-carbon monocyclic or fused polycyclic (in which the rings share a pair of adjacent carbon atoms) group having a conjugated π-electron system, e.g., phenyl and naphthyl. The aryl ring includes those in which the aryl ring described above fuses with a heteroaryl, heterocyclyl, or cycloalkyl ring, wherein the ring connected to the parent structure is the aryl ring; its non-limiting examples include:

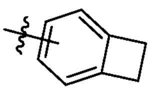 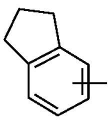 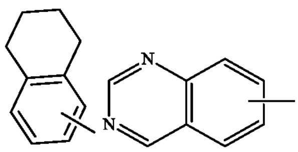 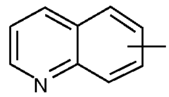 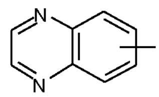 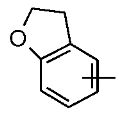

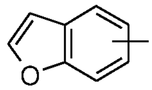 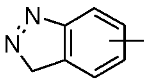 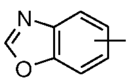 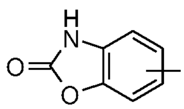 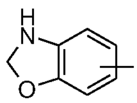 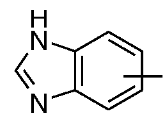

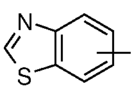 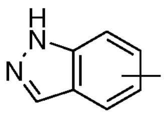 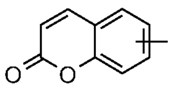 and 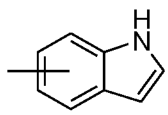 .

Aryl may be substituted or unsubstituted. When substituted, it may be substituted at any accessible connection site, and the substituent is preferably selected from one or more of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "heteroaryl" refers to a heteroaromatic system containing 1 to 4 (e.g., 1, 2, 3, and 4) heteroatoms and 5 to 14 ring atoms, wherein the heteroatoms are selected from the group consisting of oxygen, sulfur, and nitrogen. The heteroaryl is preferably 5- to 10-membered (e.g., 5-membered, 6-membered, 7-membered, 8-membered, 9-membered, or 10-membered) and more preferably 5-membered or 6-membered, e.g., furyl, thienyl, pyridinyl, pyrrolyl, N-alkylpyrrolyl, pyrimidinyl, pyrazinyl, pyridazinyl, imidazolyl, pyrazolyl, triazolyl, and tetrazolyl. The heteroaryl ring includes those in which the heteroaryl ring described above fuses with an aryl, heterocyclyl, or cycloalkyl ring, wherein the ring connected to the parent structure is the heteroaryl ring; its non-limiting examples include:

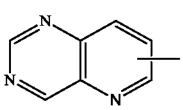 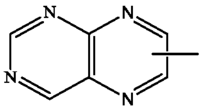 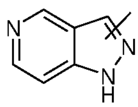 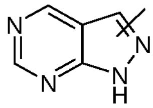 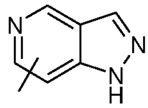 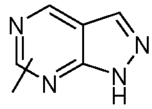

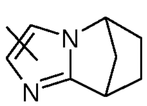 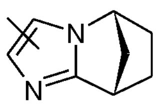 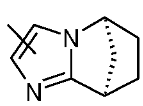 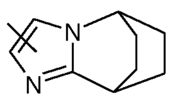 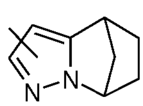 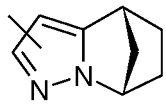

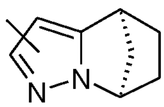 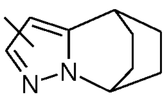 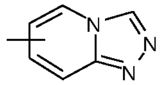 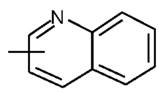 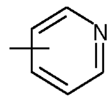 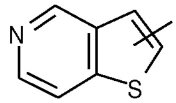

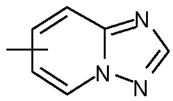 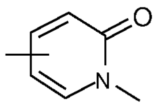 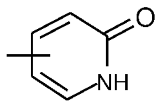 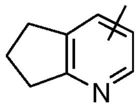 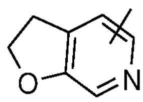, 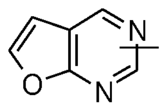,

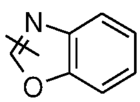, 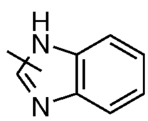, 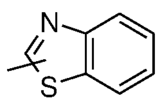, 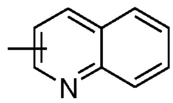, 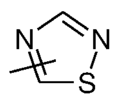

and

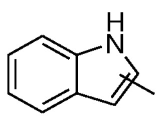.

Heteroaryl may be substituted or unsubstituted. When substituted, it may be substituted at any accessible connection site, and the substituent is preferably selected from one or more of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The cycloalkyl, heterocyclyl, aryl, and heteroaryl described above include residues derived by removal of one hydrogen atom from a ring atom of the parent structure, or residues derived by removal of two hydrogen atoms from the same ring atom or two different ring atoms of the parent structure, i.e., "cycloalkylene", "heterocyclylene", "arylene", and "heteroarylene".

The term "amino protecting group" refers to a group that is introduced onto an amino group in order for the amino group to remain unchanged when other parts of the molecule are involved in reactions, and the group can be easily removed. Non-limiting examples include: (trimethylsilyl)ethoxymethyl, tetrahydropyranyl, tert-butoxycarbonyl (Boc), acetyl, benzyl, benzyloxycarbonyl (Cbz), allyl, p-methoxybenzyl, and the like. These groups may be optionally substituted with 1-3 substituents selected from the group consisting of halogen, alkoxy, and nitro.

The term "hydroxy protecting group" refers to a group that is generally introduced onto a hydroxy group in order to block or protect the hydroxy group when other functional groups of the compound are involved in reactions, and the group can be easily removed. Non-limiting examples include: trimethylsilyl (TMS), triethylsilyl (TES), triisopropylsilyl (TIPS), tert-butyldimethylsilyl (TBS), tert-butyldiphenylsilyl, methyl, tert-butyl, allyl, benzyl, methoxymethyl (MOM), ethoxyethyl, 2-tetrahydropyranyl (THP), formyl, acetyl, benzoyl, p-nitrobenzoyl, and the like.

The term "cycloalkyloxy" refers to cycloalkyl-O-, wherein the cycloalkyl is as defined above.

The term "heterocyclyloxy" refers to heterocyclyl-O-, wherein the heterocyclyl is as defined above.

The term "aryloxy" refers to aryl-O-, wherein the aryl is as defined above.

The term "heteroaryloxy" refers to heteroaryl-O-, wherein the heteroaryl is as defined above.

The term "alkylthio" refers to alkyl-S-, wherein the alkyl is as defined above.

The term "haloalkyl" refers to alkyl substituted with one or more halogens, wherein the alkyl is as defined above.

The term "haloalkoxy" refers to alkoxy substituted with one or more halogens, wherein the alkoxy is as defined above.

The term "deuterated alkyl" refers to alkyl substituted with one or more deuterium atoms, wherein the alkyl is as defined above.

The term "hydroxyalkyl" refers to alkyl substituted with one or more hydroxy groups, wherein the alkyl is as defined above.

The term "halogen" refers to fluorine, chlorine, bromine or iodine.

The term "hydroxy" refers to -OH.

The term "sulfhydryl" refers to -SH.

The term "amino" refers to $-NH_2$.

The term "cyano" refers to -CN.

The term "nitro" refers to $-NO_2$.

The term "oxo" refers to "=O".

The term "carbonyl" refers to C=O.

The term "aldehyde" refers to -C(O)H.

The term "carboxyl" refers to -C(O)OH.

The term "carboxylate group" refers to -C(O)O(alkyl), -C(O)O(cycloalkyl), (alkyl)C(O)O- or (cycloalkyl)C(O)O-, wherein the alkyl and cycloalkyl are as defined above.

In the chemical structure of the compound of the present disclosure, a bond "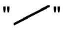" represents an unspecified configuration, namely if chiral isomers exist in the chemical structure, the bond "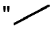" may be "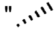" or "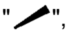", or contains both the configurations of "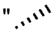" and "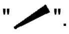".

The compounds and intermediates of the present disclosure may also exist in different tautomeric forms, and all such forms are included within the scope of the present disclosure. The term "tautomer" or "tautomeric form" refers to structural isomers of different energies that can interconvert via a low energy barrier, for example, keto-enol, imine-enamine, and lactam-lactim isomerization. An example of a lactam-lactim equilibrium is present between A and B as shown below:

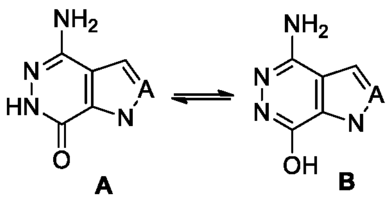

A B .

All tautomeric forms are within the scope of the present disclosure. The nomenclature of the compounds does not exclude any tautomers.

The present disclosure also comprises isotopically-labeled compounds which are identical to those recited herein but have one or more atoms replaced by an atom having an atomic mass or mass number different from the atomic mass or mass number usually found in nature. Examples of isotopes that can be incorporated into the compound of the present disclosure include isotopes of hydrogen, carbon, nitrogen, oxygen, phosphorus, sulfur, fluorine, iodine, and chlorine, such as $^{2}H$, $^{3}H$, $^{11}C$, $^{13}C$, $^{14}C$, $^{13}N$, $^{15}N$, $^{15}O$, $^{17}O$, $^{18}O$, $^{31}P$, $^{32}P$, $^{35}S$, $^{18}F$, $^{123}I$, $^{125}I$, and $^{36}Cl$. Such a compound can be used as an analytical tool or a probe in, for example, a biological assay, or may be used as a tracer for *in vivo* diagnostic imaging of disease, or as a tracer in a pharmacodynamic, pharmacokinetic, or receptor study.

The term "optionally" or "optional" means that the event or circumstance subsequently described may, but does not necessarily, occur, and that the description includes instances where the event or circumstance occurs or does not occur. For example, "$C_{1-6}$ alkyl optionally substituted with halogen or cyano" means that halogen or cyano may, but does not necessarily, be present, and the description includes the instance where alkyl is substituted with halogen or cyano and the instance where alkyl is not substituted with halogen and cyano.

"Substituted" means that one or more, preferably 1 to 6, and more preferably 1 to 3 hydrogen atoms in the

group are independently substituted with a corresponding number of substituents. Those skilled in the art can determine (experimentally or theoretically) possible or impossible substitution without undue effort. For example, it may be unstable when amino or hydroxy having free hydrogen is bound to a carbon atom having an unsaturated (e.g., olefinic) bond.

"Pharmaceutical composition" refers to a mixture containing one or more of the compounds described herein or a physiologically/pharmaceutically acceptable salt or pro-drug thereof, and other chemical components, and other components, for example, physiologically/pharmaceutically acceptable carriers and excipients. The pharmaceutical composition is intended to promote the administration to an organism, so as to facilitate the absorption of the active ingredient, thereby exerting biological activities.

"Pharmaceutically acceptable salt" refers to a salt of the compound disclosed herein, which may be selected from the group consisting of inorganic and organic salts. The salts are safe and effective for use in the body of a mammal and possess the requisite biological activity. The salts may be prepared separately during the final separation and purification of the compound, or by reacting an appropriate group with an appropriate base or acid. Bases commonly used to form pharmaceutically acceptable salts include inorganic bases such as sodium hydroxide and potassium hydroxide, and organic bases such as ammonia. Acids commonly used to form pharmaceutically acceptable salts include inorganic acids and organic acids.

For drugs or pharmacologically active agents, the term "therapeutically effective amount" refers to an amount of a medicament or an agent that is sufficient to provide the desired effect but is non-toxic. The determination of the effective amount varies from person to person. It depends on the age and general condition of a subject, as well as the particular active substance used. The appropriate effective amount in a case may be determined by those skilled in the art in the light of routine tests.

The term "solvate" used herein refers to a substance formed by the physical binding of the compound of the present disclosure to one or more, preferably 1 to 3, solvent molecules, whether organic or inorganic. The physical bonding includes hydrogen bonding. In certain cases, e.g., when one or more, preferably 1 to 3, solvent molecules are incorporated in the crystal lattice of the crystalline solid, the solvate will be isolated. Exemplary solvates include, but are not limited to, hydrates, ethanolates, methanolates, and isopropanolates. Solvation methods are well known in the art.

The term "pharmaceutically acceptable" used herein means that those compounds, materials, compositions, and/or dosage forms that are, within the scope of reasonable medical judgment, suitable for use in contact with the tissues of patients without excessive toxicity, irritation, allergic reaction, or other problems or complications, and are commensurate with a reasonable benefit/risk ratio and effective for the intended use. As used herein, the singular forms "a", "an" and "the" include plural references and vice versa, unless otherwise clearly defined in the context.

When the term "about" is applied to parameters such as pH, concentration, and temperature, it means that the parameter may vary by $\pm 10\%$, and sometimes more preferably within $\pm 5\%$. As will be appreciated by those skilled in the art, when the parameters are not critical, the numbers are generally given for illustrative purposes only and are not intended to be limiting.

## Synthesis Method of Compounds of the Present Disclosure

To achieve the purpose of the present disclosure, the following technical schemes are adopted in the present disclosure:

Scheme 1

A method for preparing the compound of general formula (II) or the pharmaceutically acceptable salt thereof disclosed herein is provided, and the method comprises the following steps:

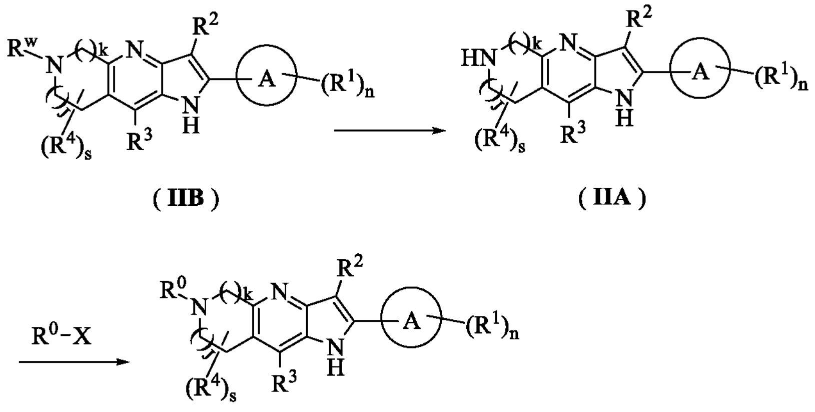


(IIB) (IIA)

(II)

removing the protecting group $R^w$ from a compound of general formula (IIB) or a pharmaceutically acceptable salt thereof under an acidic condition to give the compound of general formula (IIA) or the pharmaceutically acceptable salt thereof; and
conducting a nucleophilic substitution reaction of a compound of general formula (IIA) or a pharmaceutically acceptable salt thereof with $R^0$-X under an alkaline condition to give the compound of general formula (II) or the pharmaceutically acceptable salt thereof,
wherein:

$R^w$ is an amino protecting group, preferably tert-butoxycarbonyl;
X is a leaving group, preferably halogen, and more preferably bromine or iodine;
$R^0$ is selected from the group consisting of alkyl, alkenyl, alkynyl, heteroalkyl, haloalkyl, hydroxyalkyl, and

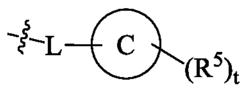
,

wherein the alkyl is optionally substituted with one or more substituents selected from the group consisting of alkoxy, haloalkoxy, cyano, $-NR^7R^8$, $-C(O)NR^7R^8$, cycloalkyl, heterocyclyl, aryl, and heteroaryl;
preferably, $R^0$ is selected from the group consisting of alkyl, alkenyl, alkynyl, heteroalkyl, haloalkyl, hydroxyalkyl, and

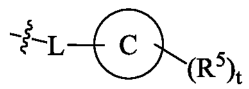
;

L is a chemical bond or alkylene, wherein the alkylene is optionally substituted with one or more substituents selected from the group consisting of halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl;
ring A, ring C, $R^1$ to $R^5$, n, s, t, J, and k are as defined in the compound of general formula (II).

Scheme 2

A method for preparing the compound of general formula (IV) or the pharmaceutically acceptable salt thereof disclosed herein is provided, and the method comprises the following step:

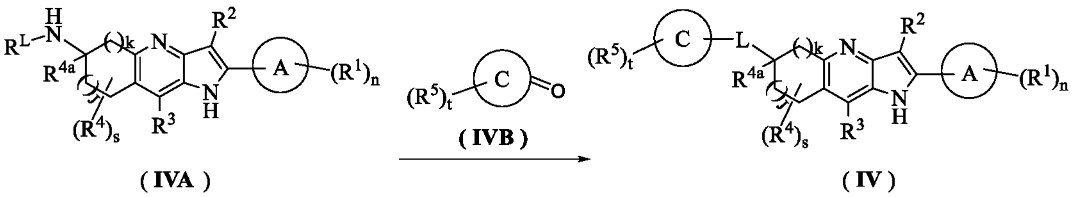

conducting a reductive amination reaction of a compound of general formula (IVA) or a pharmaceutically acceptable salt thereof with a compound of general formula (IVB) in the presence of a reductant to give the compound of general formula (IV) or the pharmaceutically acceptable salt thereof,
wherein:

L is $NR^L$;
$R^L$ is selected from the group consisting of a hydrogen atom, alkyl, alkenyl, alkynyl, haloalkyl, hydroxyalkyl, cycloalkyl, heterocyclyl, aryl, and heteroaryl; preferably, $R^L$ is a hydrogen atom or $C_{1-6}$ alkyl;
ring A, ring C, $R^1$ to $R^5$, $R^{4a}$, n, s, t, J, and k are as defined in general formula (IV).

Scheme 3

A method for preparing the compound of general formula (IIG) or the pharmaceutically acceptable salt thereof disclosed herein is provided, and the method comprises the following step:

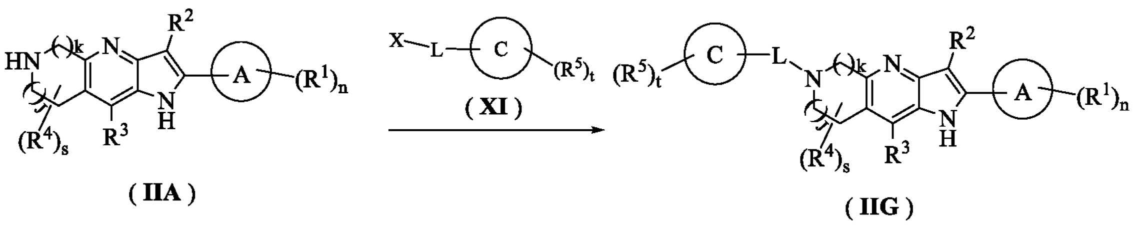

conducting a nucleophilic substitution reaction of a compound of general formula (IIA) or a pharmaceutically acceptable salt thereof with a compound of general formula (XI) under an alkaline condition to give the compound of general formula (IIG) or the pharmaceutically acceptable salt thereof,
wherein:

X is a leaving group, preferably halogen, and more preferably bromine or iodine;
L is a chemical bond or alkylene, wherein the alkylene is optionally substituted with one or more substituents selected from the group consisting of halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl;
ring A, ring C, $R^1$ to $R^5$, n, s, t, J, and k are as defined in the compound of general formula (IIG).

Scheme 4

A method for preparing the compound of general formula (V) or the pharmaceutically acceptable salt thereof disclosed herein is provided, and the method comprises the following step:

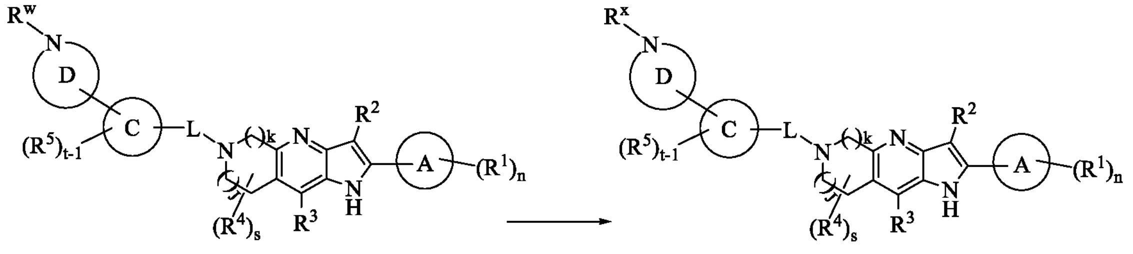

(VA) (V)

removing the protecting group $R^w$ from a compound of general formula (VA) or a pharmaceutically acceptable salt thereof under an acidic condition, and optionally conducting a further reaction with an alkylating reagent $R^x$-W under an alkaline condition to give the compound of general formula (V) or the pharmaceutically acceptable salt thereof,

wherein:

W is a leaving group, preferably halogen;
$R^x$ is selected from the group consisting of a hydrogen atom, $C_{1-6}$ alkyl, and $C_{1-6}$ haloalkyl;
$R^w$ is an amino protecting group, preferably tert-butoxycarbonyl;
ring D is 4- to 12-membered heterocyclyl containing at least one nitrogen atom;
t is 1, 2, 3, or 4;
ring A, ring C, L, $R^1$ to $R^5$, J, k, n, and s are as defined in general formula (V).

Scheme 5

A method for preparing the compound of general formula (VI) or the pharmaceutically acceptable salt thereof disclosed herein is provided, and the method comprises the following step:

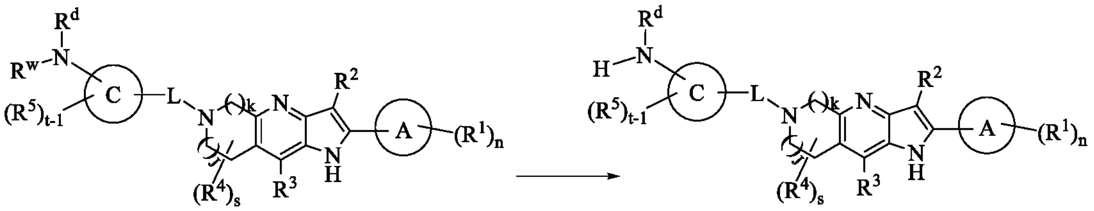

(VIA) (VI)

removing the protecting group $R^w$ from a compound of general formula (VIA) or a pharmaceutically acceptable salt thereof under an acidic condition to give the compound of general formula (VI) or the pharmaceutically acceptable salt thereof,

wherein:

$R^w$ is an amino protecting group, preferably tert-butoxycarbonyl;
t is 1, 2, 3, or 4;
ring A, ring C, L, $R^1$ to $R^5$, $R^d$, J, k, n, and s are as defined in general formula (VI).

Scheme 6

A method for preparing the compound of general formula (IIA) or the pharmaceutically acceptable salt thereof disclosed herein is provided, and the method comprises the following step:

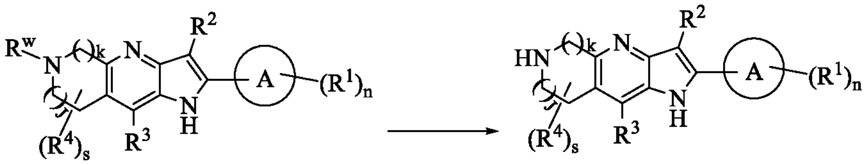

( IIB ) ( IIA )

removing the protecting group $R^w$ from a compound of general formula (IIB) or a pharmaceutically acceptable salt thereof under an acidic condition to give the compound of general formula (IIA) or the pharmaceutically acceptable salt thereof,
wherein:

$R^w$ is an amino protecting group, preferably tert-butoxycarbonyl;
ring A, $R^1$ to $R^4$, n, s, J, and k are as defined in general formula (IIA).

Scheme 7

A method for preparing the compound of general formula (II) or the pharmaceutically acceptable salt thereof disclosed herein is provided, and the method comprises the following step:

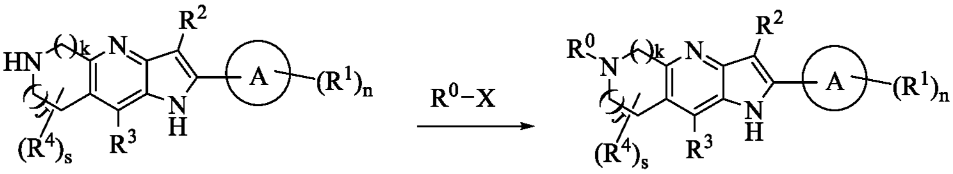


( IIA ) ( II )

conducting a nucleophilic substitution reaction of a compound of general formula (IIA) or a pharmaceutically acceptable salt thereof with $R^0$-X under an alkaline condition to give the compound of general formula (II) or the pharmaceutically acceptable salt thereof,
wherein:

X is a leaving group, preferably halogen, and more preferably bromine or iodine;
$R^0$ is selected from the group consisting of alkyl, alkenyl, alkynyl, heteroalkyl, haloalkyl, hydroxyalkyl, and

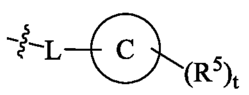,

wherein the alkyl is optionally substituted with one or more substituents selected from the group consisting of alkoxy, haloalkoxy, cyano, $-NR^7R^8$, $-C(O)NR^7R^8$, cycloalkyl, heterocyclyl, aryl, and heteroaryl; preferably, $R^0$ is selected from the group consisting of alkyl, alkenyl, alkynyl, heteroalkyl, haloalkyl, hydroxyalkyl, and

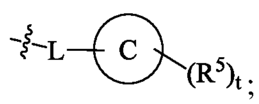

L is a chemical bond or alkylene, wherein the alkylene is optionally substituted with one or more substituents selected from the group consisting of halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl;

ring A, ring C, $R^1$ to $R^5$, n, s, t, J, and k are as defined in the compound of general formula (II).

Scheme 8

A method for preparing the compound of general formula (IIB) or the pharmaceutically acceptable salt thereof disclosed herein is provided, and the method comprises the following steps:

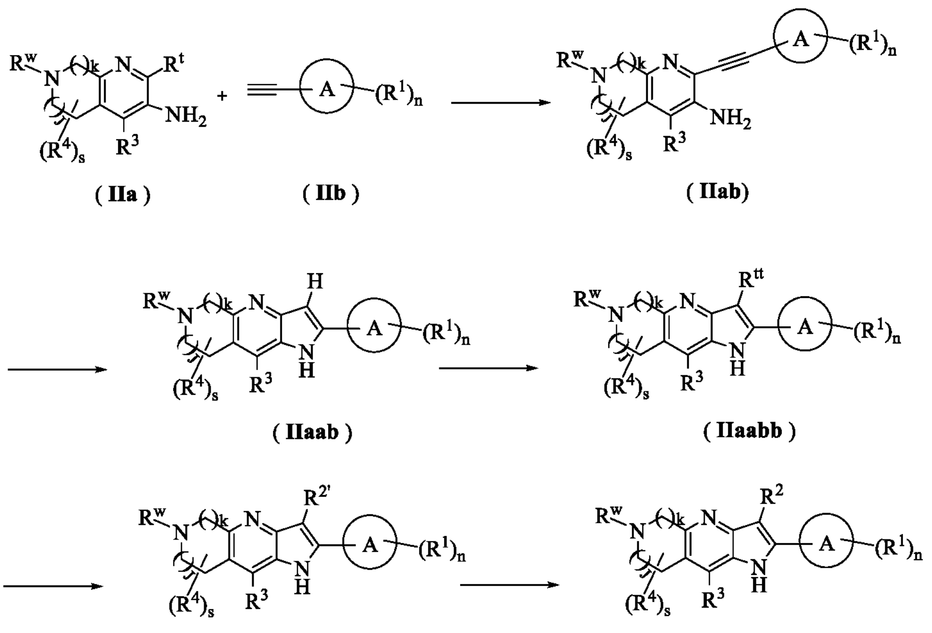

( IIa ) ( IIb ) ( IIab) ( IIaab ) ( IIaabb ) ( IIaaabb ) ( IIB )

conducting an addition reaction of a compound of general formula (IIa) or a pharmaceutically acceptable salt thereof with a compound of general formula (IIb) (preferably in the presence of cuprous iodide, bis(triphenylphosphine)palladium(II) dichloride, and triethylamine) to give a compound of formula (IIab) or a pharmaceutically acceptable salt thereof,

conducting an intramolecular cyclization reaction of the compound of general formula (IIab) or the pharmaceutically acceptable salt thereof under an alkaline condition to give a compound of formula (IIaab) or a pharmaceutically acceptable salt thereof,

conducting a reaction of the compound of general formula (IIaab) or the pharmaceutically acceptable salt thereof with a halogenating reagent (e.g., N-bromosuccinimide) to give a compound of formula (IIaabb) or a pharmaceutically acceptable salt thereof,

conducting a coupling reaction of the compound of general formula (IIaabb) or the pharmaceutically acceptable salt thereof with isopropenylboronic acid pinacol ester or 3,6-dihydro -2*H*-pyran-4-boronic acid pinacol ester (preferably in the presence of [1,1'-bis(diphenylphosphino)ferrocene]palladium(II) dichloride and anhydrous tripotassium phosphate) to give a compound of formula (IIaaabb) or a pharmaceutically acceptable salt thereof, and
conducting a reduction reaction of the compound of general formula (IIaaabb) or the pharmaceutically acceptable salt thereof (preferably in a palladium on carbon and hydrogen atmosphere) to give the compound of formula (IIB) or the pharmaceutically acceptable salt thereof,
wherein:

$R^w$ is an amino protecting group, preferably tert-butoxycarbonyl;
$R^t$ or $R^{tt}$ is halogen, preferably bromine;
$R^{2'}$ is

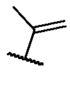 or 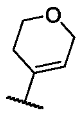 ;

$R^2$ is isopropyl or

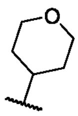 ;

ring A, $R^1$, $R^3$, $R^4$, n, s, J, and k are as defined in general formula (IIB).

Scheme 9

A method for preparing the compound of general formula (IIB) or the pharmaceutically acceptable salt thereof disclosed herein is provided, and the method comprises the following
step:

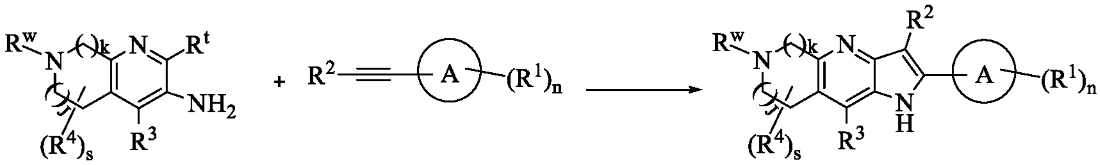

( **IIa** ) ( **IIbb** ) ( **IIB** )

conducting a Larock indole synthesis reaction of a compound of general formula (IIa) or a pharmaceutically acceptable salt thereof with a compound of general formula (IIbb) (preferably in the presence of anhydrous lithium chloride, potassium carbonate, and tetrakis(triphenylphosphine)palladium(0)) to give the compound of general formula (IIB) or the pharmaceutically acceptable salt thereof,
wherein:

$R^w$ is an amino protecting group, preferably tert-butoxycarbonyl;
$R^t$ is halogen, preferably bromine;
ring A, $R^1$ to $R^4$, n, s, J, and k are as defined in general formula (IIB).

In the above synthetic schemes, reagents that provide acidic conditions include, but are not limited to, hydrogen chloride, hydrogen chloride in 1,4-dioxane, trifluoroacetic acid, formic acid, acetic acid, hydrochloric acid, sulfuric acid, methanesulfonic acid, nitric acid, phosphoric acid, p-toluenesulfonic acid, $Me_3SiCl$, and TMSOTf; trifluoroacetic acid or hydrogen chloride in 1,4-dioxane is preferred.

In the above synthetic schemes, reagents that provide alkaline conditions include organic bases and inorganic

bases, wherein the organic bases include, but are not limited to, triethylamine, *N*,*N*-diisopropylethylamine, n-butyllithium, lithium diisopropylamide, potassium acetate, sodium acetate, 1,8-diazabicyclo[5.4.0]undec-7-ene, sodium ethoxide, sodium tert-butoxide, or potassium tert-butoxide, and the inorganic bases include, but are not limited to, sodium hydride, potassium phosphate, sodium carbonate, potassium carbonate, cesium carbonate, sodium hydroxide, lithium hydroxide monohydrate, lithium hydroxide, and potassium hydroxide; potassium tert-butoxide, 1,8-diazabicyclo[5.4.0]undec-7-ene, potassium carbonate, and sodium acetate are preferred.

In the above synthetic schemes, the reductive amination reaction is preferably conducted in the presence of a reductant and a weak acid; the reductant includes, but is not limited to, sodium triacetoxyborohydride, sodium borohydride, lithium borohydride, sodium cyanoborohydride, sodium acetylborohydride, and the like, and sodium triacetoxyborohydride is preferred; the weak acid is preferably acetic acid.

The above synthetic schemes are preferably carried out in solvents, including but not limited to: ethylene glycol dimethyl ether, acetic acid, methanol, ethanol, acetonitrile, n-butanol, toluene, tetrahydrofuran, dichloromethane, petroleum ether, ethyl acetate, n-hexane, dimethyl sulfoxide, 1,4-dioxane, water, *N*,*N*-dimethylacetamide, *N*,*N*-dimethylformamide, and mixtures thereof.

## DETAILED DESCRIPTION

The following examples further illustrate the present disclosure, but the present disclosure is not limited thereto.

## Examples

The structures of the compounds were determined by nuclear magnetic resonance (NMR) spectroscopy and/or mass spectrometry (MS). NMR shifts ($\delta$) are given in $10^{-6}$ (ppm). NMR spectra were measured on Bruker AVANCE NEO 500M, with deuterated dimethyl sulfoxide (DMSO-$d_6$), deuterated chloroform ($CDCl_3$), and deuterated methanol ($CD_3OD$) as determination solvents and tetramethylsilane (TMS) as internal standard.

MS analyses were performed on an Agilent 1200/1290 DAD-6110/6120 Quadrupole MS liquid chromatography-mass spectrometry system (manufacturer: Agilent; MS model: 6110/6120 Quadrupole MS), Waters ACQuity UPLC-QD/SQD (manufacturer: Waters, MS model: Waters ACQuity Qda Detector/Waters SQ Detector) and THERMO Ultimate 3000-Q Exactive (manufacturer: THERMO, MS model: THERMO Q Exactive).

High performance liquid chromatography (HPLC) analyses were performed on Agilent HPLC 1200DAD, Agilent HPLC 1200VWD, and Waters HPLC e2695-2489 high performance liquid chromatographs.

Chiral HPLC analyses were performed on an Agilent 1260 DAD high performance liquid chromatograph.

Preparative HPLC was performed on Waters 2767, Waters 2767-SQ Detecor2, Shimadzu LC-20AP, and Gilson-281 preparative chromatographs.

Preparative chiral chromatography was performed on a Shimadzu LC-20AP preparative chromatograph.

The CombiFlash preparative flash chromatograph used was CombiFlash Rf200 (TELEDYNE ISCO).

Yantai Huanghai HSGF254 or Qingdao GF254 silica gel plates, 0.15-0.2 mm layer thickness, were adopted for thin-layer chromatography (TLC) analysis and 0.4-0.5 mm layer thickness for TLC separation and purification.

Silica gel column chromatography generally used 200- to 300-mesh silica gel (Huanghai, Yantai) as the carrier.

The mean inhibition of kinase and the $IC_{50}$ value were determined on a NovoStar microplate reader (BMG, Germany).

Known starting materials described herein may be synthesized using or according to methods known in the art, or may be purchased from ABCR GmbH & Co. KG, Acros Organics, Aldrich Chemical Company, Accela ChemBio Inc., Chembee Chemicals, and other companies.

In the examples, the reactions could all be conducted in an argon atmosphere or a nitrogen atmosphere unless otherwise specified.

The argon atmosphere or nitrogen atmosphere means that the reaction flask is connected to a balloon containing about 1 L of argon or nitrogen.

The hydrogen atmosphere means that the reaction flask is connected to a balloon containing about 1 L of hydrogen.

Parr 3916EKX hydrogenator, Qinglan QL-500 hydrogenator, or HC2-SS hydrogenator was used in the pressurized hydrogenation reactions.

Hydrogenation reactions generally involve 3 cycles of vacuumization and hydrogen purging.

A CEM Discover-S 908860 microwave reactor was used in the microwave reactions.

In the examples, a solution refers to an aqueous solution unless otherwise specified.

In the examples, the reaction temperature is room temperature, i.e., 20 °C to 30 °C, unless otherwise specified.

The monitoring of the reaction progress in the examples was conducted by thin-layer chromatography (TLC). The developing solvent for reactions, the eluent system for column chromatography purification and the developing

solvent system for thin-layer chromatography included: A: n-hexane/ethyl acetate system, and B: dichloromethane/methanol system. The volume ratio of the solvents was adjusted according to the polarity of the compound, or by adding a small amount of basic or acidic reagents such as triethylamine and acetic acid.

Example 1

2-(2,6-Dimethylpyridin-4-yl)-3-isopropyl-5,6,7,8-tetrahydro-1*H*-pyrrolo[3,2-*b*][1,7]nap hthyridine **1**

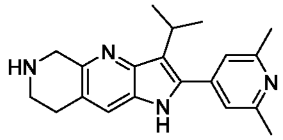

**1**

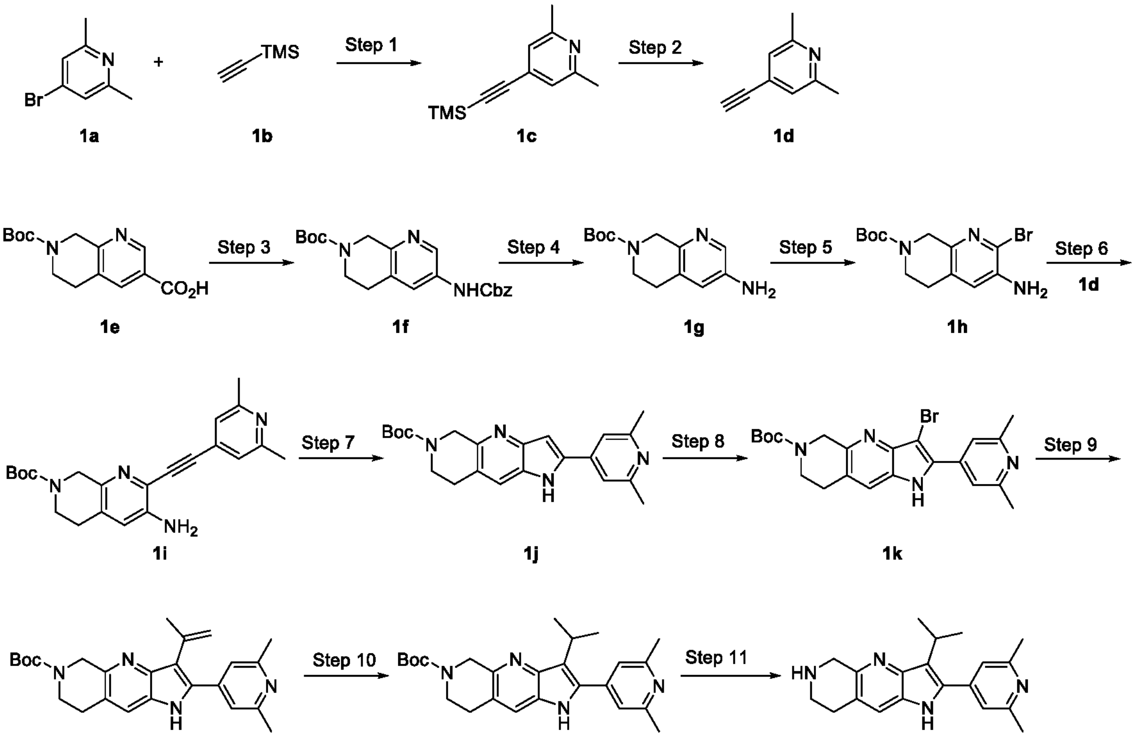


**1a** **1b** **1c** **1d** **1e** **1f** **1g** **1h** **1i** **1j** **1k** **1l** **1m** **1**

Step 1

2,6-Dimethyl-4-((trimethylsilyl)ethynyl)pyridine **1c** 4-Bromo-2,6-dimethylpyridine **1a** (5.0 g, 26.9 mmol, Bide Pharmatech Ltd.), trimethylsilylacetylene **1b** (8.0 g, 81.5 mmol, Accela ChemBio (Shanghai)), cuprous iodide (550 mg, 2.89 mmol, Alfa Aesar (China)), bis(triphenylphosphine)palladium(II) dichloride (1.0 g, 1.42 mmol, J&K Chemical), and triethylamine (8.15 g, 80.7 mmol) were dissolved in anhydrous *N*,*N*-dimethylformamide (100 mL), and the reaction was stirred at 45 °C for 16 h in a nitrogen atmosphere. The reaction mixture was cooled to room temperature and concentrated under reduced pressure. Ethyl acetate (100 mL) was added, and the mixture was filtered and washed. The filtrate was concentrated under reduced pressure, and the residue was purified by silica gel column chromatography with eluent system A to give the title product **1c** (5.3 g, yield: 97%).

MS m/z (ESI): 204.0 [M+1].

Step 2

4-Ethynyl-2,6-dimethylpyridine **1d**

Compound **1c** (5.3 g, 26.1 mmol) was dissolved in methanol (100 mL), and potassium carbonate (5.25 g, 38.0 mmol) was added. The mixture was stirred at room temperature for 2.0 h, filtered, and concentrated under reduced pressure. The residue was purified by silica gel column chromatography with eluent system A to give the title product **1d** (1.7 g, yield: 49.7%).
$^{1}$H NMR (500 MHz, $CDCl_3$) $\delta$ 7.06 (s, 2H), 3.22 (s, 1H), 2.53 (s, 6H).

Step 3

tert-Butyl

3-(((benzyloxy)carbonyl)amino)-5,8-dihydro-1,7-naphthyridine-7(6*H*)-carboxylate **1f** 7-(tert-Butoxycarbonyl)-5,6,7,8-tetrahydro-1,7-naphthyridine-3-carboxylic acid **1e** (4.17 g, 15.0 mmol, Jiangsu Aikon Biopharmaceutical), diphenyl phosphoryl azide (4.35 g, 17.9 mmol, Bide Pharmatech Ltd.), and *N*,*N*-diisopropylethylamine (2.72 g, 21.0 mmol) were dissolved in anhydrous toluene (85 mL). The mixture was stirred at room temperature for 1.0 h in a nitrogen atmosphere. Benzyl alcohol (8.0 g, 74.0 mmol, Sinopharm) was added, and the mixture was stirred at 105 °C for 3.0 h in a nitrogen atmosphere. Water (100 mL) was added to the reaction mixture, and extraction was performed with ethyl acetate (100 mL × 3). The organic phases were combined and concentrated under reduced pressure, and the residue was purified by silica gel column chromatography with eluent system A to give the title product **1f** (5.5 g, yield: 95.7%).
MS m/z (ESI): 383.9 [M+1].

Step 4

tert-Butyl 3-amino-5,8-dihydro-1,7-naphthyridine-7(6*H*)-carboxylate **1g**

Compound **1f** (6.8 g, 17.7 mmol) was dissolved in methanol (100 mL), and palladium on carbon (1.89 g, 1.77 mmol, 10% purity, adamas) was added. The reaction was stirred at room temperature for 2.0 h in a hydrogen atmosphere. The reaction mixture was filtered and concentrated under reduced pressure to give the crude title product **1g** (4.42 g, yield: 100%). The product was directly used in the next step without purification.
MS m/z (ESI): 250.0 [M+1].

Step 5

tert-Butyl 3-amino-2-bromo-5,8-dihydro-1,7-naphthyridine-7(6*H*)-carboxylate **1h**

The crude compound **1g** (4.42 g, 17.7 mmol) was dissolved in *N*,*N*-dimethylformamide (120 mL), and *N*-bromosuccinimide (3.35 g, 18.8 mmol, Accela ChemBio (Shanghai)) was added. The mixture was stirred at room temperature for 15 h. The reaction mixture was concentrated under reduced pressure, and the residue was purified by silica gel column chromatography with eluent system A to give the title product **1h** (4.7 g, yield: 80.8%).
MS m/z (ESI): 328.0 [M+1].

Step 6

tert-Butyl

3-amino-2((2,6-dimethylpyridin-4-yl)ethynyl)-5,8-dihydro-1,7-naphthyridine-7(6*H*)-car boxylate **1i**

Compound **1h** (1.35 g, 4.11 mmol), compound **1d** (600 mg, 4.57 mmol), cuprous iodide (160 mg, 0.84 mmol, Alfa Aesar (China)), bis(triphenylphosphine)palladium(II) dichloride (290 mg, 0.41 mmol, J&K Chemical), and triethylamine (1.25 g, 12.4 mmol) were dissolved in anhydrous *N*,*N*-dimethylformamide (20 mL), and the reaction was stirred at 40 °C for 16 h in a nitrogen atmosphere. Water (50 mL) was added to the reaction mixture, and extraction was performed with ethyl acetate (50 mL × 4). The organic phases were combined and concentrated under reduced pressure, and the residue was purified by silica gel column chromatography with eluent system B to give the title product **1i** (1.50 g, yield: 96.4%).
MS m/z (ESI): 379.0 [M+1].

Step 7

tert-Butyl

2-(2,6-dimethylpyridin-4-yl)-1,5,7,8-tetrahydro-6*H*-pyrrolo[3,2-*b*][1,7]naphthyridine-6-carboxylate **1j**

Compound **1i** (1.40 g, 3.70 mmol) and potassium tert-butoxide (2.10 g, 18.7 mmol, Accela ChemBio (Shanghai)) were dissolved in anhydrous N-methylpyrrolidinone (20 mL). The reaction was stirred at 40 °C for 16 h in a nitrogen atmosphere. Saturated aqueous ammonium chloride solution (100 mL) was added, and extraction was performed with ethyl acetate (80 mL × 4). The organic phases were combined, washed with saturated sodium chloride solution (50 mL × 4), and concentrated under reduced pressure. The residue was purified by silica gel column chromatography with eluent system B to give the title product **1j** (900 mg, yield: 64.3%).

MS m/z (ESI): 378.9 [M+1].

Step 8

tert-Butyl

3-bromo-2-(2,6-dimethylpyridin-4-yl)-1,5,7,8-tetrahydro-6*H*-pyrrolo[3,2-*b*][1,7]naphth yridine-6-carboxylate **1k**

Compound **1j** (900 mg, 2.38 mmol) was dissolved in *N*,*N*-dimethylformamide (20 mL), the solution was cooled to 0 °C, and *N*-bromosuccinimide (430 mg, 2.42 mmol, Accela ChemBio (Shanghai)) was added. The mixture was stirred at 0 °C for 2.0 h. Water (50 mL) was added to the reaction mixture, and extraction was performed with ethyl acetate (50 mL × 4). The organic phases were combined and concentrated under reduced pressure, and the residue was purified by silica gel column chromatography with eluent system B to give the title product **1k** (1.08 g, yield: 99.3%).

MS m/z (ESI): 457.0 [M+1].

Step 9

tert-Butyl

2-(2,6-dimethylpyridin-4-yl)-3-(prop-1-en-2-yl)-1,5,7,8-tetrahydro-6*H*-pyrrolo[3,2-*b*][1, 7]naphthyridine-6-carboxylate **1l**

Compound **1k** (1.08 g, 2.36 mmol), isopropenylboronic acid pinacol ester (1.5 g, 8.93 mmol, Accela ChemBio (Shanghai)), [1,1'-bis(diphenylphosphino)ferrocene]palladium(II) dichloride (180 mg, 0.25 mmol, Bide Pharmatech Ltd.), and anhydrous tripotassium phosphate (1.50 g, 7.07 mmol) were dissolved in anhydrous dioxane (16.0 mL) and water (4.0 mL), and the reaction was stirred at 80 °C for 16 h in a nitrogen atmosphere. The reaction mixture was cooled to room temperature. Water (15 mL) was added, and extraction was performed with ethyl acetate (30 mL × 4). The organic phases were combined and concentrated under reduced pressure, and the residue was purified by silica gel column chromatography with eluent system B to give the title product **1l** (900 mg, yield: 91.1%).

MS m/z (ESI): 419.0 [M+1].

Step 10

tert-Butyl

2-(2,6-dimethylpyridin-4-yl)-3-isopropyl-1,5,7,8-tetrahydro-6*H*-pyrrolo[3,2-*b*][1,7]naph thyridine-6-carboxylate **1m**

Compound **1l** (900 mg, 2.15 mmol) was dissolved in methanol (30 mL), and palladium on carbon (500 mg, 0.47 mmol, 10% purity, adamas) was added. The reaction was stirred at room temperature for 16 h in a hydrogen atmosphere. The reaction mixture was filtered, and the filtrate was concentrated under reduced pressure. The residue was dissolved in methanol (30 mL), and palladium on carbon (500 mg, 0.47 mmol, 10% purity, adamas) was added. The reaction was stirred at room temperature for 16 h in a hydrogen atmosphere. The reaction mixture was filtered, and the filtrate was concentrated under reduced pressure. The residue was purified by silica gel column chromatography with eluent system A to give the title product **1m** (350 mg, yield: 38.7%).

MS m/z (ESI): 421.0 [M+1].

Step 11

2-(2,6-Dimethylpyridin-4-yl)-3-isopropyl-5,6,7,8-tetrahydro-1*H*-pyrrolo[3,2-*b*][1,7]nap hthyridine **1**

Compound **1m** (65.0 mg, 0.15 mmol) was dissolved in dichloromethane (4 mL) and trifluoroacetic acid (2 mL). The mixture was stirred at room temperature for 1 h. The reaction mixture was concentrated under reduced pressure, and a 7 M ammonia/methanol solution (5 mL) was added to the residue. The mixture was concentrated under reduced pressure, and the residue was purified by high performance liquid chromatography (column: Boston Phlex Prep C18, 5 μm 30 × 150 mm; mobile phases: A-water (10 mmol of ammonium bicarbonate):B-acetonitrile, 28-48% acetonitrile, 15 min of gradient elution, flow rate: 30 mL/min) to give the title product **1** (26.0 mg, yield: 52.5%).
MS m/z (ESI): 321.2 [M+1].
$^1$H NMR (500 MHz, $CD_3OD$) δ 7.47 (s, 1H), 7.27 (s, 2H), 4.19 (s, 2H), 3.41 (m, 1H), 3.18 (t, 2H), 3.04 (t, 2H), 2.59 (s, 6H), 1.54 (d, 6H).

Example 2

2-(2,6-Dimethylpyridin-4-yl)-3,6-diisopropyl-5,6,7,8-tetrahydro-1*H*-pyrrolo[3,2-*b*][1,7] naphthyridine

**2**

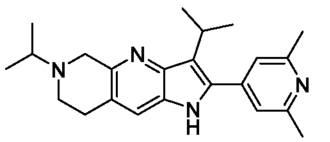

**2**

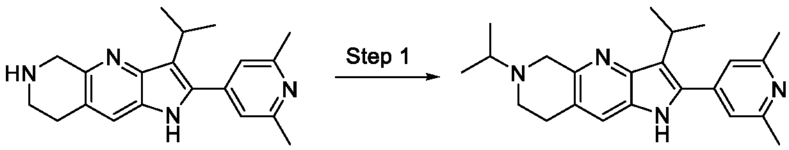


**1** **2**

Step 1

2-(2,6-Dimethylpyridin-4-yl)-3,6-diisopropyl-5,6,7,8-tetrahydro-1*H*-pyrrolo[3,2-*b*][1,7] naphthyridine **2**

Compound **1** (50 mg, 0.16 mmol) and 2-iodopropane (100 mg, 0.59 mmol) were dissolved in acetonitrile (5 mL), and potassium carbonate (100 mg, 0.72 mmol) was added. The mixture was stirred at 80 °C for 2.0 h, filtered, and concentrated under reduced pressure. The residue was purified by high performance liquid chromatography (column: Boston Phlex Prep C18, 5 μm 30 × 150 mm; mobile phases: A-water (10 mmol of ammonium bicarbonate):B-acetonitrile, 40-60% acetonitrile, 15 min of gradient elution, flow rate: 30 mL/min) to give the title product **2** (28.0 mg, yield: 49.5%).
MS m/z (ESI): 363.2 [M+1].
$^1$H NMR (500 MHz, $CD_3OD$) δ 7.49 (s, 1H), 7.27 (s, 2H), 3.98 (s, 2H), 3.42 (m, 1H), 3.08 (t, 2H), 3.01 (m, 1H), 2.93 (t, 2H), 2.59 (s, 6H), 1.54 (d, 6H), 1.26 (d, 6H).

Example 3

2-(2,6-Dimethylpyridin-4-yl)-3-isopropyl-*N*-(tetrahydrofuran-3-yl)-5,6,7,8-tetrahydro-1 *H*-pyrrolo[3,2-*b*]quinolin-6-amine **3**

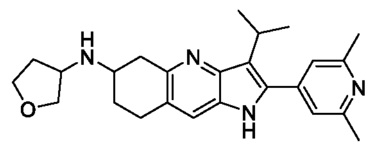

3

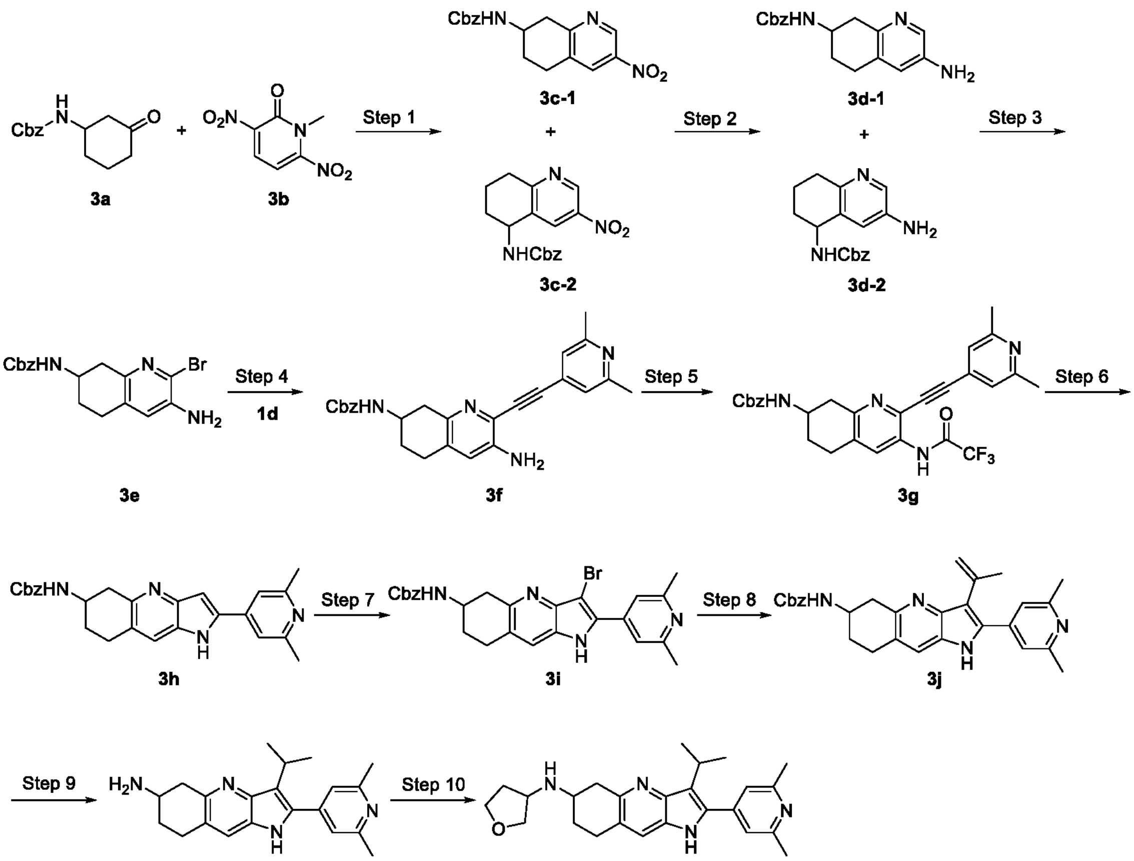


3a 3b 3c-1 + 3c-2 3d-1 + 3d-2 3e 3f 3g 3h 3i 3j 3k 3

Step 1

Benzyl (3-nitro-5,6,7,8-tetrahydroquinolin-7-yl)carbamate **3c-1**

Benzyl (3-nitro-5,6,7,8-tetrahydroquinolin-5-yl)carbamate **3c-2**

Benzyl (3-oxocyclohexyl)carbamate **3a** (3.0 g, 12.1 mmol, Bide Pharmatech Ltd.) and 1-methyl-3,6-dinitropyridin-2(1*H*)-one **3b** (3.0 g, 15.1 mmol, PharmaBlock Sciences (Nanjing)) were dissolved in a 7 M ammonia/methanol solution (15 mL) and methanol (15 mL), and the mixture was stirred at 90 °C for 2.0 h under a sealed condition. The reaction mixture was concentrated under reduced pressure, and the residue was dissolved in dichloromethane (150 mL) and washed with water (100 mL) and saturated sodium chloride solution (100 mL). The organic phase was concentrated under reduced pressure, and the residue was purified by silica gel column chromatography with eluent system A to give a mixture of the title products **3c-1** and **3c-2** (3.5 g, yield: 88.1%).

MS m/z (ESI): 327.9 [M+1].

Step 2

Benzyl (3-amino-5,6,7,8-tetrahydroquinolin-7-yl)carbamate **3d-1**

Benzyl (3-amino-5,6,7,8-tetrahydroquinolin-5-yl)carbamate **3d-2**

A mixture of the compounds **3c-1** and **3c-2** (8.0 g, 24.4 mmol) was dissolved in ethanol (100 mL) and water

(25 mL). Iron powder (12.0 g, 215 mmol, Sinopharm) and ammonium chloride (13.0 g, 241 mmol) were added. The reaction was stirred at 80 °C for 3.0 h. The reaction mixture was cooled to room temperature and concentrated under reduced pressure. Water (200 mL) was added, and extraction was performed with ethyl acetate (200 mL × 4). The organic phases were combined and concentrated under reduced pressure to give a crude mixture of the title products **3d-1** and **3d-2** (7.26 g, yield: 99.9%). The crude products were directly used in the next step without purification.

MS m/z (ESI): 298.0 [M+1].

Step 3

Benzyl 2-(3-amino-2-bromo-5,6,7,8-tetrahydroquinolin-7-yl)carbamate **3e**

The crude mixture of the compounds **3d-1** and **3d-2** (7.3 g, 24.6 mmol) was dissolved in N,N-dimethylformamide (100 mL), and N-bromosuccinimide (4.50 g, 25.3 mmol, Accela ChemBio (Shanghai)) was added. The mixture was stirred at 0 °C for 2.0 h. The reaction mixture was warmed to room temperature and concentrated under reduced pressure, and the residue was purified by silica gel column chromatography with eluent system A to give the title product **3e** (3.5 g, yield: 37.9%).

MS m/z (ESI): 376.0 [M+1].

Step 4

Benzyl

(3-amino-2((2,6-dimethylpyridin-4-yl)ethynyl)-5,6,7,8-tetrahydroquinolin-7-yl)carbama **te 3f**

Compound **3e** (1.0 g, 2.66 mmol), compound **1d** (400 mg, 3.05 mmol), cuprous iodide (105 mg, 0.55 mmol, Alfa Aesar (China)), bis(triphenylphosphine)palladium(II) dichloride (190 mg, 0.27 mmol, J&K Chemical), and triethylamine (810 mg, 8.02 mmol) were dissolved in anhydrous *N*,*N*-dimethylformamide (20 mL), and the reaction was stirred at 40 °C for 16 h in a nitrogen atmosphere. The reaction mixture was cooled to room temperature. Water (50 mL) was added, and extraction was performed with ethyl acetate (50 mL × 4). The organic phases were combined and concentrated under reduced pressure, and the residue was purified by silica gel column chromatography with eluent system B to give the title product **3f** (300 mg, yield: 26.5%).

MS m/z (ESI): 426.9 [M+1].

Step 5

Benzyl

(2-((2,6-dimethylpyridin-4-yl)ethynyl)-3-(2,2,2-trifluoroacetylamino)-5,6,7,8-tetrahydro quinolin-7-yl)carbamate **3g**

Compound **3f** (580 mg, 1.36 mmol) was dissolved in anhydrous dioxane (20 mL), and potassium carbonate (500 mg, 3.62 mmol) and trifluoroacetic anhydride (450 mg, 2.14 mmol, adamas) were added. The mixture was stirred at room temperature for 3.0 h. Water (30 mL) was added, and extraction was performed with ethyl acetate (30 mL × 4). The organic phases were combined and concentrated under reduced pressure, and the residue was purified by silica gel column chromatography with eluent system A to give the title product **3g** (270 mg, yield: 38.0%).

MS m/z (ESI): 522.9 [M+1].

Step 6

Benzyl

(2-(2,6-dimethylpyridin-4-yl)-5,6,7,8-tetrahydro-1*H*-pyrrolo[3,2-*b*]quinolin-6-yl)carba mate **3h**

Compound **3g** (270 mg, 0.52 mmol), cuprous iodide (100 mg, 0.53 mmol, Alfa Aesar (China)), and triethylamine (200 mg, 1.98 mmol) were dissolved in anhydrous *N*,*N*-dimethylformamide (10 mL), and the reaction was stirred at 105 °C for 3.0 h in a nitrogen atmosphere. Water (20 mL) was added, and extraction was performed with ethyl acetate (20 mL × 4). The organic phases were combined and concentrated under reduced pressure, and the residue was purified by silica gel column chromatography with eluent system B to give the title product **3h** (220 mg, yield: 99.8%).

MS m/z (ESI): 426.9 [M+1].

Step 7

Benzyl

(3-bromo-2-(2,6-dimethylpyridin-4-yl)-5,6,7,8-tetrahydro-1*H*-pyrrolo[3,2-*b*]quinolin-6-yl)carbamate **3i**

Compound **3h** (220 mg, 0.51 mmol) was dissolved in *N*,*N*-dimethylformamide (5.0 mL), the solution was cooled to 0 °C, and *N*-bromosuccinimide (95 mg, 0.53 mmol, Accela ChemBio (Shanghai)) was added. The mixture was stirred at room temperature for 2.0 h, The reaction mixture was concentrated under reduced pressure, and the residue was purified by silica gel column chromatography with eluent system B to give the title product **3i** (225 mg, yield: 86.5%).

MS m/z (ESI): 505.8 [M+1].

Step 8

Benzyl

(2-(2,6-dimethylpyridin-4-yl)-3-(prop-1-en-2-yl)-5,6,7,8-tetrahydro-1*H*-pyrrolo[3,2-*b*]q uinolin-6-yl)carbamate **3j**

Compound **3i** (225 mg, 0.45 mmol), isopropenylboronic acid pinacol ester (200 mg, 1.19 mmol, Accela ChemBio (Shanghai)), [1,1'-bis(diphenylphosphino)ferrocene]palladium(II) dichloride (35 mg, 0.05 mmol, Bide Pharmatech Ltd.), and anhydrous tripotassium phosphate (285 mg, 1.34 mmol) were dissolved in anhydrous dioxane (4.0 mL) and water (1.0 mL), and the reaction was stirred at 80 °C for 16 h in a nitrogen atmosphere. The reaction mixture was cooled to room temperature. Water (15 mL) was added, and extraction was performed with ethyl acetate (20 mL × 4). The organic phases were combined and concentrated under reduced pressure, and the residue was purified by silica gel column chromatography with eluent system A to give the title product **3j** (110 mg, yield: 53%).

MS m/z (ESI): 467.0 [M+1].

Step 9

2-(2,6-Dimethylpyridin-4-yl)-3-isopropyl-5,6,7,8-tetrahydro-1*H*-pyrrolo[3,2-*b*]quinolin-6-amine **3k**

Compound **3j** (110 mg, 0.24 mmol) was dissolved in methanol (10 mL), and palladium on carbon (200 mg, 0.19 mmol, 10% purity, adamas) was added. The reaction was stirred at room temperature for 18 h in a hydrogen atmosphere. The reaction mixture was filtered and concentrated under reduced pressure to give the crude title product **3k** (50 mg, yield: 63.4%). The product was directly used in the next step without purification.

MS m/z (ESI): 335.0 [M+1].

Step 10

2-(2,6-Dimethylpyridin-4-yl)-3-isopropyl-*N*-(tetrahydrofuran-3-yl)-5,6,7,8-tetrahydro-1 *H*-pyrrolo[3,2-b]quinolin-6-amine **3**

The crude compound **3k** (50.0 mg, 0.15 mmol) and dihydrofuran-3(2*H*)-one (20 mg, 0.23 mmol, Accela ChemBio (Shanghai)) were dissolved in dichloromethane (5.0 mL), and the mixture was stirred at room temperature for 1 h. Sodium triacetoxyborohydride (70 mg, 0.33 mmol, Accela ChemBio (Shanghai)) was added, and the mixture was stirred at room temperature for 16 h. The reaction mixture was concentrated under reduced pressure, and the residue was purified by high performance liquid chromatography (column: Welch Ultimate XB-C18, 5 μm, 30 mm × 150 mm; mobile phases: A-water (10 mmol of ammonium bicarbonate):B-acetonitrile, 20-95% acetonitrile, 20 min of gradient elution, flow rate: 30 mL/min) to give the title product **3** (5.0 mg, yield: 8.3%).

MS m/z (ESI): 405.0 [M+1].

$^1$H NMR (500 MHz, $CD_3OD$) δ 7.45 (s, 1H), 7.27 (s, 2H), 4.01-3.94 (m, 2H), 3.82 (m, 1H), 3.73 (m, 1H), 3.62 (m, 1H), 3.46-3.39 (m, 2H), 3.15 (m, 1H), 3.04-3.00 (m, 2H), 2.84 (m, 1H), 2.58 (s, 6H), 2.28-2.20 (m, 2H), 1.85 (m, 1H), 1.67 (m, 1H), 1.54 (d, 6H).

Example 4

2-(2,6-Dimethylpyridin-4-yl)-3-isopropyl-6-(2-(6-(piperazin-4-yl)pyridin-3-yl)ethyl)-5, 6,7,8-tetrahydro-1*H*-pyrrolo[3,2-*b*][1,7]naphthyridine **4**

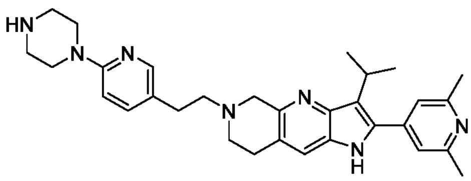

**4**

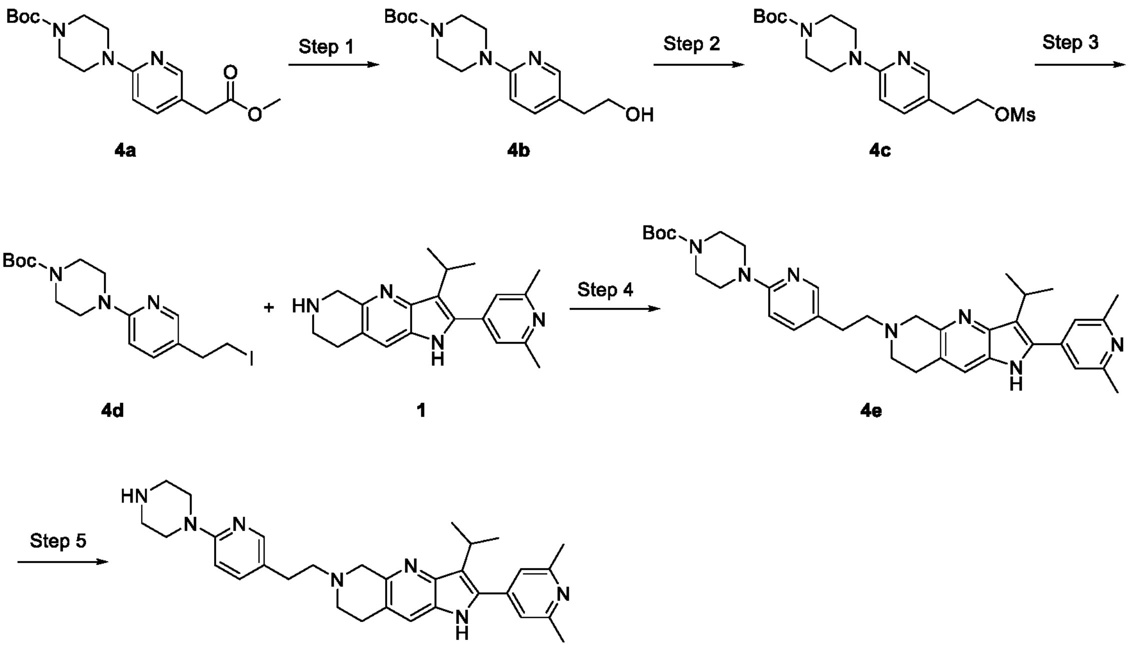


**4a** **4b** **4c**

**4d** + **1** **4e**

**4**

Step 1

tert-Butyl 4-(5-(2-(hydroxyethyl)pyridin-2-yl)piperazine-1-carboxylate **4b**

tert-Butyl 4-(5-(2-methoxy-2-oxoethyl)pyridin-2-yl)piperazine-1-carboxylate **4a** (120 mg, 0.36 mmol, "prepared using the method of synthesizing compound 4a on page 37 of the specification of WO2018138356 A1") was dissolved in anhydrous tetrahydrofuran (5.0 mL), the solution was cooled to 0 °C, and lithium borohydride (16 mg, 0.74 mmol, Sinopharm) was added. The reaction was stirred at room temperature for 16 h. Sodium sulfate decahydrate was added, and the mixture was stirred for 30 min, filtered, and washed. The filtrate was concentrated under reduced pressure, and the residue was purified by silica gel thin-layer chromatography with developing solvent system A to give the title product **4b** (110 mg, yield: 100%).

MS m/z (ESI): 307.8 [M+1].

Step 2

tert-Butyl 4-(5-(2-((methylsulfonyl)oxy)ethyl)pyridin-2-yl)piperazine-1-carboxylate **4c** Compound **4b** (140 mg, 0.46 mmol), methanesulfonyl chloride (70 mg, 0.61 mmol, Sinopharm), and triethylamine (90 mg, 0.89 mmol) were dissolved in dichloromethane (5.0 mL). The mixture was stirred at 0 °C for 2.0 h. Water (20 mL) was added to the reaction mixture, and extraction was performed with dichloromethane (20 mL × 3). The organic phases were combined and concentrated under reduced pressure to give the crude title product **4c** (175 mg, yield: 99.7%). The product was directly

used in the next step without purification.

MS m/z (ESI): 385.9 [M+1].

Step 3

tert-Butyl 4-(5-(2-iodoethyl)pyridin-2-yl)piperazine-1-carboxylate **4d**

The crude compound **4c** (175 mg, 0.45 mmol) and sodium iodide (200 mg, 1.33 mmol, adamas) were dissolved in acetone (10 mL). The mixture was stirred at 60 °C for 16 h. The reaction mixture was concentrated under reduced pressure. Water (20 mL) was added to the residue, and extraction was performed with ethyl acetate (20 mL × 3). The organic phases were combined and concentrated under reduced pressure, and the residue was purified by silica gel column chromatography with eluent system A to give the title product **4d** (140 mg, yield: 73.9%).

MS m/z (ESI): 417.8 [M+1].

Step 4

tert-Butyl

4-(5-(2-(2-(2,6-dimethylpyridin-4-yl)-3-isopropyl)-1,5,7,8-tetrahydro-6*H*-pyrrolo[3,2-*b*] [1,7]naphthyridin-6-yl)ethyl)pyridin-2-yl)piperazine-1-carboxylate **4e**

Compound **4d** (70 mg, 0.17 mmol), compound **1** (30 mg, 0.094 mmol), and cesium carbonate (100 mg, 0.31 mmol, Accela ChemBio (Shanghai)) were dissolved in dimethyl sulfoxide (3.0 mL), and the reaction was stirred at 100 °C for 4.0 h. Ethyl acetate (30 mL) was added to the reaction mixture, and the mixture was washed with water (10 mL) and saturated sodium chloride solution (10 mL × 2). The organic phase was concentrated under reduced pressure, and the residue was purified by silica gel thin-layer chromatography with developing solvent system A to give the title product **4e** (40 mg, yield: 70%).

MS m/z (ESI): 610.3 [M+1].

Step 5

2-(2,6-Dimethylpyridin-4-yl)-3-isopropyl-6-(2-(6-(piperazin-4-yl)pyridin-3-yl)ethyl)-5, 6,7,8-tetrahydro-1*H*-pyrrolo[3,2-*b*][1,7]naphthyridine **4**

Compound **4e** (70.0 mg, 0.11 mmol) was dissolved in dichloromethane (4 mL) and trifluoroacetic acid (1 mL). The mixture was stirred at room temperature for 1 h. The reaction mixture was concentrated under reduced pressure, and a 7 M ammonia/methanol solution (5 mL) was added to the residue. The mixture was concentrated under reduced pressure, and the residue was purified by high performance liquid chromatography (column: Sharpsil-T Prep C18, 5 μm 30 × 150 mm; mobile phases: A-water (10 mmol of ammonium bicarbonate):B-acetonitrile, 30-95% acetonitrile, 18 min of gradient elution, flow rate: 30 mL/min) to give the title product **4** (25.0 mg, yield: 42.7%).

MS m/z (ESI): 510.3 [M+1].

$^1$H NMR (500 MHz, $CD_3OD$) δ 8.07 (d, 1H), 7.58 (dd, 1H), 7.50 (s, 1H), 7.28 (s, 2H), 6.83 (d, 1H), 3.95 (s, 2H), 3.50-3.48 (m, 4H), 3.42 (m, 1H), 3.11 (t, 2H), 2.99-2.97 (m, 4H), 2.93 (t, 2H), 2.91-2.87 (m, 2H), 2.85-2.81 (m, 2H), 2.59 (s, 6H), 1.55 (d, 6H).

Example 5

2-(2,6-Dimethylpyridin-4-yl)-3-isopropyl-6-((5-(piperazin-1-yl)pyridin-2-yl)methyl)-5, 6,7,8-tetrahydro-1*H*-pyrrolo[3,2-*b*][1,7]naphthyridine **5**

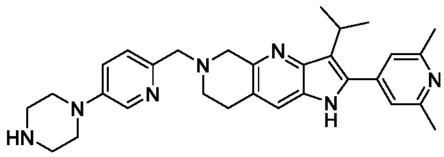

5

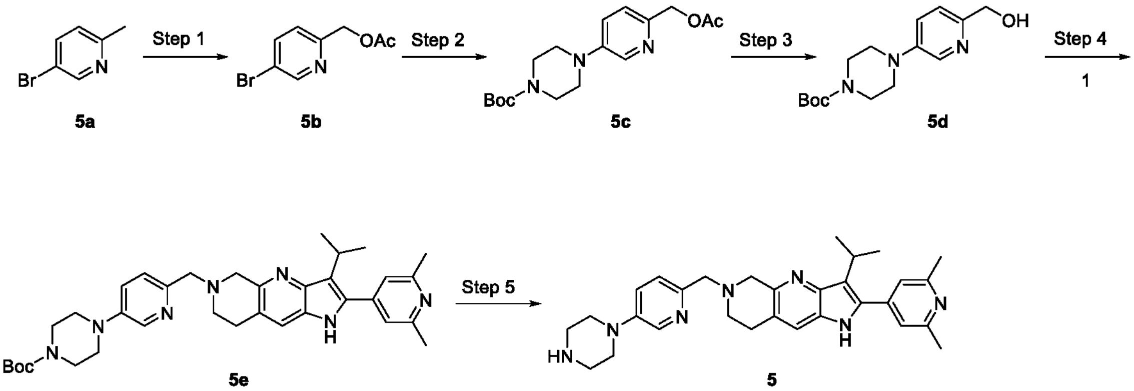


5a 5b 5c 5d

5e 5

Step 1

(5-Bromopyridin-2-yl)methyl acetate **5b**

5-Bromo-2-methylpyridine **5a** (4.0 g, 23.3 mmol, Accela ChemBio (Shanghai)) was dissolved in dichloromethane (50 mL), the solution was cooled to 0 °C, and 3-chloroperbenzoic acid (6.1 g, 35.3 mmol, Sinopharm) was added. The reaction was stirred at room temperature for 16 h. The reaction mixture was washed with saturated sodium bicarbonate solution (50 mL), and the organic phase was concentrated under reduced pressure. The residue was dissolved in acetic anhydride (16 mL), and the mixture was heated at reflux for 1.0 h. Ethanol was added to quench the reaction, and the reaction mixture was concentrated under reduced pressure. The residue was neutralized with saturated potassium bicarbonate solution (50 mL) and extracted with dichloromethane (50 mL × 2). The organic phases were combined and concentrated under reduced pressure, and the residue was purified by silica gel column chromatography with eluent system A to give the title product **5b** (2.1 g, yield: 39.2%).

MS m/z (ESI): 229.6 [M+1].

Step 2

tert-Butyl 4-(6-(acetoxymethyl)pyridin-3-yl)piperazine-1-carboxylate **5c**

Compound **5b** (1.0 g, 4.35 mmol), tert-butyl piperazine-1-carboxylate (1.62 g, 8.7 mmol, Accela ChemBio (Shanghai)), cesium carbonate (4.3 g, 13.2 mmol, Accela ChemBio (Shanghai)), tris(dibenzylideneacetone)dipalladium(0) (399 mg, 0.44 mmol, Bide Pharmatech Ltd.), and 2,2'-bis(diphenylphosphino)-1,1'-binaphthyl (542 mg, 0.87 mmol, Bide Pharmatech Ltd.) were dissolved in toluene (30 mL). The reaction was stirred at 90 °C for 12 h in a nitrogen atmosphere. The reaction mixture was concentrated under reduced pressure, and the residue was purified by silica gel column chromatography with eluent system A to give the title product **5c** (1.2 g, yield: 82.3%).

MS m/z (ESI): 335.9 [M+1].

Step 3

tert-Butyl 4-(6-(hydroxymethyl)pyridin-3-yl)piperazine-1-carboxylate **5d**

Compound **5c** (390 mg, 1.16 mmol) and lithium hydroxide monohydrate (150 mg, 3.57 mmol, Sinopharm) were dissolved in tetrahydrofuran (9.0 mL) and water (3.0 mL). The mixture was stirred at room temperature for 16 h. Water (20 mL) was added to the reaction mixture, and extraction was performed with ethyl acetate (20 mL × 3). The organic phases were combined and concentrated under reduced pressure, and the residue was purified by silica gel column chromatography with eluent system A to give the title product **5d** (240 mg, yield: 70.4%).

MS m/z (ESI): 294.0 [M+1].

Step 4

tert-Butyl

4-(6-((2-(2,6-dimethylpyridin-4-yl)-3-isopropyl)-1,5,7,8-tetrahydro-6*H*-pyrrolo[3,2-*b*][1 ,7]naphthyridin-6-yl)methyl)pyridin-3-yl)piperazine-1-carboxylate **5e**

Compound **5d** (150 mg, 0.51 mmol), methanesulfonyl chloride (60 mg, 0.52 mmol, Sinopharm), and triethylamine (55 mg, 0.54 mmol) were dissolved in dichloromethane (3.0 mL), and the reaction was stirred at 0 °C for 2.0 h. The reaction solution was added dropwise to a solution of compound **1** (80 mg, 0.25 mmol) and potassium carbonate (100 mg, 0.72 mmol) in acetonitrile (5.0 mL), and the reaction was stirred at 70 °C for 2.0 h. Water (20 mL) was added to the reaction mixture, and extraction was performed with ethyl acetate (20 mL × 3). The organic phases were combined and concentrated under reduced pressure, and the residue was purified by silica gel thin-layer chromatography with developing solvent system A to give the title product **5e** (100 mg, yield: 67.2%).

MS m/z (ESI): 596.3 [M+1].

Step 5

2-(2,6-Dimethylpyridin-4-yl)-3-isopropyl-6-((5-(piperazin-1-yl)pyridin-2-yl)methyl)-5, 6,7,8-tetrahydro-1*H*-pyrrolo[3,2-*b*][1,7]naphthyridine **5**

Compound **5e** (100.0 mg, 0.17 mmol) was dissolved in dichloromethane (4 mL) and trifluoroacetic acid (1 mL). The mixture was stirred at room temperature for 1 h. The reaction mixture was concentrated under reduced pressure, and a 7 M ammonia/methanol solution (5 mL) was added to the residue. The mixture was concentrated under reduced pressure, and the residue was purified by high performance liquid chromatography (column: Sharpsil-T Prep C18, 5 μm 30 × 150 mm; mobile phases: A-water (10 mmol of ammonium bicarbonate):B-acetonitrile, 26-95% acetonitrile, 18 min of gradient elution, flow rate: 30 mL/min) to give the title product **5** (15.0 mg, yield: 18.0%).

MS m/z (ESI): 496.3 [M+1].

$^1$H NMR (500 MHz, $CD_3OD$) δ 8.24 (d, 1H), 7.51-7.47 (m, 3H), 7.26 (s, 2H), 3.86-3.85 (m, 4H), 3.39 (m, 1H), 3.27-3.25 (m, 4H), 3.09-3.04 (m, 6H), 2.90-2.88 (m, 2H), 2.59 (s, 6H), 1.51 (d, 6H).

Example 6

2-(2,6-Dimethylpyridin-4-yl)-3-(tetrahydro-2*H*-pyran-4-yl)-5,6,7,8-tetrahydro-1*H*-pyrro lo[3,2-*b*][1,7]naphthyridine **6**

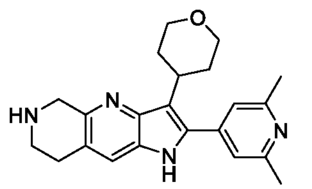

**6**

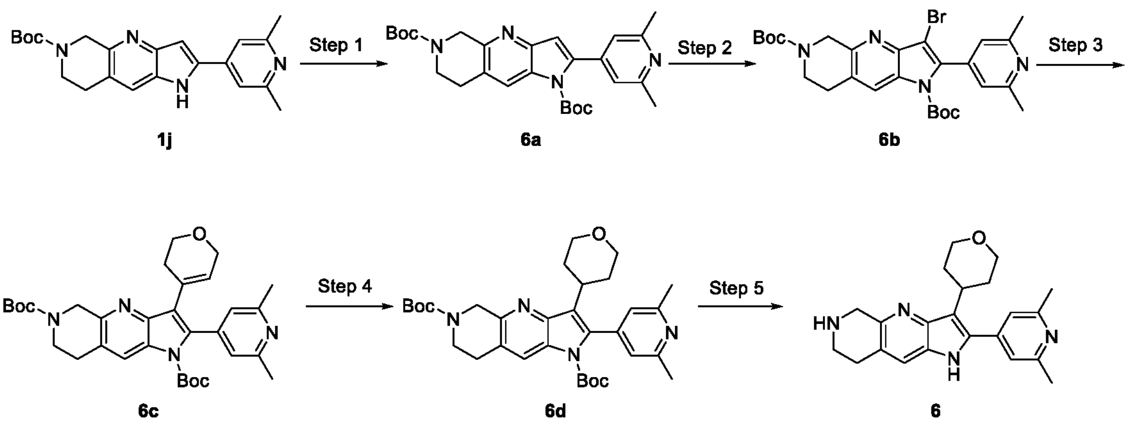


**1j** **6a** **6b**

**6c** **6d** **6**

Step 1

Di-tert-butyl

2-(2,6-dimethylpyridin-4-yl)-7,8-dihydro-1*H*-pyrrolo[3,2-*b*][1,7]naphthyridine-1,6(5*H*)-dicarboxylate **6a**

Compound **1j** (1.59 g, 4.20 mmol), di-tert-butyl dicarbonate (2.0 g, 9.16 mmol, Accela ChemBio (Shanghai)), 4-dimethylaminopyridine (100 mg, 0.81 mmol, adamas), and triethylamine (3.0 g, 29.7 mmol) were dissolved in tetrahydrofuran (30 mL), and the reaction was stirred at room temperature for 16 h. Water (60 mL) was added to the reaction mixture, and extraction was performed with ethyl acetate (50 mL × 3). The organic phases were combined and concentrated under reduced pressure, and the residue was purified by silica gel column chromatography with eluent system A to give the title product **6a** (1.35 g, yield: 67.1%).
MS m/z (ESI): 479.0 [M+1].

Step 2

Di-tert-butyl

3-bromo-2-(2,6-dimethylpyridin-4-yl)-7,8-dihydro-1*H*-pyrrolo[3,2-*b*][1,7]naphthyridine -1,6(5*H*)-dicarboxylate **6b**

Compound **6a** (1.35 g, 2.82 mmol) was dissolved in *N*,*N*-dimethylformamide (20 mL), the solution was cooled to 0 °C, and *N*-bromosuccinimide (600 mg, 3.37 mmol, Accela ChemBio (Shanghai)) was added. The mixture was stirred at room temperature for 20 h. The reaction mixture was concentrated under reduced pressure, and the residue was purified by silica gel column chromatography with eluent system A to give the title product **6b** (1.05 g, yield: 66.8%).
MS m/z (ESI): 556.9 [M+1].

Step 3

Di-tert-butyl

3-(3,6-dihydro-2*H*-pyran-4-yl)-2-(2,6-dimethylpyridin-4-yl)-7,8-dihydro-1*H*-pyrrolo[3, 2-*b*][1,7]naphthyridine-1,6(5*H*)-dicarboxylate **6c**

Compound **6b** (100 mg, 0.18 mmol), 3,6-dihydro-2*H*-pyran-4-boronic acid pinacol ester (50 mg, 0.24 mmol, Accela ChemBio (Shanghai)), [1,1'-bis(diphenylphosphino)ferrocene]palladium(II) dichloride (20 mg, 0.03 mmol, Bide Pharmatech Ltd.), and anhydrous tripotassium phosphate (120 mg, 0.57 mmol) were dissolved in dioxane (3.0 mL) and water (1.0 mL), and the reaction was stirred at 80 °C for 16 h in a nitrogen atmosphere. The reaction mixture was cooled to room temperature. Water (15 mL) was added, and extraction was performed with ethyl acetate (20 mL × 3). The organic phases were combined and concentrated under reduced pressure, and the residue was purified by silica gel column chromatography with eluent system A to give the title product **6c** (90 mg, yield: 89.5%).
MS m/z (ESI): 561.0 [M+1].

Step 4

Di-tert-butyl

2-(2,6-dimethylpyridin-4-yl)-3-(tetrahydro-2*H*-pyran-4-yl)-7,8-dihydro-1*H*-pyrrolo[3,2-*b*][1,7]naphthyridine-1,6(5*H*)-dicarboxylate **6d**

Compound **6c** (90 mg, 0.16 mmol) was dissolved in methanol (10 mL), and palladium on carbon (100 mg, 0.094 mmol, 10% purity, adamas) was added. The reaction was stirred at room temperature for 16 h in a hydrogen atmosphere. The reaction mixture was filtered, and the filtrate was concentrated under reduced pressure to give the crude title product **6d** (80 mg, yield: 88.6%). The product was directly used in the next step without purification.
MS m/z (ESI): 563.0 [M+1].

Step 5

2-(2,6-Dimethylpyridin-4-yl)-3-(tetrahydro-2*H*-pyran-4-yl)-5,6,7,8-tetrahydro-1*H*-pyrro lo[3,2-*b*][1,7]naphthyridine **6**

The crude compound **6d** (80.0 mg, 0.14 mmol) was dissolved in dichloromethane (5 mL) and trifluoroacetic acid (2 mL), and the mixture was stirred at room temperature for 1 h. The reaction mixture was concentrated under reduced pressure, and a 7 M ammonia/methanol solution (5 mL) was added to the residue. The mixture was concentrated under reduced pressure, and the residue was purified by high performance liquid chromatography (column: Sharpsil-T Prep C18, 5 μm 30 × 150 mm; mobile phases: A-water (10 mmol of ammonium bicarbonate):B-acetonitrile, 28-95% acetonitrile, 18 min of gradient elution, flow rate: 30 mL/min) to give the title product **6** (14 mg, yield: 27.2%).
MS m/z (ESI): 363.3 [M+1].
$^1H$ NMR (500 MHz, $CD_3OD$) δ 7.49 (s, 1H), 7.28 (s, 2H), 4.20 (s, 2H), 4.08-4.05 (m, 2H), 3.59-3.54 (m, 2H), 3.26 (m, 1H), 3.21-3.19 (m, 2H), 3.06-3.04 (m, 2H), 2.82-2.73 (m, 2H), 2.59 (s, 6H), 1.66-1.62 (m, 2H).

Example 7

4-((2-(2,6-Dimethylpyridin-4-yl)-3-isopropyl-1,5,7,8-tetrahydro-6*H*-pyrrolo[3,2-*b*][1,7] naphthyridin-6-yl)methyl)bicyclo[2.2.2]octyl-1-amine **7**

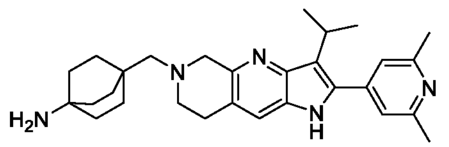

**7**

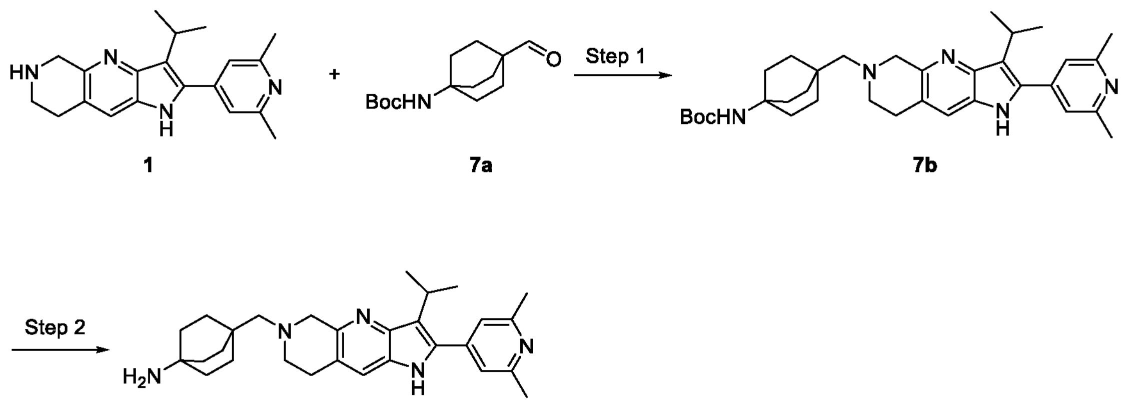


**1** **7a** **7b**

**7**

Step 1

tert-Butyl

(4-((2-(2,6-dimethylpyridin-4-yl)-3-isopropyl-1,5,7,8-tetrahydro-6*H*-pyrrolo[3,2-*b*][1,7] naphthyridin-6-yl)methyl)bicyclo[2.2.2]oct-1-yl)carbamate **7b**

Compound **1** (1.05 g, 3.28 mmol) and tert-butyl (4-formylbicyclo[2.2.2]oct-1-yl)carbamate **7a** (1.60 g, 6.32 mmol, "prepared using the method of synthesizing intermediate A on page 67 of the specification of WO2013003383A1") were dissolved in 1,2-dichloroethane (20 mL) and *N*,*N*-dimethylformamide (4.0 mL), and 1 drop of acetic acid was added. The reaction was stirred at room temperature for 2.0 h. Sodium triacetoxyborohydride (1.50 g, 7.08 mmol) was added, and the reaction was stirred at 60 °C for 16 h. The reaction mixture was concentrated under reduced pressure. Water (50 mL) was added to the residue, and extraction was performed with ethyl acetate (50 mL × 4). The organic phases were combined and concentrated under reduced pressure, and the residue was purified by silica gel column chromatography with eluent system B to give the title product **7b** (1.05 g, yield: 57.4%).
MS m/z (ESI): 558.3 [M+1].

Step 2

4-((2-(2,6-Dimethylpyridin-4-yl)-3-isopropyl-1,5,7,8-tetrahydro-6*H*-pyrrolo[3,2-*b*][1,7] naphthyridin-6-yl)methyl)bicyclo[2.2.2]octyl-1-amine **7**

Compound **7b** (1.05 g, 0.11 mmol) was dissolved in dichloromethane (20 mL) and trifluoroacetic acid (4.0 mL). The mixture was stirred at room temperature for 1 h. The reaction mixture was concentrated under reduced pressure, and a 7 M ammonia/methanol solution (20 mL) was added to the residue. The mixture was concentrated under reduced pressure, and the residue was purified by high performance liquid chromatography (column: Waters XBridge C18, 5 μm, 30 mm × 150 mm; mobile phases: A-aqueous phase (0.5% ammonium hydroxide):B-acetonitrile (0.5% ammonium hydroxide), 80-95% acetonitrile, 18 min of gradient elution, flow rate: 30 mL/min) to give the title product **7** (330 mg, yield: 38.3%).

MS m/z (ESI): 458.3 [M+1].

$^1$H NMR (500 MHz, $CD_3OD$) δ 7.45 (s, 1H), 7.27 (s, 2H), 3.86 (s, 2H), 3.42 (m, 1H), 3.02-3.00 (m, 2H), 2.83-2.80 (m, 2H), 2.59 (s, 6H), 2.30 (s, 2H), 1.66-1.62 (m, 6H), 1.60-1.57 (m, 6H), 1.53 (d, 6H).

Example 8

2-(2-(2,6-Dimethylpyridin-4-yl)-3-isopropyl-1,5,7,8-tetrahydro-6*H*-pyrrolo[3,2-*b*][1,7]n aphthyridin-6-yl)acetamide **8**

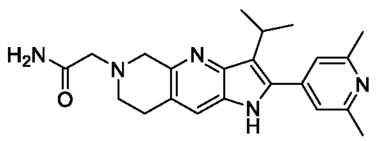

**8**

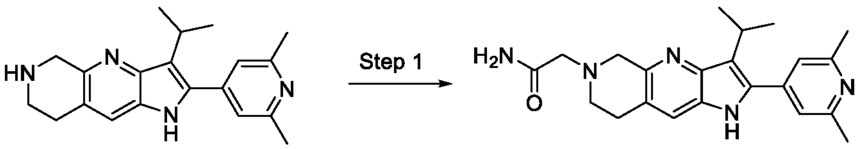


**1** **8**

Compound **1** (300 mg, 0.94 mmol), 2-bromoacetamide (200 mg, 1.45 mmol, Accela ChemBio (Shanghai)), and 1,8-diazabicyclo[5.4.0]undec-7-ene (500 mg, 1.99 mmol, adamas) were dissolved in dichloromethane (10 mL), and the reaction was stirred at room temperature for 6.0 h. The reaction mixture was concentrated under reduced pressure, and the residue was purified by high performance liquid chromatography (column: Boston Phlex Prep C18, 5 μm 30 × 150 mm; mobile phases: A-aqueous phase (10 mmol of ammonium bicarbonate):B-acetonitrile, 20-80% acetonitrile, 20 min of gradient elution, flow rate: 30 mL/min) to give the title product **8** (120 mg, yield: 34%).

MS m/z (ESI): 378.0 [M+1].

$^1$H NMR (500 MHz, DMSO-$d_6$) δ 11.14 (s, 1H), 7.43 (s, 1H), 7.30 (d, 1H), 7.20 (s, 2H), 7.17 (d, 1H), 3.78 (s, 2H), 3.34 (s, 6H), 3.31 (m, 1H), 3.11 (s, 2H), 3.01-2.99 (m, 2H), 2.78-2.76 (m, 2H), 1.50 (d, 6H).

Example 9

2-(7,8-Dimethyl-[1,2,4]triazolo[1,5-*a*]pyridin-6-yl)-3-isopropyl-5,6,7,8-tetrahydro-1*H*-p yrrolo[3,2-*b*][1,7]naphthyridine **9**

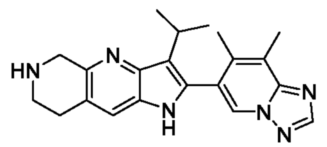

9

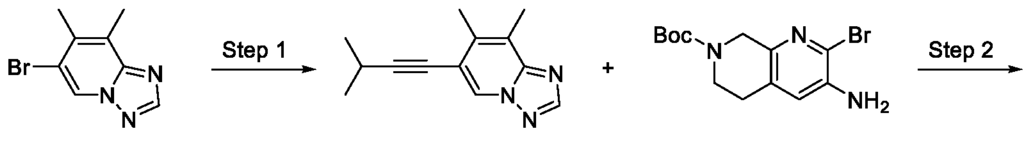


9a 9b 1h

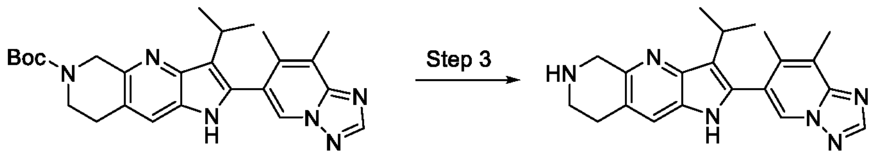


9c 9

Step 1

7,8-Dimethyl-6-(3-methylbut-1-yn-1-yl)-[1,2,4]triazolo[1,5-*a*]pyridine **9b**

6-Bromo-7,8-dimethyl-[1,2,4]triazolo[1,5-*a*]pyridine **9a** (760 mg, 3.36 mmol, "prepared using the method of synthesizing intermediate F-4 on page 140 of the specification of the patent application WO2018005586A1") and 3-methyl-1-yne (500 mg, 7.34 mmol, Acros), cuprous iodide (130 mg, 0.682 mmol, Alfa), bis(triphenylphosphine)palladium(II) dichloride (240 mg, 0.341 mmol, adamas), and triethylamine (1.0 g, 10.0 mmol) were dissolved in N,N-dimethylformamide (15 mL), and the mixture was stirred at 60 °C for 30 h in a nitrogen atmosphere. Water (50 mL) was added to quench the reaction, and extraction was performed with ethyl acetate (50 mL × 3). The organic phases were combined and concentrated under reduced pressure, and the residue was purified by silica gel column chromatography with eluent system A to give the title product **9b** (650 mg, yield: 90.6%).

MS m/z (ESI): 214.0 [M+1].

Step 2

tert-Butyl

2-(7,8-dimethyl-[1,2,4]triazolo[1,5-*a*]pyridin-6-yl)-3-isopropyl-1,5,7,8-tetrahydro-6*H*-p yrrolo[3,2-*b*][1,7]naphthyridine-6-carboxylate **9c**

Compound **1h** (1.0 g, 3.05 mmol) and compound **9b** (650 mg, 3.05 mmol) were dissolved in dimethylacetamide (20 mL), and anhydrous lithium chloride (130 mg, 3.07 mmol), potassium carbonate (1.27 g, 9.20 mmol), and tetrakis(triphenylphosphine)palladium(0) (360 mg, 0.31 mmol, adamas) were added. The mixture was reacted at 115 °C for 20 h. The reaction mixture was cooled to room temperature. Water (60 mL) was added, and extraction was performed with ethyl acetate (50 mL × 3). The organic phases were combined and concentrated under reduced pressure, and the residue was purified by silica gel column chromatography with eluent system B to give the title product **9c** (700 mg, yield: 49.9%).

MS m/z (ESI): 461.0 [M+1].

Step 3

2-(7,8-Dimethyl-[1,2,4]triazolo[1,5-*a*]pyridin-6-yl)-3-isopropyl-5,6,7,8-tetrahydro-1*H*-p yrrolo[3,2-*b*][1,7]naphthyridine **9**

Compound **9c** (700 mg, 1.52 mmol) was dissolved in dichloromethane (20 mL), and trifluoroacetic acid (5.0 mL) was added. The mixture was stirred at room temperature for 1 h and concentrated, and the residue was purified by

silica gel column chromatography with eluent system B to give a crude product. The crude product was purified by chiral chromatography (column: CHIRALPAK IF 20 × 250 mm, mobile phases: A-hexane, B-ethanol (containing 10 mM $NH_3$), flow rate: 20 mL/min, detection wavelength 214 & 254 nm) to give the title product **9** (120 mg, 21.9%).

MS m/z (ESI): 361.0 [M+1].

$^1$H NMR (500 MHz, DMSO-$d_6$) δ 11.30 (s, 1H), 8.84 (s, 1H), 8.50 (s, 1H), 7.56 (s, 1H), 4.32 (s, 2H), 3.39-3.37 (m, 2H), 3.31 (s, 1H), 3.14-3.12 (m, 2H), 2.91 (m, 1H), 2.60 (s, 3H), 2.15 (s, 3H), 1.37 (d, 6H).

Example 10

2-(2-(7,8-Dimethyl-[1,2,4]triazolo[1,5-*a*]pyridin-6-yl)-3-isopropyl-1,5,7,8-tetrahydro-6 *H*-pyrrolo[3,2-*b*][1,7]naphthyridin-6-yl)acetamide **10**

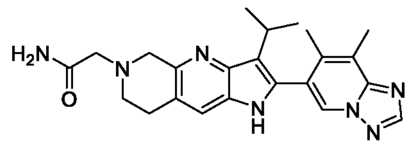

**10**

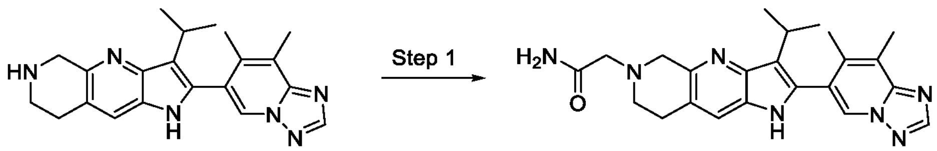


**9** **10**

Compound **9** (85 mg, 0.24 mmol), 2-bromoacetamide (60 mg, 0.43 mmol, Accela ChemBio (Shanghai)), and 1,8-diazabicyclo[5.4.0]undec-7-ene (120 mg, 0.48 mmol, adamas) were dissolved in dichloromethane (5.0 mL), and the reaction was stirred at room temperature for 6.0 h. The reaction mixture was concentrated under reduced pressure, and the residue was purified by chiral chromatography (column: CHIRALPAK IF 20 × 250 mm, mobile phases: A-hexane, B-ethanol (containing 10 mM $NH_3$), flow rate: 20 mL/min, detection wavelength 214 & 254 nm) to give the title product **10** (20 mg, 20.3%).

MS m/z (ESI): 418.0 [M+1].

$^1$H NMR (500 MHz, CD3OD) δ 8.67 (s, 1H), 8.43 (s, 1H), 7.51 (s, 1H), 3.98 (s, 2H), 3.32 (s, 2H), 3.22 (m, 1H), 3.15-3.12 (m, 2H), 2.96-2.93 (m, 2H), 2.67 (s, 3H), 2.26 (s, 3H), 1.34 (d, 6H).

Biological Evaluation

The present disclosure is further described and explained below with reference to test examples, but these examples are not intended to limit the scope of the present disclosure.

Test Example 1. Inhibitory Effect of Compounds Disclosed Herein on Human TLR7 Activation Pathway

I. Materials and instruments

1. HEK-Blue™ hTLR7 cell (Invivogen)
2. Resiquimod (R848, Invivogen)
3. Alkaline phosphatase detection medium (Quanti-Blue Detection, Invivogen)
4. Blasticidin (Invivogen)
5. Zeocin (Invivogen)
6. Normocin (Invivogen)
7. DMEM/HIGH Glucose (GE Healthcare)
8. Fetal bovine serum (FBS, Gibco)
9. Phosphate-buffered saline (Shanghai BasalMedia Technologies Co., Ltd.)
10. Sterile purified water (made in-house by Shanghai Hengrui)
11. 15 mL centrifuge tube (Corning)

12. 96-well formulating plate (Corning)
13. 96-well flat-bottom cell culture plate (Corning)
14. Constant-temperature cell incubator (Thermo scientific)
15. Constant-temperature incubator (Shanghai Yiheng Scientific Instruments Co., Ltd.)
16. PHERAstar FS microplate reader (BMG Labtech)

II. Procedures

HEK-Blue™ hTLR7 cells were purchased from Invivogen. These cells are HEK293 cells transfected with the human Toll-like receptor 7 (TLR7) gene and the secreted alkaline phosphatase reporter gene (SEAP). The alkaline phosphatase reporter gene (SEAP) is under the regulation of the IFN-β minimal promoter comprising 5 NF-kB and AP-1 binding sites. Upon activation of TLR7 with an agonist, SEAP secretion is induced via downstream NF-kB and AP-1. After addition of an antagonist compound, the pathway described above is inhibited, and SEAP secretion is reduced. The activity of the compounds for the TLR7 pathway was evaluated by measuring OD620 through the SEAP substrate.

20 mM test compounds dissolved in 100% DMSO were serially diluted with 100% DMSO to 2000, 400, 80, 16, 3.2, 0.64, 0.128, 0.0256 μM, with blank wells containing 100% DMSO. The compounds and 100% DMSO were then 20-fold diluted in DMEM/HIGH glucose media containing 10% inactivated FBS (complete media, the same applies below). Resiquimod was diluted to 10 μM with sterile water. The 10 μM resiquimod dilution in sterile water was added to a 96-well cell culture plate at 20 μL/well. The above compounds diluted in complete media and 100% DMSO were then added at 20 μL/well to wells containing resiquimod. 20 μL of sterile water and 20 μL of 100% DMSO diluted in the complete medium were added to negative control wells.

HEK-Blue™ hTLR7 cells were cultured in a DMEM/HIGH glucose medium containing 10% inactivated FBS, 100 μg/mL Normocin, 10 μg/mL blasticidin, and 100 μg/mL Zeocin. Cells that were in a good state of growth and had grown to 70%-80% were collected. The growth medium was discarded, 5-10 mL of PBS pre-heated at 37 °C was added to wash the cells once, and 2-5 mL of pre-heated PBS was added. The cells were cultured at 37 °C for 1-2 min, dispersed by pipetting, transferred to a 15 mL centrifuge tube, and counted, and the cell density was adjusted to $4.8 \times 10^5$/mL with the complete medium. After density adjustment, 160 μL of the resulting cell suspension was added to the above 96-well cell culture plate to a final number of cells per well of 76,500/well. The final concentration of resiquimod was 1 μM, and the final concentrations of the test compounds were 10,000, 2000, 400, 80, 16, 3.2, 0.64, and 0.128 nM. The cells were cultured in a 37 °C, 5% $CO_2$ incubator for 20 h. Then 20 μL of the supernatant was added to 180 μL of the prepared alkaline phosphatase detection medium and incubated in the dark in a constant-temperature incubator at 37 °C for 120 min. Then the OD620 absorbance readings were taken on a microplate reader. The inhibition rate was calculated using the following formula:

$$\text{Inhibition rate} = \{1- (OD_{\text{test compound}} - OD_{\text{negative control well}}) / (OD_{\text{blank well}} - OD_{\text{negative control well}})\} \times 100\%$$

Inhibition curves were plotted using Graphpad Prism software according to the concentrations of the compounds and the corresponding inhibition rates, and the concentrations of the compounds at which the inhibition rate reached 50%, i.e., $IC_{50}$ values, were calculated and are shown in Table 1.

Table 1. The measured $IC_{50}$ values of the compounds of the present disclosure for the human TLR7 pathway

| Example No. | $IC_{50}$ (nM) |
|---|---|
| 1 | 7.7 |
| 2 | 29.9 |
| 3 | 14.9 |
| 4 | 6.8 |
| 5 | 5.7 |
| 7 | 7.1 |
| 8 | 18.2 |
| 9 | 4.9 |

(continued)

| Example No. | $IC_{50}$ (nM) |
|---|---|
| 10 | 21.5 |
| Conclusion: the compounds of the present disclosure have an inhibitory effect on the TLR7 pathway. | |

Test Example 2. Inhibitory Effect of Compounds Disclosed Herein on Human TLR8 Pathway

I. Materials and instruments

1. HEK-Blue™ hTLR8 cell (Invivogen)
2. Resiquimod (R848, Invivogen)
3. Alkaline phosphatase detection medium (Quanti-Blue Detection, Invivogen)
4. Blasticidin (Invivogen)
5. Zeocin (Invivogen)
6. Normocin (Invivogen)
7. DMEM/HIGH Glucose (GE Healthcare)
8. Fetal bovine serum (FBS, Gibco)
9. Phosphate-buffered saline (Shanghai BasalMedia Technologies Co., Ltd.)
10. Sterile purified water (made in-house by Shanghai Hengrui)
11. 15 mL centrifuge tube (Corning)
12. 96-well formulating plate (Corning)
13. 96-well flat-bottom cell culture plate (Corning)
14. Constant-temperature cell incubator (Thermo scientific)
15. Constant-temperature incubator (Shanghai Yiheng Scientific Instruments Co., Ltd.)
16. PHERAstar FS microplate reader (BMG Labtech)

II. Procedures

HEK-Blue™ hTLR8 cells were purchased from Invivogen. These cells are HEK293 cells transfected with the human Toll-like receptor 8 (TLR8) gene and the secreted alkaline phosphatase reporter gene (SEAP). The alkaline phosphatase reporter gene (SEAP) is under the regulation of the IFN-β minimal promoter comprising 5 NF-kB and AP-1 binding sites. Upon activation of TLR8 with an agonist, SEAP secretion is induced via downstream NF-kB and AP-1. After addition of an antagonist compound, the pathway described above is inhibited, and SEAP secretion is reduced. The activity of the compounds for the TLR8 pathway was evaluated by measuring OD620 through the SEAP substrate.

20 mM test compounds dissolved in 100% DMSO were serially diluted with 100% DMSO to 2000, 400, 80, 16, 3.2, 0.64, 0.128, 0.0256 μM, with blank wells containing 100% DMSO. The compounds and 100% DMSO were then 20-fold diluted in DMEM/HIGH glucose media containing 10% inactivated FBS (complete media, the same applies below). Resiquimod was diluted to 60 μM with sterile water. The 60 μM resiquimod dilution in sterile water was added to a 96-well cell culture plate at 20 μL/well. The above compounds diluted in complete media and 100% DMSO were then added at 20 μL/well to wells containing resiquimod. 20 μL of sterile water and 20 μL of 100% DMSO diluted in the complete medium were added to negative control wells. HEK-Blue™ hTLR8 cells were cultured in a DMEM/HIGH glucose medium containing 10% inactivated FBS, 100 μg/mL Normocin, 10 μg/mL blasticidin, and 100 μg/mL Zeocin. Cells that were in a good state of growth and had grown to 70%-80% were collected. The growth medium was discarded, 5-10 mL of PBS pre-heated at 37 °C was added to wash the cells once, and 2-5 mL of pre-heated PBS was added. The cells were cultured at 37 °C for 1-2 min, dispersed by pipetting, transferred to a 15 mL centrifuge tube, and counted, and the cell density was adjusted to $4.8 \times 10^5$/mL with the complete medium. After density adjustment, 160 μL of the resulting cell suspension was added to the above 96-well cell culture plate to a final number of cells per well of 76,500/well. The final concentration of resiquimod was 6 μM, and the final concentrations of the test compounds were 10,000, 2000, 400, 80, 16, 3.2, 0.64, and 0.128 nM. The cells were cultured in a 37 °C, 5% $CO_2$ incubator for 20 h. Then 20 μL of the supernatant was added to 180 μL of the prepared alkaline phosphatase detection medium and incubated in the dark in a constant-temperature incubator at 37 °C for 120 min. The OD620 absorbance readings were taken on a microplate reader. The inhibition rate was calculated using the following formula:

$$\text{Inhibition rate} = \{1 - (OD_{\text{test compound}} - OD_{\text{negative control well}}) / (OD_{\text{blank well}} - OD_{\text{negative control well}})\} \times 100\%$$

Inhibition curves were plotted using Graphpad Prism software according to the concentrations of the compounds and the corresponding inhibition rates, and the concentrations of the compounds at which the inhibition rate reached 50%, i.e., $IC_{50}$ values, were calculated and are shown in Table 2.

Table 2. The measured $IC_{50}$ values of the compounds of the present disclosure for the human TLR8 pathway

| Example No. | $IC_{50}$ (nM) |
|---|---|
| 1 | 0.8 |
| 2 | 30.4 |
| 4 | 1.8 |
| 5 | 2.5 |
| 6 | 10.5 |
| 7 | 2.2 |
| 8 | 18.8 |
| 9 | 0.6 |
| 10 | 8.2 |
| Conclusion: the compounds of the present disclosure have an inhibitory effect on the TLR8 pathway. | |

Test Example 3. Inhibitory Effect of Compounds Disclosed Herein on Human TLR9 Activation Pathway

I. Materials and instruments

1. HEK-Blue™ hTLR9 cell (Invivogen)
2. CpG ODN2006 (Invivogen)
3. Alkaline phosphatase detection medium (Quanti-Blue Detection, Invivogen)
4. Blasticidin (Invivogen)
5. Zeocin (Invivogen)
6. Normocin (Invivogen)
7. DMEM/HIGH Glucose (GE Healthcare)
8. Fetal bovine serum (FBS, Gibco)
9. Phosphate-buffered saline (Shanghai BasalMedia Technologies Co., Ltd.)
10. Sterile purified water (made in-house by Shanghai Hengrui)
11. 15 mL centrifuge tube (Corning)
12. 96-well formulating plate (Corning)
13. 96-well flat-bottom cell culture plate (Corning)
14. Constant-temperature cell incubator (Thermo scientific)
15. Constant-temperature incubator (Shanghai Yiheng Scientific Instruments Co., Ltd.)
16. PHERAstar FS microplate reader (BMG Labtech)

II. Procedures

HEK-Blue™ hTLR9 cells were purchased from Invivogen. These cells are HEK293 cells transfected with the human Toll-like receptor 9 (TLR9) gene and the secreted alkaline phosphatase reporter gene (SEAP). The alkaline phosphatase reporter gene (SEAP) is under the regulation of the IFN-β minimal promoter comprising 5 NF-kB and AP-1 binding sites. Upon activation of TLR9 with an agonist, SEAP secretion is induced via downstream NF-kB and AP-1. After addition of an antagonist compound, the pathway described above is inhibited, and SEAP secretion is reduced. The activity of the compounds for the TLR9 pathway was evaluated by measuring OD620 through the SEAP substrate.

20 mM test compounds dissolved in 100% DMSO were serially diluted with 100% DMSO to 2000, 400, 80, 16, 3.2, 0.64, 0.128, 0.0256 μM, with blank wells containing 100% DMSO. The compounds and 100% DMSO were then 20-fold diluted in DMEM/HIGH glucose media containing 10% inactivated FBS (complete media, the same applies below). CpG ODN2006 was diluted to 10 μM with sterile water. The 10 μM CpG ODN2006 dilution in sterile water was added to a 96-well cell culture plate at 20 μL/well. The above compounds diluted in complete media and 100% DMSO were then added at 20 μL/well to wells containing CpG ODN2006. 20 μL of sterile water and 20 μL of 100% DMSO diluted in the complete medium were added to negative control wells.

HEK-Blue™ hTLR9 cells were cultured in a DMEM/HIGH glucose medium containing 10% FBS, 100 μg/mL Normocin, 10 μg/mL blasticidin, and 100 μg/mL Zeocin. Cells that were in a good state of growth and had grown to 70%-80% were collected. The growth medium was discarded, 5-10 mL of PBS pre-heated at 37 °C was added to wash the cells once, and 2-5 mL of pre-heated PBS was added. The cells were cultured at 37 °C for 1-2 min, dispersed by pipetting, transferred to a 15 mL centrifuge tube, and counted, and the cell density was adjusted to $4.8 \times 10^5$/mL with the DMEM/HIGH glucose medium containing 10% inactivated FBS. After density adjustment, 160 μL of the resulting cell suspension was added to the above 96-well cell culture plate to a final number of cells per well of 76,500/well. The final concentration of CpG ODN2006 was 1 μM, and the final concentrations of the test compounds were 10,000, 2000, 400, 80, 16, 3.2, 0.64, and 0.128 nM. The cells were cultured in a 37 °C, 5% $CO_2$ incubator for 20 h. Then 20 μL of the supernatant was added to 180 μL of the prepared alkaline phosphatase detection medium and incubated in the dark in a constant-temperature incubator at 37 °C for 15 min. Then the OD620 absorbance readings were taken on a microplate reader. The inhibition rate was calculated using the following formula:

$$\text{Inhibition rate} = \{1- (OD_{\text{test compound}} - OD_{\text{negative control well}}) / (OD_{\text{blank well}} - OD_{\text{negative control well}})\} \times 100\%$$

Inhibition curves were plotted using Graphpad Prism software according to the concentrations of the compounds and the corresponding inhibition rates, and the concentrations of the compounds at which the inhibition rate reached 50%, i.e., $IC_{50}$ values, were calculated and are shown in Table 3.

Table 3. The measured $IC_{50}$ values of the compounds of the present disclosure for the human TLR9 pathway

| Example No. | $IC_{50}$ (nM) |
|---|---|
| 4 | 74 |
| 5 | 87 |
| 7 | 90 |
| Conclusion: the compounds of the present disclosure have an inhibitory effect on the TLR9 pathway. | |

## Claims

1. A compound of general formula (I) or a pharmaceutically acceptable salt thereof:

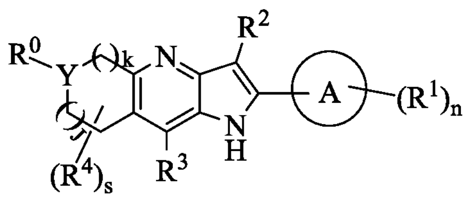

(I)

wherein:

Y is $CR^{4a}$ or a nitrogen atom;
ring A is selected from the group consisting of cycloalkyl, heterocyclyl, aryl, and heteroaryl;
$R^0$ is selected from the group consisting of a hydrogen atom, halogen, alkyl, alkenyl, alkynyl, heteroalkyl, alkoxy,

haloalkyl, haloalkoxy, cyano, $-NR^gR^L$, nitro, hydroxy, hydroxyalkyl, and

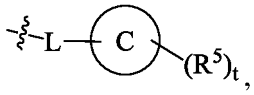

wherein the alkyl is optionally substituted with one or more substituents selected from the group consisting of alkoxy, haloalkoxy, cyano, $-NR^7R^8$, $-C(O)NR^7R^8$, cycloalkyl, heterocyclyl, aryl, and heteroaryl;

L is selected from the group consisting of a chemical bond, $NR^L$, an oxygen atom, a sulfur atom, and alkylene, wherein the alkylene is optionally substituted with one or more substituents selected from the group consisting of halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl;

$R^g$ and $R^L$ are identical or different and are each independently selected from the group consisting of a hydrogen atom, alkyl, alkenyl, alkynyl, haloalkyl, hydroxyalkyl, cycloalkyl, heterocyclyl, aryl, and heteroaryl;

ring C is selected from the group consisting of cycloalkyl, heterocyclyl, aryl, and heteroaryl;

each $R^1$ is identical or different and is independently selected from the group consisting of a hydrogen atom, halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and

heteroaryl, wherein the alkyl, alkenyl, alkynyl, alkoxy, cycloalkyl, heterocyclyl, aryl, and heteroaryl are each independently and optionally substituted with one or more substituents selected from the group consisting of halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl;

$R^2$ is selected from the group consisting of a hydrogen atom, halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl, wherein the alkyl, alkenyl, alkynyl, alkoxy, cycloalkyl, heterocyclyl, aryl, and heteroaryl are each independently and optionally substituted with one or more substituents selected from the group consisting of halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl;

$R^3$ is selected from the group consisting of a hydrogen atom, halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl, wherein the alkyl, alkenyl, alkynyl, alkoxy, cycloalkyl, heterocyclyl, aryl, and heteroaryl are each independently and optionally substituted with one or more substituents selected from the group consisting of halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl;

each $R^4$ is identical or different and is independently selected from the group consisting of a hydrogen atom, halogen, alkyl, alkenyl, alkynyl, alkoxy, oxo, haloalkyl, haloalkoxy, cyano, amino, nitro, hydroxy, hydroxyalkyl, cycloalkyl, heterocyclyl, aryl, and

heteroaryl, wherein the alkyl, alkenyl, alkynyl, alkoxy, cycloalkyl, heterocyclyl, aryl, and heteroaryl are each independently and optionally substituted with one or more substituents selected from the group consisting of halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl;

$R^{4a}$ is selected from the group consisting of a hydrogen atom, halogen, alkyl, alkenyl, alkynyl, alkoxy, haloalkyl, haloalkoxy, cyano, amino, nitro, hydroxy, and hydroxyalkyl;

each $R^5$ is identical or different and is independently selected from the group consisting of a hydrogen atom, halogen, alkyl, alkenyl, alkynyl, alkoxy, oxo, haloalkyl, haloalkoxy, cyano, amino, $-NR^cR^d$, nitro, hydroxy, hydroxyalkyl, cycloalkyl, heterocyclyl, aryl, and heteroaryl, wherein the alkyl, alkenyl, alkynyl, alkoxy, cycloalkyl, heterocyclyl, aryl, and heteroaryl are each independently and optionally substituted with one or more substituents selected from the group consisting of halogen, alkyl, oxo, alkenyl, alkynyl, alkoxy, haloalkyl, haloalkoxy, cyano, amino, nitro, hydroxy, hydroxyalkyl, $-C(O)OR^6$, $-C(O)NR^7R^8$, $-NR^7R^8$, $-S(O)_2R^9$, cycloalkyl, heterocyclyl, aryl, and heteroaryl;

$R^6$ is selected from the group consisting of a hydrogen atom, alkyl, alkenyl, alkynyl, haloalkyl, hydroxyalkyl, cycloalkyl, heterocyclyl, aryl, and heteroaryl;

$R^c$, $R^d$, $R^7$, and $R^8$ are identical or different and are each independently selected from the group consisting of a hydrogen atom, alkyl, alkenyl, alkynyl, haloalkyl, hydroxyalkyl, cycloalkyl, heterocyclyl, aryl, and heteroaryl;

or $R^c$ and $R^d$, together with the nitrogen atom to which they are attached, form heterocyclyl, and the heterocyclyl is optionally substituted with one or more substituents selected from the group consisting of halogen, alkyl, oxo, alkenyl, alkynyl, alkoxy, haloalkyl, haloalkoxy, cyano, amino, nitro, hydroxy, hydroxyalkyl, cycloalkyl, heterocyclyl, aryl, and heteroaryl;

or $R^7$ and $R^8$, together with the nitrogen atom to which they are attached, form heterocyclyl, and the heterocyclyl is optionally substituted with one or more substituents selected from the group consisting of halogen, alkyl, oxo, alkenyl, alkynyl, alkoxy, haloalkyl, haloalkoxy, cyano, amino, nitro, hydroxy, hydroxyalkyl, cycloalkyl, heterocyclyl, aryl, and heteroaryl;

$R^9$ is selected from the group consisting of a hydrogen atom, alkyl, alkenyl, alkynyl, haloalkyl, hydroxyalkyl, cyano, amino, hydroxy, cycloalkyl, heterocyclyl, aryl, and

heteroaryl;

J is 0, 1, or 2;

k is 0, 1, or 2;

n is 0, 1, 2, 3, or 4;

s is 0, 1, 2, 3, or 4; and

t is 0, 1, 2, 3, or 4.

**2.** The compound of general formula (I) or the pharmaceutically acceptable salt thereof according to claim 1, being a compound of general formula (II) or general formula (III) or a pharmaceutically acceptable salt thereof:

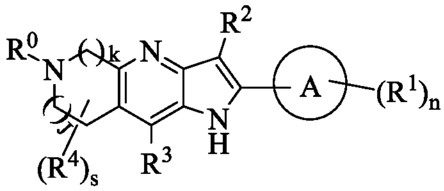

( II )

or

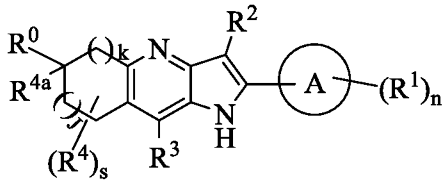

( III )

wherein:

ring A, $R^0$, $R^1$ to $R^4$, $R^{4a}$, J, k, n, and s are as defined in claim 1.

**3.** The compound of general formula (I) or the pharmaceutically acceptable salt thereof according to claim 1, being a compound of general formula (IIG) or general formula (IV) or a pharmaceutically acceptable salt thereof:

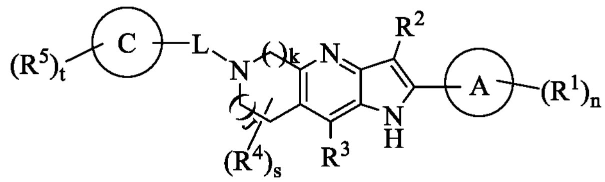

( IIG )

or

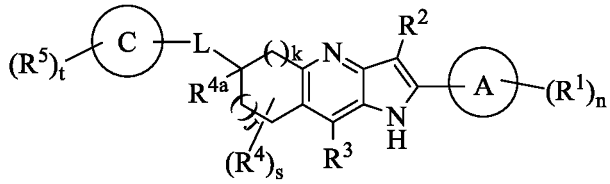

( IV )

wherein:

ring A, ring C, L, $R^1$ to $R^5$, $R^{4a}$, J, k, n, s, and t are as defined in claim 1.

**4.** The compound of general formula (I) or the pharmaceutically acceptable salt thereof according to any one of claims 1 to 3, wherein ring A is 6- to 10-membered aryl or 5- to 10-membered heteroaryl; preferably, ring A is pyridinyl or

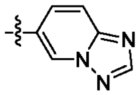;

more preferably, ring A is pyridinyl.

5. The compound of general formula (I) or the pharmaceutically acceptable salt thereof according to any one of claims 1 to 4, wherein J is 0 or 1, and k is 1 or 2; preferably, J is 1, and k is 1.

6. The compound of general formula (I) or the pharmaceutically acceptable salt thereof according to any one of claims 1 to 2, and 4 to 5, wherein $R^0$ is selected from the group consisting of a hydrogen atom, $C_{1-6}$ alkyl, $-NR^gR^L$, and

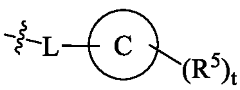,

wherein the $C_{1-6}$ alkyl is optionally substituted with one or more $-C(O)NR^7R^8$; L, ring C, $R^5$, $R^7$, $R^8$, $R^g$, $R^L$, and t are as defined in claim 1.

7. The compound of general formula (I) or the pharmaceutically acceptable salt thereof according to any one of claims 1 to 6, wherein L is $NR^L$ or $C_{1-6}$ alkylene; $R^L$ is a hydrogen atom or $C_{1-6}$ alkyl; and/or ring C is selected from the group consisting of 3- to 8-membered cycloalkyl, 3- to 8-membered heterocyclyl, 6- to 10-membered aryl, and 5-to 10-membered heteroaryl.

8. The compound of general formula (I) or the pharmaceutically acceptable salt thereof according to any one of claims 1 to 7, wherein each $R^1$ is identical or different and is independently selected from the group consisting of a hydrogen atom, halogen, $C_{1-6}$ alkyl, and $C_{1-6}$ haloalkyl.

9. The compound of general formula (I) or the pharmaceutically acceptable salt thereof according to any one of claims 1 to 8, wherein $R^2$ is selected from the group consisting of a hydrogen atom, halogen, $C_{1-6}$ alkyl, $C_{1-6}$ haloalkyl, and 3- to 12-membered heterocyclyl.

10. The compound of general formula (I) or the pharmaceutically acceptable salt thereof according to any one of claims 1 to 9, wherein $R^3$ is selected from the group consisting of a hydrogen atom, halogen, $C_{1-6}$ alkyl, and $C_{1-6}$ haloalkyl.

11. The compound of general formula (I) or the pharmaceutically acceptable salt thereof according to any one of claims 1 to 10, wherein each $R^4$ is identical or different and is independently a hydrogen atom or $C_{1-6}$ alkyl.

12. The compound of general formula (I) or the pharmaceutically acceptable salt thereof according to any one of claims 1 to 11, wherein each $R^5$ is identical or different and is independently selected from the group consisting of a hydrogen atom, halogen, $C_{1-6}$ alkyl, $C_{1-6}$ haloalkyl, $-NR^cR^d$, and 3- to 12-membered heterocyclyl, and $R^c$ and $R^d$ are identical or different and are each independently selected from the group consisting of a hydrogen atom, $C_{1-6}$ alkyl, and 3- to 12-membered heterocyclyl.

13. The compound of general formula (I) or the pharmaceutically acceptable salt thereof according to any one of claims 1 to 12, being selected from any one of the following compounds:

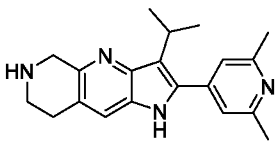
1 ,

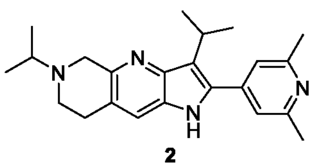
2 ,

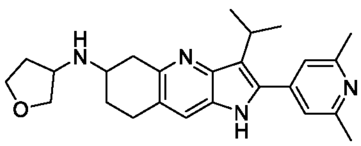
3 ,

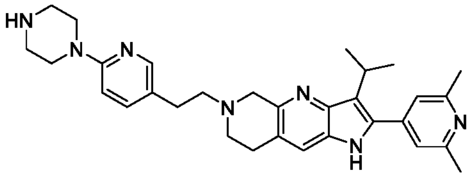

4

,

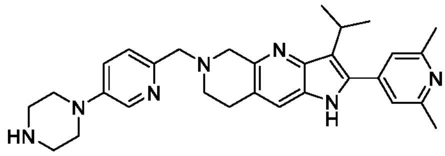

5

,

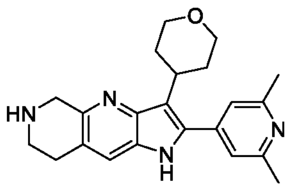

6

,

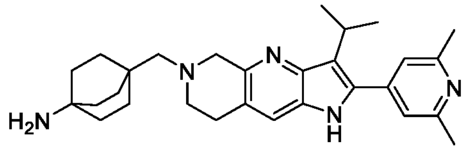

7

,

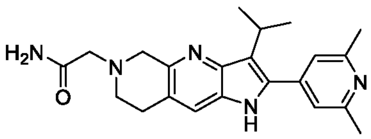

8

,

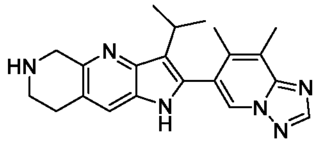

9

,

and

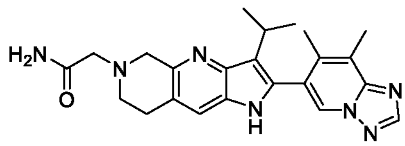

10

.

**14.** The compound of general formula (I) or the pharmaceutically acceptable salt thereof according to claim 1, being a compound of general formula (IIA) or (IVA) or a pharmaceutically acceptable salt thereof:

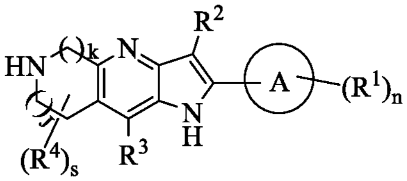

( IIA )

or

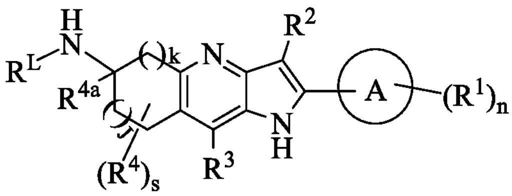

( IVA )

wherein:
ring A, $R^1$ to $R^4$, $R^{4a}$, $R^L$, J, k, n, and s are as defined in claim 1.

**15.** A compound of general formula (IIB) or a pharmaceutically acceptable salt thereof,

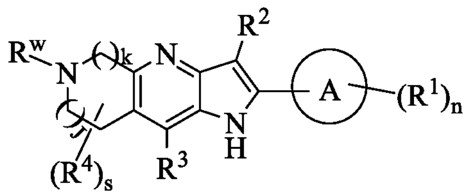

**( IIB )**

wherein:

$R^w$ is an amino protecting group, preferably tert-butoxycarbonyl;
ring A, $R^1$ to $R^4$, J, k, n, and s are as defined in claim 1.

**16.** A compound or a pharmaceutically acceptable salt thereof, being selected from any one of the following compounds:

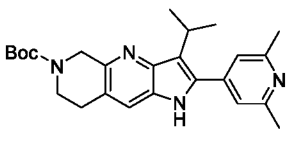

**1m** ,

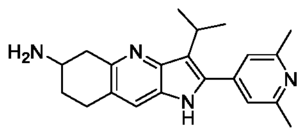

**3k** ,

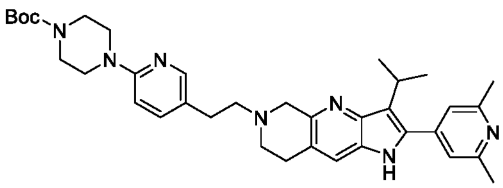

**4e** ,

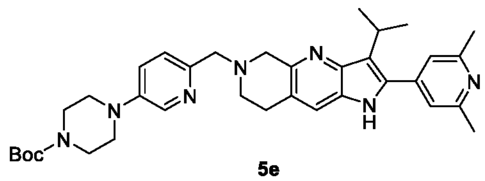

**5e** ,

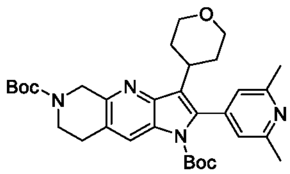

**6d** ,

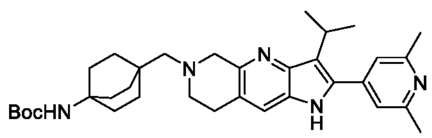

**7b** ,

and

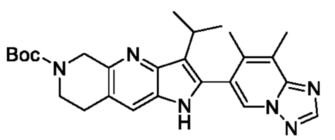

**9c** .

**17.** A method for preparing a compound of general formula (II) or a pharmaceutically acceptable salt thereof, wherein the method comprises:

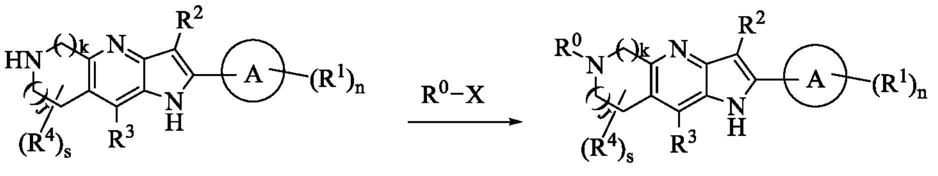

( IIA ) ( II )

conducting a nucleophilic substitution reaction of a compound of general formula (IIA) or a pharmaceutically acceptable salt thereof with $R^0$-X to give the compound of general formula (II) or the pharmaceutically acceptable salt thereof, wherein:

X is a leaving group, preferably halogen, and more preferably bromine or iodine;
$R^0$ is selected from the group consisting of alkyl, alkenyl, alkynyl, heteroalkyl, haloalkyl, hydroxyalkyl, and

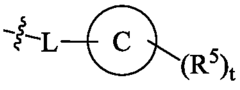

,

wherein the alkyl is optionally substituted with one or more substituents selected from the group consisting of alkoxy, haloalkoxy, cyano, $-NR^7R^8$, $-C(O)NR^7R^8$, cycloalkyl, heterocyclyl, aryl, and heteroaryl;
preferably, $R^0$ is selected from the group consisting of alkyl, alkenyl, alkynyl, heteroalkyl, haloalkyl, hydroxyalkyl, and

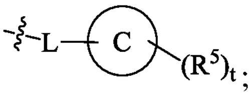

;

L is a chemical bond or alkylene, wherein the alkylene is optionally substituted with one or more substituents selected from the group consisting of halogen, alkyl, alkenyl, alkynyl, alkoxy, haloalkyl, haloalkoxy, cyano, amino, nitro, hydroxy, hydroxyalkyl, cycloalkyl, heterocyclyl, aryl, and heteroaryl;
ring A, ring C, $R^1$ to $R^5$, n, s, t, J, and k are as defined in claim 2.

**18.** A method for preparing a compound of general formula (IIA) or a pharmaceutically acceptable salt thereof, wherein the method comprises:

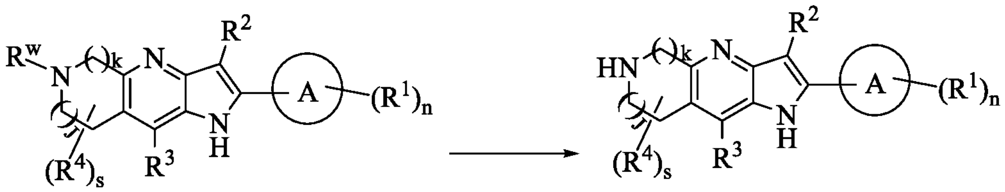

( IIB ) ( IIA )

removing the protecting group $R^w$ from a compound of general formula (IIB) or a pharmaceutically acceptable salt thereof to give the compound of general formula (IIA) or the pharmaceutically acceptable salt thereof, wherein:

$R^w$ is an amino protecting group, preferably tert-butoxycarbonyl;
ring A, $R^1$ to $R^4$, n, s, J, and k are as defined in claim 14.

**19.** A method for preparing a compound of general formula (IIB) or a pharmaceutically acceptable salt thereof, wherein the method comprises:

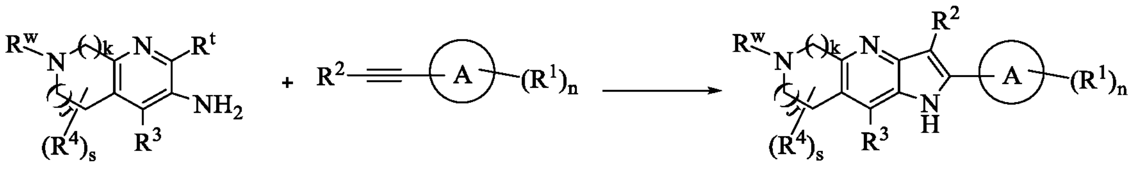

( IIa ) ( IIbb ) ( IIB )

conducting a Larock indole synthesis reaction of a compound of general formula (IIa) or a pharmaceutically acceptable salt thereof with a compound of general formula (IIbb) to give the compound of general formula (IIB) or the pharmaceutically acceptable salt thereof,
wherein:

$R^w$ is an amino protecting group, preferably tert-butoxycarbonyl;
$R^t$ is halogen, preferably bromine;
ring A, $R^1$ to $R^4$, n, s, J, and k are as defined in claim 15.

**20.** A pharmaceutical composition, wherein the pharmaceutical composition comprises the compound of general formula (I) or the pharmaceutically acceptable salt thereof according to any one of claims 1 to 13, and one or more pharmaceutically acceptable carriers, diluents, or excipients.

**21.** Use of the compound of general formula (I) or the pharmaceutically acceptable salt thereof according to any one of claims 1 to 13 or the pharmaceutical composition according to claim 20 in the preparation of a medicament for inhibiting TLR7, TLR8, or TLR9, preferably in the preparation of a medicament for inhibiting TLR7, or in the preparation of a medicament for inhibiting TLR7 and TLR8, or in the preparation of a medicament for inhibiting TLR7 and TLR9.

**22.** Use of the compound of general formula (I) or the pharmaceutically acceptable salt thereof according to any one of claims 1 to 13 or the pharmaceutical composition according to claim 20 in the preparation of a medicament for inhibiting TLR7, TLR8, and TLR9.

**23.** Use of the compound of general formula (I) or the pharmaceutically acceptable salt thereof according to any one of claims 1 to 13 or the pharmaceutical composition according to claim 20 in the preparation of a medicament for treating and/or preventing an inflammatory or autoimmune disease.

**24.** The use according to claim 23, wherein the inflammatory or autoimmune disease is selected from the group consisting of systemic lupus erythematosus (SLE), rheumatoid arthritis, multiple sclerosis (MS), and Sjögren's syndrome.

**INTERNATIONAL SEARCH REPORT**

International application No. **PCT/CN2021/133540**

**A. CLASSIFICATION OF SUBJECT MATTER**

C07D 519/00(2006.01)i； C07D 487/04(2006.01)i； C07D 471/04(2006.01)i； C07D 213/80(2006.01)i； C07D 213/75(2006.01)i； A61K 31/435(2006.01)i； A61K 31/495(2006.01)i； A61K 31/519(2006.01)i； A61P 31/12(2006.01)i； A61P 31/14(2006.01)i； A61P 31/22(2006.01)i

According to International Patent Classification (IPC) or to both national classification and IPC

**B. FIELDS SEARCHED**

Minimum documentation searched (classification system followed by classification symbols)

CO7D; A61K; A61P

Documentation searched other than minimum documentation to the extent that such documents are included in the fields searched

Electronic data base consulted during the international search (name of data base and, where practicable, search terms used)

CNKI, CNPAT, EPODOC, WPI, STN, 江苏恒瑞医药, 稠合三环, 吡啶, 吡咯, 哌嗪, 萘啶, pyrazole, pyridine, piperazine, naphthyridine, structural formula search

**C. DOCUMENTS CONSIDERED TO BE RELEVANT**

| Category* | Citation of document, with indication, where appropriate, of the relevant passages | Relevant to claim No. |
|---|---|---|
| A | CN 101384593 A (PFIZER INC.) 11 March 2009 (2009-03-11) abstract, claims 1-38 | 1-24 |
| A | CN 108948016 A (JIANGSU HENGRUI MEDICINE CO., LTD. et al.) 07 December 2018 (2018-12-07) abstract, and claims 1-20 | 1-24 |
| A | CN 110177793 A (JIANGSU HENGRUI MEDICINE CO., LTD. et al.) 27 August 2019 (2019-08-27) abstract, and claims 1-19 | 1-24 |
| A | CN 105367576 A (CHIA TAI TIANQING PHARMACEUTICAL GROUP CO., LTD.) 02 March 2016 (2016-03-02) abstract, and claims 1-16 | 1-24 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **28 January 2022** | **24 February 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**
**Information on patent family members**

International application No.
**PCT/CN2021/133540**

| Patent document cited in search report | | | Publication date (day/month/year) | Patent family member(s) | | | Publication date (day/month/year) |
|---|---|---|---|---|---|---|---|
| CN | 101384593 | A | 11 March 2009 | DO | P2007000030 | A | 15 September 2007 |
| | | | | ZA | 200806811 | A | 27 January 2010 |
| | | | | ZA | 200806811 | B | 27 January 2010 |
| CN | 108948016 | A | 07 December 2018 | None | | | |
| CN | 110177793 | A | 27 August 2019 | EP | 3636646 | A1 | 15 April 2020 |
| | | | | EP | 3636646 | A4 | 09 December 2020 |
| | | | | TW | 201900647 | A | 01 January 2019 |
| | | | | WO | 2018210298 | A1 | 22 November 2018 |
| | | | | JP | 2020520379 | A | 09 July 2020 |
| | | | | US | 2020131187 | A1 | 30 April 2020 |
| | | | | US | 11111249 | B2 | 07 September 2021 |
| CN | 105367576 | A | 02 March 2016 | None | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019233941 A1
- WO 2020020800 A1
- WO 2018049089 A1
- WO 2017106607 A1
- CN 109923108 A
- WO 2020048605 A1
- WO 2018138356 A1
- WO 2013003383 A1
- WO 2018005586 A1


### Non-patent literature cited in the description


- **KAWAI, T. et al.** *Nature Immunol.,* 2010, vol. 11, 373-384
- **NAVARRA, S. V. et al.** *Lancet,* 2011, vol. 377, 721
- **BEVERLY D.LC et al.** *Mol Immunol.,* 2014, vol. 61, 38-43
- **SANTIAGO-RABER ML et al.** *J Immunol.,* 2008, vol. 181, 1556-1562
- **DWIGHT H.KONO et al.** *PNAS,* 2009, vol. 106 (29), 12061-12066